Europäisches Patentamt

European Patent Office

Office européen des brevets

(11) Publication number: **0 471 407 A2**

## EUROPEAN PATENT APPLICATION

(21) Application number: **91202025.2**

(22) Date of filing: **07.08.91**

(51) Int. Cl.⁵: **C07K 17/02**, A61K 39/385, A61K 39/21

(30) Priority: **13.08.90 US 566654**
**13.08.90 US 566656**
**13.08.90 US 566638**

(43) Date of publication of application:
**19.02.92 Bulletin 92/08**

(84) Designated Contracting States:
**AT BE CH DE DK ES FR GB GR IT LI LU NL SE**

(71) Applicant: **MERCK & CO. INC.**
**126, East Lincoln Avenue P.O. Box 2000**
**Rahway New Jersey 07065-0900(US)**

(72) Inventor: **Lewis, John A.**
**1229 Clearbrook Road**
**West Chester, PA 19380(US)**
Inventor: **Davide, Joseph P.**
**471 Wexford Circle**
**Harleysville, PA 19438(US)**
Inventor: **Waterbury, Julie ANN**
**2610 Skippack Pike, Apt. 1, RD 3**
**Norristown, PA 19403(US)**

(74) Representative: **Barrett-Major, Julie Diane et al**
**Merck & Co., Inc. European Patent Department Terlings Park Eastwick Road Harlow Essex CM20 2OR(GB)**

(54) **New embodiments of the HIV principal neutralizing determinant.**

(57) New amino acid sequences of an envelope fragment of HIV are disclosed, as well as immunological conjugates for immunological purposes, including vaccination against AIDS.

EP 0 471 407 A2

Acquired Immune Deficiency Syndrome (AIDS) is the clinical manifestation of the apparent infection of CD4 helper T-cells and other cell targets by human immunodeficiency virus (HIV), also previously referred to as human T-lymphotropic virus type III (HTLV-III), Lymphoadenopathy-associated virus (LAV), or AIDS-related virus (ARV) (hereinafter collectively "HIV"). AIDS is a transmissible deficiency of cellular immunity characterized by opportunistic infections and certain malignancies. A similar disease, AIDS-related complex (ARC), shares many of the epidemiological features and immune abnormalities with AIDS, and often precedes the clinical manifestations of AIDS.

A vaccine against AIDS and/or ARC is an ideal prophylactic treatment for preventing the delibilitating effects of infection by HIV. Applicants have discovered new immunogens useful for such a vaccine. The immunogens are new principal neutralizing determinants (PNDs) of HIV.

Many of the details of the genetic function and virion structure of HIV have not yet been elucidated. However, certain general features have emerged. An RNA virus with a genome totaling about 9 kilobases (kb), its nucleotide sequence contains seven major open reading frames (ORFs) corresponding to the gag, pol and env, vif, tat, rev, and nef genes. The genes gag, pol and env code respectively for core subunits, viral enzymes such as reverse transcriptase or protease, and outer surface subunits. The gene vif codes for a viral infectivity factor, which is a protein involved with enhancement of cell-to-cell transmission of virions without affecting the budding process. The gene tat codes for a small protein that transactivates the expression of all viral proteins. The gene rev regulates expression of the viral proteins of gag, pol and env genes, possibly by facilitating transport of incompletely spliced RNA. The nef gene codes for a viral protein found in the cell cytoplasm, and it may modulate the host cellular signaling system and serve as a transciptional silencer. Terminal repeats in the nucleotide sequence are common to many retroviruses such as HIV and are required for viral replication and integration into the host chromosome. More recent discussions on the general nature of HIV genomic structure, replication and regulation are found in Ratner, L. et al. "Human T-Lymphotropic Retroviruses," in O'Brien, S.J. (ed.) Genetic Maps 1987 Cold Spring Harbor 1987 pp. 124-129; Franchini, G. et al., Nature 328, 539 (1987); Varmus, H. Genes & Dev 2, 1055 (1988).

Principal neutralizing determinants (PNDs) have been located within a selected, conserved region of the env gene. These PNDs are still undefined. Applicants have discovered and defined new embodiments of PND.

AIDS is a disease of a virus with a unique collection of attributes. HIV itself targets the immune system; it possesses a reverse transcriptase capable of turning out highly mutated progeny; it is sequestered from the immune system and it has a hypervariable surface in the (env) region. See, e.g. Hilleman, M.R., Vaccine 6, 175 (1988); Barnes, D.M., Science 240, 719 (1988). In view of these attributes, it was neither anticipated nor expected that the principal neutralizing determinants of this invention would serve as effective AIDS immunogens.

## BRIEF DESCRIPTION OF THE INVENTION

New principal neutralizing determinants of HIV are disclosed, and are useful as immunogens for AIDS vaccines, particularly in the form of conjugates

## ABBREVIATIONS AND DEFINITIONS

| | |
|---|---|
| AIDS | Acquired immune deficiency syndrome |
| ARC | AIDS-related complex |
| conjugation | The process of covalently attaching 2 molecules each containing one or more immunological determinants, e.g., HIV envelope fragments and Omp |
| conjugate | Result of conjugation, also known as an antigenic conjugate or immunological conjugate |
| HIV | Generic term for the presumed etiological agent of AIDS and/or ARC, also referred to as strains HTLV-III, LAV, and ARV. |
| PND | Principal neutralization determinant of HIV |
| Omp | Outer membrane proteosome |
| Recombinant protein | A polypeptide or oligopeptide expressed by foreign DNA in a recombinant eukaryotic or procaryotic expression system. |
| Recombinant expression system | A cell containing a foreign DNA expressing a foreign protein or a foreign oligopeptide. |

| Amino Acids | | |
|---|---|---|
| Full Name | Three-letter symbol | One-letter symbol |
| Alanine | Ala | A |
| Arginine | Arg | R |
| Asparagine | Asn | N |
| Aspartic acid | Asp | D |
| Asn and/or Asp | Asx | B |
| Cysteine | Cys | C |
| Glutamine | Gln | Q |
| Glutamic acid | Glu | E |
| Gln and/or Glu | Glx | Z |
| Glycine | Gly | G |
| Histidine | His | H |
| Isoleucine | Ile | I |
| Leucine | Leu | L |
| Lysine | Lys | K |
| Methionine | Met | M |
| Phenylalanine | Phe | F |
| Proline | Pro | P |
| Serine | Ser | S |
| Threonine | Thr | T |
| Tryptophan | Trp | W |
| Tyrosine | Tyr | Y |
| Valine | Val | V |

| Nucleotides Bases in DNA or RNA | |
|---|---|
| Name | One-letter symbol |
| Adenine | A |
| Cytosine | C |
| guamine | G |
| thymine | T |
| uracil | U |

The terms "protein," "peptide," "oligopeptide," and "polypeptide" and their plurals have been used interchangeably to refer to chemical compounds having amino acid sequences of five or more amino acids. "Amino acid" refers to any of the 20 common amino acids for which codons are naturally available, and are listed in the table of amino acids given above.

When any variable (e.g. PND) occurs more than one time in any constituent or in Formula I, its definition on each occurrence is independent of its definition at every other occurrence. Also, combinations of substituents and/or variables are permissible only if such combinations result in stable compounds.

## DETAILED DESCRIPTION OF THE INVENTION

The present invention provides an effective immunogen against AIDS or ARC, and comprises an antigenic conjugate of the formula

$(PND)_n \sim (Omp)$     I,

wherein:

PND    is the principal neutraliziation determinant of HIV, which is a polypeptide of one or more amino acid sequences;

n =    1-50, wherein n is the number of polypeptides of PND covalently linked to Omp;

~  indicates covalent linkage;

Omp  is outer membrane proteosome of the microorganism Neisseria;

said polypeptide containing in its sequence Gly-X-Gly, wherein X is proline, leucine, alanine, glutamine or serine.

The antigenic conjugates of this invention are prepared by isolating and purifying their component parts PND and Omp, then conjugating PND and Omp together. Subsequent purification of conjugate mixtures may be performed as desired.

The new PND amino acid sequences of this invention include any fragment thereof, provided said fragment is at least five amino acids in length.

Each PND amino acid sequence is determined by DNA sequencing of HIV clones amplified by the polymerase chain reaction.

Polymerase Chain Reaction Amplification

Large amounts of DNA coding for PND protein may be obtained using polymerase chain reaction (PCR) amplification techniques as described in Mullis et al., U.S. Patent No. 4,800,159 and other published sources. See also, for example, Innis, M.A. et al. PCR Protocals Academic Press 1990. The extension product of one primer, when hybridized to another primer, becomes a template for the synthesis of another nucleic acid molecule.

The primer template complexes act as substrate for DNA polymerase which, in performing its replication function, extends the primers. The region in common with both primer extensions, upon denaturation, serves as template for a repeated primer extension.

Taq DNA Polymerase catalyzes primer extension in the amplification process. The enzyme is a thermostable DNA polymerase isolated from Thermus aquaticus. Because it stays active through repeated elevations to high denaturation temperatures, it needs to be added only once. Deoxynucleotide triphosphates provide the building blocks for primer extension.

The nucleic acid sequence strands are heated until they separate, in the presence of oligonucleotide primers that bind to their complementary strand at a particular site of the template. This process is continued with a series of heating and cooling cycles, heating to separate strands, and cooling to reanneal and extend the sequences. More and more copies of the strands are generated as the cycle is repeated. Through amplification, the coding domain and any additional primer-encoded information such as restriction sites or translation signals (signal sequences, start codons and/or stop codons) is obtained.PCR protocols are often performed at the 100 $\mu$L scale in 0.5 ml microcentrifuge tubes. The PCR sample may be single-or double-stranded DNA or RNA. If the starting material is RNA, reverse transcriptase is used to prepare first strand cDNA prior to PCR. Typically, nanogram amounts of cloned template, up to microgram amounts of genomic DNA, or 20,000 target copies are chosen to start optimization trials.

PCR primers are oligonucleotides, typically 15 to 50 bases long, and are complementary to sequences defining the 5' ends of the complementary template strands. Non-template complementary 5' extensions may be added to primers to allow a variety of useful post amplification operations on the PCR product without significant perturbation of the amplification itself. It is important that the two PCR primers not contain more than two bases complementary with each other, especially at their 3' ends. Internal secondary structure should be avoided in primers.

Because Taq DNA Polymerase has activity in the 37-55°C range, primer extension will occur during the annealing step and the hybrid will be stabilized. The concentrations of the primers are preferably equal in conventional PCR and, typically, are in vast excess of the template to be reproduced.

In one typical PCR protocol, each deoxynucleotide triphosphate concentration is preferably about 200 $\mu$M. The four dNTP concentrations are preferably above the estimated Km of each dNTP (10-15 $\mu$M).

Preferably PCR buffer is composed of about 500 mM potassium chloride, 10.0 mM Tris-HCl (pH 8.3 at room temperature), 1.5 mM magnesium chloride, and 0.01% w/v gelatin. In the presence of 0.8 mM total dNTP concentration, a titration series in small increments over the 1.5-to 4-mM range will locate the magnesium concentration producing the highest yield of a specific product. Too little free magnesium will result in no PCR product and too much free magnesium may produce a variety of unwanted products.

Preferably, in a 100-$\mu$L reaction volume, 2.0 to 2.5 units of Taq DNA Polymerase are recommended. The enzyme can be added conveniently to a fresh master mix prepared for a number of reactions, thereby avoiding accuracy problems associated with adding individual 0.5-$\mu$L enzyme aliquots to each tube. A typical PCR protocol for amplification of the DNA template includes a 1 minute 94°C denaturation step, a 1 minute 37°C primer annealing step, and a 2 minute 72°C primer extension step. This will amplify a 500 base-pair product at least 100,000-fold in 25 cycles.

During DNA denaturation, sufficient time must be allowed for thermal equilibration of the sample. The practical range of effective denaturation temperatures for most samples is 92-95°C, with 94°C being the standard choice.

Primer annealing is usually performed first at 37°C, and the specificity of the product is evaluated. If unwanted bands are observed, the annealing temperature should be raised in subsequent optimization runs. While the primer annealing temperature range is often 37-55°C, it may be raised as high as the extension temperature in some cases. Merging of the primer annealing and primer extension steps results in a two-step PCR process.

Primer extension, in most applications, occurs effectively at a temperature of 72°C and seldom needs optimization. In the two-temperature PCR process the temperature range may be 65-70°C. In situations where enzyme concentration limits amplification in late cycles, the extension is preferably increased linearly with cyclic number. Usually, 25 to 45 cycles are required for extensive amplification (i.e., 1,000,000 fold) of a specific target.

Once the DNA sequence is determined, through conventional and well-known techniques, its amino acid sequence can be deduced by "translating" the DNA sequence. The resulting amino acid sequence having the principal neutralizing determinant of the envelope gene is then employed to synthesize large quantities of PND protein or fragment thereof. Synthesis is performed by organic synthesis or by recombinant expression systems, or both.

Preparation of Principal Neutralization Determinant

A. Organic Synthesis of PND:

Standard and conventional methods exist for rapid and accurate synthesis of long peptides on solid-phase supports. Solution-phase synthesis is usually feasible only for selected smaller peptides.

Synthesis on solid-phase supports, or solid-phase synthesis, is most conveniently performed on an automated peptide synthesizer according to e.g., Kent, S. et al., "Modern Methods for the Chemical Synthesis of Biologically Active Peptides," in Alitalo, K. et al., (eds.). Synthetic Peptides in Biology and Medicine, Elsevier 1985, pp. 29-57. Manual solid-phase synthesis may be employed instead, by following the classical Merrifield techniques, as described, for example, in Merrifield, R.B. J. Am. Chem. Soc. 85, 2149 (1963), or known improvements thereof. Solid-phase peptide synthesis may also be performed by the Fmoc method, which employs very dilute base to remove the Fmoc protecting group. Segment synthesis-condensation is a further variant of organic synthesis of peptides as within the scope of the techniques of the present invention.

In organic synthesis of peptides, protected amino acids are condensed to form amide or peptide bonds with the N-terminus of a growing peptide. Condensation is usually performed with the carbodiimide method by reagents such as dicyclohexylcarbodiimide, or N-ethyl, $N_1$-($\gamma$-dimethylaminopropyl) carbodiimide. Other methods of forming the amide or peptide bond include, but are not limited to, synthetic routes via an acid chloride, azide, mixed anhydride or activated ester. Common solid-phase supports include polystyrene or polyamide resins.

The selection of protecting groups of amino acid side claims is, in part, dictated by particular coupling conditions, in part by the amino acid and peptide components involved in the reaction. Such amino-protecting groups ordinarily employed include those which are well known in the art, for example, urethane protecting substituents such as benzyloxycarbonyl (carbobenzoxy), p-methoxycarbobenzoxy, p-nitrocarbobenzoxy, t-butyloxycarbonyl, and the like. It is preferred to utilize t-butoxycarbonyl (BOC) for protecting the $\epsilon$-amino group, in part because the BOC protecting group is readily removed by relatively mild acids such as trifluoroacetic acid (TFA), or hydrogen chloride in ethyl acetate.

The OH group of Thr and Ser may be protected by the Bzl (benzyl) group and the $\epsilon$-amino group of Lys may be protected by the isopropoxycarbonyl (IPOC) group or the 2-chlorobenzyloxycarbonyl (2-Cl-CBZ) group. Treatment with HF or catalytic hydrogenation are typically employed for removal of IPOC or 2-Cl-CBZ.

For preparing cocktails of closely related peptides, see, e.g., Houghton, R.A., Proc. Natl. Acad. Sci. USA 82, 5131 (1985).

B. Expression of PND in a Recombinant Expression System

It is now a relatively straightforward technology to prepare cells expressing a foreign gene. Such cells act as hosts and include E. coli, B. subtilis, yeasts, fungi, plant cells or animal cells. Expression vectors for many of these host cells have been isolated and characterized, and are used as starting materials in the construction, through conventional recombinant DNA techniques, of vectors having a foreign DNA insert of interest. Any DNA is foreign if it does not naturally derive from the host cells used to express the DNA insert. The foreign DNA insert may be expressed on extrachromosomal plasmids or after integration in whole or in part in the host cell chromosome(s), or may actually exist in the host cell as a combination of more than one molecular form. The choice of host cell and expression vector for the expression of a desired foreign DNA largely depends on availability of the host cell and how fastidious it is, whether the host cell will support the replication of the expression vector, and other factors readily appreciated by those of ordinary skill in the art.

The technology for recombinant procaryotic expression systems is now old and conventional. The typical host cell is E. coli. The technology is illustrated by treatises such as Wu, R (ed) Meth. Enzymol. 68 - (1979) and Maniatis, T. et. al., Molecular Cloning: A Laboratory Manual Cold Spring Harbor 1982.

The foreign DNA insert of interest comprises any DNA sequence coding for a PND (or fragment thereof of at least 5 amino acids in length) of the present invention, including any synthetic sequence with this coding capacity or any such cloned sequence or combination thereof. For example, PND peptide coded and expressed by an entirely recombinant DNA sequence is encompassed by this invention.

Vectors useful for constructing eukaryotic expression systems for the production of recombinant PND comprise the DNA sequence for PND, fragment or variant thereof, operatively linked thereto with appropriate transcriptional activation DNA sequences, such as a promoter and/or operator. Other typical features may include appropriate ribosome binding sites, termination codons, enhancers, terminators, or replicon elements. These additional features can be inserted into the vector at the appropriate site or sites by conventional splicing techniques such as restriction endonuclease digestion and ligation.

Yeast expression systems, which are one variety of recombinant eukaryotic expression systems, generally employ Saccharomyces cerevisiae as the species of choice for expressing recombinant proteins. S. cerevisiae and similar yeasts possess well known promoters useful in the construction of yeast expression systems, including but not limited to GAP491, GAL10, ADH2, and alpha mating factor.

Yeast vectors useful for constructing recombinant yeast expression systems for expressing PND include, but are not limited to, shuttle vectors, cosmid plasmids, chimeric plasmids, and those having sequences derived from 2-micron circle plasmids.

Insertion of the appropriate DNA sequence coding for PND, fragment or variant thereof, into these vectors will, in principle, result in a useful recombinant yeast expression system for PND where the modified vector is inserted into the appropriate host cell, by transformation or other means.

Recombinant mammalian expression systems are another means of producing the recombinant PND for the conjugates of this invention. In general, a host mammalian cell can be any cell that has been efficiently cloned in cell culture. Host mammalian cells useful for the purposes of constructing a recombinant mammalian expression system include, but are not limited to, Vero cells, NIH3T3, GH3, COS, murine C127 or mouse L cells. Mammalian expression vectors can be based on virus vectors, plasmid vectors which may have SV40, BPV or other viral replicons, or vectors without a replicon for animal cells. Detailed discussions on mammalian expression vectors can be found in the treatises of Glover, D.M. (ed.) "DNA Cloning: A Practical Approach," IRL 1985, Vols. I and II.

Recombinant PND may possess additional and desirable structural modifications not shared with the same organically synthesized peptide, such as adenylation, carboxylation, glycosylation, hydroxylation, methylation, phosphorylation or myristoylation. These added features may be chosen or preferred as the case may be, by the appropriate choice of recombinant expression system. On the other hand, recombinant PND may have its sequence extended by the principles and practice of organic synthesis of section A above.

Conjugation of PND and Omp to Form a Covalent Linkage(s) Yielding Conjugate

Antigenic conjugates of PND and Omp are useful for vaccination against AIDS or ARC. Such conjugates have at least one covalent linkage between the antigen PND and Omp, and typically have more than one PND molecule covalently bound to each Omp molecule.

PND and Omp are prepared separately, then linked by non-specific cross-linking agents, monogeneric spaces or bigeneric spacers. Methods for non-specific cross-linking include, but are not limited to, reaction with glutaraldehyde; reaction with N-ethyl-N'-(3-dimethylaminopropyl) carbodiimide, with or without admixture of a succinylated carrier; periodate oxidation of glycosylated substituents followed by coupling to free amino groups of a protein carrier in the presence of sodium borohydride or sodium cyanoborohydride; diazotization of aromatic amino groups followed by coupling on tyrosine side chain residues of the protein;

6

reaction with isocyanates; or reaction of mixed anhydrides. See, generally, Briand, J.P. et al. J. Imm. Meth. 78, 59 (1985). These methods of non-specifically cross-linking are conventional and well-known in the typical practice of preparing conjugates for immunological purposes.

In another embodiment of the invention conjugates formed with a monogeneric spacer are prepared. These spacers are bifunctional and require functionalization of only one of the partners of the reaction pair to be conjugated before conjugation takes place.

By way of illustration rather than limitation, an example of a monogeneric spacer involves coupling the polypeptide PND to one end of the bifunctional molecule adipic acid dihydrazide in the presence of carbodiimide. A diacylated hydrazine presumably forms with pendant glutamic or aspartic carboxyl groups of PND. Conjugation then is performed by a second coupling reaction with carrier protein in the presence of carbodiimide. For similar procedures, see for example, Schneerson, R. et al., J. Exp. Med. 152, 361 (1980). Another example of a monogeneric spacer is described in Fujii, N. et al. Int. J. Peptide Protein Res. 26, 121 (1985).

In another embodiment of the invention conjugates of PND and Omp are formed with a bigeneric spacer. These spacers are formed after each partner of the reaction pair to be conjugated, e.g., PND and Omp, is functionalized with a bifunctional spacer. Conjugation occurs when each functionalized partner is reacted with its opposite partner to form a stable covalent bond or bonds. See, for example, Marburg, S. et al., J. Am. Chem. Soc. 108, 5282-5287 (1986) and Marburg, S. et al., U.S. Patent 4,695,624, issued 22 September 1987, each incorporated by reference. Bigeneric spacers are preferred for preparing conjugates in human vaccines since the conjugation reaction is well characterized and easily controlled.

Typical and conventional immunological practice provides for the ready and easy synthesis of antigenic conjugates within the scope of the present invention, including the conjugation of Omp with virtually any desired degree of substitution of virtually any peptide of the Sequence Listing. Heterogeneous products of the conjugation reaction are easily separable if needed by a variety of suitable column chromatography techniques.

Vaccine Formulation

The form of the immunogen within the vaccine takes various molecular configurations. A single molecular species of the antigenic conjugate $(PND)_n \sim Omp$ will often suffice as a useful and suitable antigen for the prevention or treatment of AIDS or ARC. Other antigens in the form of cocktails are also advantageous, and consist of a mixture of conjugates that differ by, for example, the degree of substitution (n) or the amino acid sequence of PND or both.

An immunological vector or adjuvant may be added as an immunological vehicle according to conventional immunological testing or practice.

The conjugates of this invention when used as a vaccine, are to be administered in immunologically effective amounts. Dosages of between 1 $\mu$g and 500 $\mu$g of conjugate, and perferably between 50 $\mu$g and 300 $\mu$g of conjugate are to be administered to a mammal to induce anti-peptide, anti-HIV, or HIV-neutralizing immune responses. About two weeks after the initial administration, a booster dose may be administered, and then again whenever serum antibody titers diminish. The conjugate should be given intramuscularly at a concentration of between 10 $\mu$g/ml and 1 mg/ml, and preferably between 50 and 500 $\mu$g/ml, in a volume sufficient to make up the total required for immunological efficacy.

Adjuvants may or may not be added during the preparation of the vaccines of this invention. Alum is the typical and preferred adjuvant in human vaccines, especially in the form of a thixotropic, viscous, and homogeneous aluminum hydroxide gel. For example, one embodiment of the present invention is the prophylactic vaccination of patients with a suspension of alum adjuvant as vehicle and a cocktail of $(PND)_n$-Omp as the selected set of immunogens or antigens.

The vaccines of this invention may be effectively administered, whether at periods of pre-exposure and/or post-exposure, in combination with effective amounts of the AIDS antivirals, immunomodulators, anti-infectives, or vaccines of Table I.

## TABLE I

### ANTI-VIRALS

| Drug Name | Manufacturer | Indication |
|---|---|---|
| AL-721 | Ethigen (Los Angeles, CA) | ARC, PGL HIV positive, AIDS |
| Recombinant Human Interferon Beta | Triton Biosciences (Almeda, CA) | AIDS, Kaposi's sarcoma, ARC |
| Acemannan | Carrington Labs (Irving, TX) | ARC (See also immuno-modulators) |
| Cytovene Ganciclovir | Syntex (Palo Alto, CA) | sight threateining CMV peripheral CMV retinitis |
| d4T Didehydrodeoxy-thymidine | Bristol-Myers (New York, NY) | AIDS, ARC |
| dd Dideoxyinosine | Bristol-Myers (New York, NY) | AIDS, ARC |
| EL10 | Elan Corp, PLC (Gainesville, GA) | HIV infection (See also immuno-modulators) |
| Foscarnet Trisodium Phosphonoformate | Astra Pharm. Products, Inc. (Westborough, MA) | CMV retinitis, HIV infection, other CMV infections |

8

| Drug Name | Manufacturer | Indication |
|---|---|---|
| Dideoxycytidine; ddc | Hoffman-La Roche (Nutley, NJ) | AIDS, ARC |
| Novapren | Novaferon Labs, Inc. (Akron, OH) Diapren, Inc. (Roseville, MN, marketer) | HIV inhibitor |
| Peptide T Octapeptide Sequence | Peninsula Labs (Belmont, CA) | AIDS |
| Retrovir Zidovudine; AZT | Burroughs Wellcome (Rsch. Triangle Park, NC) | AIDS, adv, ARC pediatric AIDS, Kaposi's sarcoma, asymptomatic HIV infection, less severe HIV disease, neurological involvement, in combination w/other therapies, post-exposure prophylaxis in health care workers |
| Rifabutin Ansamycin LM 427 | Adria Laboratories (Dublin, OH) Erbamont (Stamford, CT) | ARC |

| Drug Name | Manufacturer | Indication |
|---|---|---|
| Dextran Sulfate | Ueno Fine Chem. Ind. Ltd. (Osaka, Japan) | AIDS, ARC, HIV positive asymptomatic |
| Virazole Ribavirin | Viratek/ICN (Costa Mesa, CA) | asymptomatic HIV positive, LAS, ARC |
| Alpha Interferon | Burroughs Wellcome (Rsch. Triangle Park, NC) | Kaposi's sarcoma, HIV in combination w/Retrovir |

Immuno-modulators

| Drug Name | Manufacturer | Indication |
|---|---|---|
| Antibody which neutralizes pH labile alpha aberrant Interferon in an immuno-adsorption column | Advanced Biotherapy Concepts (Rockville, MD) | AIDS, ARC |
| AS-101 | Wyeth-Ayerst Labs. (Philadelphia, PA) | AIDS |
| Bropirimine | Upjohn (Kalamazoo, MI) | advanced AIDS |
| Acemannan | Carrington Labs, Inc. (Irving, TX) | AIDS, ARC (See also anti-virals) |

10

| Drug Name | Manufacturer | Indication |
|---|---|---|
| CL246,738 | American Cyanamid (Pearl River, NY) Lederle Labs (Wayne, NJ) | AIDS, Kaposi's sarcoma |
| EL10 | Elan Corp, PLC (Gainesville, GA) | HIV infection (See also anti-virals) |
| Gamma Interferon | Genentech (S. San Francisco, CA) | ARC, in combination w/TNF (tumor necrosis factor) |
| Granulocyte Macrophage Colony Stimulating Factor | Genetics Institute (Cambridge, MA) Sandoz (East Hanover, NJ) | AIDS |
| Granulocyte Macrophage Colony Stimulating Factor | Hoeschst-Roussel (Somerville, NJ) Immunex (Seattle, WA) | AIDS |
| Granulocyte Macrophage Colony Stimulating Factor | Schering-Plough (Madison, NJ) | AIDS AIDS, in combination w/Retrovir |
| HIV Core Particle Immunostimulant | Rorer (Ft. Washington, PA) | seropositive HIV |

| Drug Name | Manufacturer | Indication |
|---|---|---|
| IL-2 Interleukin-2 | Cetus (Emerycille, CA) | AIDS, in combaintion w/Retrovir |
| IL-2 Interleukin-2 | Hoffman-La Roche (Nutley, NJ) | AIDS, ARC, HIV, in combination w/Retrovir |
| Immune Globulin Intravenous (human) | Cutter Biological (Berkeley, CA) | pediatric AIDS, in combination w/Retrovir |
| IMREG-1 | Imreg (New Orleans, LA) | AIDS, Kaposi's sarcoma, ARC, PGL |
| IMREG-2 | Imreg (New Orleans, LA) | AIDS, Kaposi's sarcoma, ARC, PGL |
| Imuthiol Diethyl Dithio Carbamate | Merieux Institute (Miami, FL) | AIDS, ARC |
| INTRON A Alpha-2 Interferon | Schering Plough (Madison, NJ) | Kaposi's sarcoma w/Retrovir: AIDS |
| Methionine- Enkephalin | TNI Pharmaceutical (Chicago, IL) | AIDS, ARC |
| MTP-PE Muramyl- Tripeptide | Ciba-Geigy Corp. (Summit, NJ) | Kaposi's sarcoma |

| Drug Name | Manufacturer | Indication |
|---|---|---|
| Granulocyte Colony Stimulating Factor | Amgen (Thousand Oaks, CA) | AIDS, in combination w/Retrovir |
| rCD4 Recombinant Soluble Human CD4 | Genentech (S. San Francisco, CA) | AIDS, ARC |
| Recombinant Soluble Human CD4 | Biogen (Cambridge, MA) | AIDS, ARC |
| Roferon-A Interferon Alfa 2a | Hoffman-La Roche (Nutley, NJ) | Kaposi's sarcoma AIDS, ARC, in combination w/Retrovir |
| SK&F106528 Soluble T4 | Smith, Kline & French Laboratories (Philadelphia, PA) | HIV infection |
| Thymopentin | Immunobiology Research Institute (Annandale, NJ) | HIV infection |
| Tumor Necrosis Factor; TNF | Genentech (S. San Francisco, CA) | ARC, in combination w/gamma Interferon |

## Anti-Infectives

| Drug Name | Manufacturer | Indication |
|---|---|---|
| Clindamycin with Primaquine | Upjohn (Kalamazoo, MI) | PCP |
| Diflucan Fluconazole | Pfizer (New York, NY) | cryptococcal meningitis, candidiasis |
| Pastille Nystatin Pastille | Squibb Corp. (Princeton, NJ) | prevention of oral candidiasis |
| Ornidyl Eflornithine | Merrell Dow (Cincinnati, OH) | PCP |
| Pentamidine Isethionate (IM & IV) | LyphoMed (Rosemont, IL) | PCP treatment |
| Piritrexim | Burroughs Wellcome (Rsch. Triangle Park, NC) | PCP treatment |
| Pentamidine isethionate for inhalation | Fisons Corporation (Bedford, MA) | PCP prophylaxis |
| Spiramycin | Phone-Poulenc Pharmaceuticals (Princeton, NJ) | cryptosporidial diarrhea |

| Drug Name | Manufacturer | Indication |
|---|---|---|
| Intraconazole-R51211 | Janssen Pharm. (Piscataway, NJ) | histoplasmosis; cryptococcal meningitis |
| Trimetrexate | Warner-Lambert | PCP |

## Other

| Drug Name | Manufacturer | Indication |
|---|---|---|
| Recombinant Human Erythropoietin | Ortho Pharm. Corp. (Raritan, NJ) | severe anemia assoc. and Retrovir therapy |
| Megestrol Acetate | Bristol-Myers (New York, NY) | treatment of anorexia assoc. w/AIDS |
| Total Enteral | Norwich Eaton Pharmaceuticals (Norwich, NY) | diarrhea and malabsorption related |

It will be understood that the scope of combinations of the antigenic conjugates of this invention with AIDS antivirals, immunomodulators, anti-infectives or vaccines is not limited to the list in the above Table, but includes in principle any combination with any pharmaceutical composition useful for the treatment of AIDS. The antigenic conjugates as AIDS or HIV vaccines of this invention include vaccines to be used pre- or post-exposure to prevent or treat HIV infection or disease, and are capable of producing an immune response specific for the immunogen.

EXAMPLE 1

Isolation of Genomic DNA from Frozen (-20°C) Pellets of Peripheral Blood Lymphocytes

Each DNA was prepared respecting the principle that preparation and storage of high molecular weight DNA be segregated from all polymerase chain reaction (PCR) amplification experiments.

15

Reagents

P-K Buffer

10 mM Tris          Prepare using sterile $H_2O$ in
400 mM NaCl   pH 7.4   plastic labware.  Sterile filter
2 mM EDTA           through a 0.45 μm filter
                    device and aliquot 10 ml into
                    15 ml conical tubes.  Store at
                    -20°C.

Proteinase K   1.0mg/ml   Dissolve the contents of a bottle
                    in sterile $H_2O$ to a final conc.
                    of 1.0 mg/ml.  Aliquot 0.3-0.5 ml
                    into freezer tubes.  Store at
                    -20°C.

SDS 10%             Prepare using sterile $H_2O$ in
                    plastic labware.  Sterile filter
                    through a 0.45 μm filter device
                    and aliquot 2.0 ml into Nalgene
                    freezer tubes.  Store at -20°C.

```
Phenol:Chloroform 50:50    Prepare and aliquot 8.0 ml into
                           15 ml conical tubes and store at
                           -20°C in the dark.


RNase A 1.0 mg/ml          Dissolve the contents of a bottle
                           in sterile H₂O to a final
                           conc. of 1.0 mg/ml.  Aliquot 0.3-
                           0.5 ml into freezer tubes.  Store
                           at -20°C.


95% and 70% EtOH           Store at -20°C.


Dilution Buffer            Prepare using sterile H₂O in
   10 mM Tris              plastic labware.  Sterile filter
   25 mM NaCl    pH 8.0    through a 0.45 µm filter device
   0.1 mM EDTA             and aliquot 10 ml in 15 ml
                           conical tubes.  Store at 4°C.
```

1) Suspend cell pellets of co-cultivated patient peripheral blood lymphocytes in 0.5 ml of P-K Buffer taking care to break up pellet completely.

2) Adjust sample to 100 $\mu$g/ml Proteinase K with 1.0 mg/ml stock. Mix well.

3) Adjust sample to 0.5% SDS with 10% stock. Mix well and incubate at 50$^\circ$C for 16-24 hours.

4) Extract sample with an equal volume of Phenol: Chloroform for 10 minutes @ 21-25$^\circ$C.

5) Split phases by centrifugation @ 2K for 5 minutes.

6) Remove aqueous and re-extract with an equal vol. of $CHCl_3$ for 2-5 minutes @ 21-25$^\circ$C. Split phases as before.

7) Repeat Step 6.

8) Adjust aqueous to 100 $\mu$g/ml RNase A with 1.0 mg/ml stock and incubate @ 37$^\circ$C for 90 minutes.

9) Repeat Steps 4, 5, 6, and 7.

10) Precipitate DNA with the addition of 2.5 vol of cold 95% EtOH.

11) Collect DNA for 30 minutes at 10,000 RPM's at 4$^\circ$C.

12) Remove EtOH and wash pellet once with 70% EtOH. Spin for 2 minutes as 10,000 RPM's.

13) Remove EtOH and dissolve the pellet in 300$\lambda$ of dilution buffer.

EXAMPLE 2

PCR Amplification of Genomic DNA from HIV Isolates

Genomic DNA was amplified by the polymerase chain reaction according to Scharf, S.J. et al. Science 233, 1076 (1986). A heat resistant T. aquaticus DNA polymerase was employed to enhance stability during thermal cycling. See, e.g., Saiki, R. K. et al. Science 239, 487 (1988). Excess primer for each strand was used. The primers were

RP.Hpa having the sequence

5'-P-TCT-GTT-AAC-TTC-ACA-GAC-AAT-GCT-AAA-ACC-ATA-ATA-GTA-CAG-CTG-3'; and

RP.Cla having the sequence

5'-P-GCA-ATC-GAT-CTG-TTT-TAA-AGT-GTT-ATT-CCA-TTT-TGC-3'

The 5' phosphate was added by chemical methods, according to Horn, T. et al., Tetrahedron Letters 27, 4705 (1986).

EXAMPLE 3

Filtration of PCR Amplified Sequences

General Considerations:

This filtration step removes free nucleotides and low molecular weight oligonucleotide contaminants which inhibit ligation, according to Sharf, et al. Science, 233, 1076 (1986).

1) Dilute up to 100λ of sample (1-2 $\mu$g DNA/ml) of Example 2 to 400λ with "buffer" (10 mM Tris•HCl, 25 mM NaCl, 0.1 mM EDTA, all buffered to pH8) and spin in a microcentrifuge for 5 minutes at RT.

N.B. No more than 4 samples can be placed in same rotors at one time. Be sure that the cap of the tube is completely closed or some volume may spray out of the unit. If using a non-dedicated microcentrifuge, spin sample at 2000 x g.

2) Remove insert and place in a clean 1.5 ml plastic tube containing a polysulfone filter with a 100,000 dalton molecular weight cut off. Redilute sample to 400λ with buffer and spin as before.

3) Repeat Step 2.

4a) For Cloning purposes:

Remove sample and rinse membrane gently with 10-20λ of buffer. Combine the sample and rinse and adjust back to the original volume. Check on agarose gel for yield and purity.

4b) For Reamplification purposes:

Remove sample carefully measuring volume. Rinse the membrane gently with additional buffer as above. Adjust back to the original volume (100λ) and use 5λ of the sample for reamplification.

EXAMPLE 4

Ligation and Cloning of PCR Amplified Sequences

## Reagents

pUC13 SmaI/Bap          A cloning vector commercially prepared by Pharmacia, dissolved in 10 mM Tris pH8.0, aliquoted and stored at -20°C.  Its

sequence and preparation are described in Vieira, J. et al., Gene 19, 259 (1982), incorporated by reference for these purposes.

5X ligation buffer      prepared from stocks
  250 mM Tris  pH7.8      aliquoted and stored at
  50 mM $MgCl_2$      -20°C.
  100 mM DTT
  5.5 mM ATP
  250 mg/ml BSA

$T_4$ DNA Ligase      New England Biolabs

SOC media

Final
Concentration

| | Final Concentration | |
|---|---|---|
| Bactotryptone | 2% | – To 97 ml distilled $H_2O$, add bacto-tryptone, yeast extract, NaCl and KCl. Stir to dissolve, autoclave, and cool to room temperature. Make medium 20 mM in $Mg^{++}$ stock with a 2 M $Mg^{++}$ (1 M $MgCl_2 \bullet 6H_2O$ + 1 M $MgSO_4 \bullet$ |
| Yeast Extract | 0.5% | |
| NaCl | 10 mM | |
| KCl | 2.5 mM | |
| $MgCl_2$, $MgSO_4$ | 20 mM (10 mM each) | |
| Glucose | 20 mM | |
| Distilled $H_2O$ | ---- | |

7H$_2$O, filter-sterilized). Add 2$^4$M glucose stock (filter-sterilized) to make the medium 20 mM final. Filter the complete medium through a 0.2 – μm filter unit. pH should be 7.0 ± 0.1. Filter-sterilizing units should be pre-filtered with distilled H$_2$O before use to remove any toxic material from the filter.

Luria Bertani Agar + 100 μg/ml
Ampicillin — commercially prepared from REMEL. For composition, see Sambrook, J. et al., Molecular Cloning 3, A.1 (2nd Ed., 1989)

Xgal 2% in dimethyl-formamide — stored at -20°C. Xgal is 5-bromo-4-chloro-3-indolyl ß-D-galactoside.

IPTG 100 mM in H$_2$O — stored at -20°C. IPTG is isopropyl-thiogalactoside.

1). Combine 10λ of filtered PCR amplied DNA (10-20 ng/ml) with 20 ng of pUCl3 Smal/BAP and 100 units of T$_4$ DNA ligase in a final volume of 20λ.
2). Incubate at 21-25° C for 3 hours.
3). Transform 100λ of tranformation competent bacteria using 10λ of ligation buffer.
4). Incubate on ice for 30 minutes in sample tubes.

5). Heat shock tubes for 45 seconds at 42°C.

6). Reincubate on ice for 2 minutes before adding 1.0 ml of SOC media (21-25°C).

7). Incubate 1 hour at 37°C shaking at 225 RPM's.

8). Pellet cells in 1.5 ml plastic tubes for 10 seconds at maximum speed.

9). Remove the media except about 100λ. Care should be taken removing the media as the pellet is loose.

10). Resuspend the cells in the remaining 100λ and spread on an L agar plate containing Ampicillin and onto which 100λ of Xgal and 50λ of IPTG had been previously spread.

11). Invert the incubate at 37°C. Colonies are visible after 12 hours. Blue color indication is clear after 16 hours.

EXAMPLE 5

Isolation of Plasmid DNA for Subsequent Dideoxy Sequence Analysis

Reagents

MP Buffer 1

50 mM Glucose
10 mM EDTA
25 mM Tris pH 8.0

MP Buffer 2 - made fresh for each experiment

0.2 N NaOH
1% SDS

MP Buffer 3

Potassium Acetate pH ~5.6
60 ml 5M KOAc
28.5 ml $H_2O$
11.5 ml gl. HOAc

RNase Stock

1.0 mg/ml RNase A dissolved in $H_2O$ and boiled
10 minutes

Phenol:Chloroform (50:50)

Phenol is buffer saturated with an equal volume of buffer (50 mM Tris•HCl, 100 mM NaCl, 1mM EDTA, pH 8.0)

PEG

13% Poly Ethylene Glycol (PEG-8000)

4M NaCl

95% and 70% EtOH

1). Three individual colonies from each isolate are selected at random and placed in 10 ml of L Broth. Each are grown overnight in a 50 ml conical tube shaking @ 225-250 RPM's @ 37°C.

2). Collect 9.5 ml of overnight culture at 1K for 20 minutes.

3). Dry pellet well and resuspend by vortexing in 200λ of MP 1. Transfer to a 1.5 ml plastic tube. Incubate 5 minutes @ RT.

4). Add 40λ of MP 2 and incubate 5 minutes on ice. Mix by inversion.

5). Add 300λ of MP 3 and incubate 5 minutes on ice. Mix by inversion.

6). Centrifuge 10,000 Xg for 5 minutes @ 4°C.

7). Transfer supernatant to a fresh 1.5 ml tube and add 10λ of a 1.0 μg/ml RNase A stock. Incubate 30 minutes @ 37°C.

8). Extract with an equal volume (-500λ) of buffer saturated phenol:chloroform. Split phases.

9). Transfer aqueous to a fresh tube and precipitate by adding 1.0 ml of cold EtOH. Incubate @ -70°C for 30 minutes.

10). Collect at full speed (about 10,000 Xg) for 15 minutes @ 4°C.

11). Remove EtOH and wash with 1.0 ml cold 70% EtOH. Respin for 2 minutes.

12). Remove EtOH and drain tube well. Dry pellet by inversion and then redissolve in 80λ H$_2$O.

13). Adjust sample with 20λ 4M NaCl and 100λ PEG. Incubate 30 minutes on ice.

14). Centrifuge at full speed (about 10,000 Xg) for 15 minutes @ 4°C.

15). Remove supernatant and wash pellet with 1.0 ml cold 70% EtOH. Respin for 2 minutes.

16). Remove EtOH and drain tube well. Dry pellet in speed vac. and then redissolve in 20λ H$_2$O.

EXAMPLE 6

DETERMINATION OF THE DNA SEQUENCE

Sequencing was performed by the method of Tabor, S. et al., Proc. Nat. Acad. Sci., 84, 4767 (1987). Sequencing gels were read and checked with a scanner. Amino acid sequences were deduced from DNA.

EXAMPLE 7

Preparation of Synthetic Peptides

A. The oligopeptide EE15-1 of the sequence:

```
1                   5                   10                  15
Cys Thr Arg Pro Ser Asn Asn Thr Arg Arg Gly Ile His Ile Gly
TGT ACA AGA CCC AGC AAC AAT ACA AGA AGA GGT ATA CAT ATA GGA


                    20                  25                  30
Pro Gly Arg Ala Leu Tyr Thr Thr Gly Glu Ile Thr Gly Asp Ile
CCA GGG AGA GCA CTT TAT ACA ACA GGA GAA ATA ACA GGA GAT ATA


                    35
Arg Arg Ala Tyr Cys
AGA CGA GCA TAT TGT
```

is synthesized by conventional solid-phase techniques on an automated peptide synthesizer, according to Kent, S. et al., "Modern Methods for the Chemical Synthesis of Biologically Active Peptides," in Alitalo, K. et al.(eds.), Synthetic Peptides in Biology and Medicine, Elsevier 1985, pp. 29-57.

B. Each of the peptides of the Sequence Listing is prepared by the same method.

C. Oligopeptide EE15-1 was prepared in a recombinant expression system in E. coli according to the methods of Sambrook, J. et al., Molecular Cloning 3, 17.3 et seq. Cold Spring Harbor 2nd Ed. 1988.

Every other peptide of the Sequence Listing is also prepared in a recombinant expression system in E. coli.

EXAMPLE 8

Extraction and Purification of Omp

A. First Method

All materials, reagents and equipment were sterilized by filtration, steam autoclave or ethylene oxide, as appropriate; asceptic technique was used throughout.

A 300 gm (wet weight) aliquot of 0.5% phenol inactivated cell paste of Meningococcal group B11 was suspended in 1200 mls of distilled water than suspended by stirring magnetically for 20 minutes at room temperature. The suspended cells were pelleted at 20,000 xg for 45 minutes at 5°C.

For extraction, the washed cells were suspended in 1500 mls 0.1 M Tris, 0.01 M EDTA Buffer pH 8.5 with 0.5% sodium deoxychloate (TED Buffer) and homogenized with a 500 ml Sorvall omnimixer at setting 3 for 60 seconds. The resulting suspension was transferred to ten Erlenmeyer flasks (500 ml) for extraction in a shaking water bath for 15 minutes at 56°C. The extract was centrifuged at 20,000 x g for 90 minutes at 5°C and the viscous supernatant fluid was decanted (volume = 1500 mls). The decanted fluid was very turbid and was recentrifuged to clarify further at 20,000 x g for 90 minutes at 5°C. The twice spun supernatant fluid was stored at 5°C. The extracted cell pellets were resuspended in 1500 mls TED Buffer. The suspension was extracted for 15 minutes at 56°C and recentrifuged at 20,000 x g for 90 minutes. The supernatant fluids which contained purified Omp were decanted (volume = 1500 mls) and stored at 5°C.

B. Second Method

All material, reagents, equipment and filters were sterilized by heat, filtration or ethylene oxide. One exception was the K-2 ultracentrifuge which was sanitized with a 0.5% formalin solution. Laminar flow canopies provided sterility protection during equipment connections. Aseptic techniques were followed throughout the entire operations. Overnight storage of the protein was at 2-8°C between steps. A 0.2 micron sterile filtration was conducted just before the final diafiltration to ensure product sterility.

Two 600-liter batches of Neisseria meningitidis were fermented and killed with 0.5% phenol, then concentrated to roughly 25 liters using two 10 ft² 0.2 micron polypropylene cross-flow filtration membranes. The concentrated broth then was diafiltered with 125 liters of cell wash buffer (0.11 M Sodium Chloride, 17.6 mM Sodium Phosphate Diabasic, 23.3 mM Ammonium Chloride, 1.34 mM Potassium Chloride, adjusted to pH 7 with 85% Phorphoric Acid followed by 2.03 mM Magnesium Sulfate Heptahydrate).

For extraction, an equal volume of 2X-TED buffer (0.2M Tris, 0. 02M EDTA adjusted to pH 8.5 with concentrated HCl followed with the addition of 1.0% sodium deoxycholate) was added to the cell slurry. The resulting slurry was heated to 56 ± 3°C and maintained at this temperature for 30 minutes to complete the extraction of Omp from the cells.

For further purification, the extracted cell slurry was centrifuged at 30,000 x g (18,000 rpm) in a "one-pass" flow mode in a K-ultracentrifuge, and the supernatant stream was collected. The low-speed supernatant was concentrated to 10 liters on two 0.1-micron polysulfone autoclavable hollow-fiber membranes and collected in an 18 liter sterile bottle. The filtration equipment was given two 4-liter rinses with TED buffer (0.1M Tris, 0. 01M EDTA, adjusted to pH 8.5 with concentrated HCl, followed with the addition of sodium deoxycholate to 0.5%) which was combined with the retentate. The retentate was subdivided into two or three equal parts. Each part was centrifuged at 80,000 x g (35,000 rpm) for 30 mintues. The Omp protein was pelleted, and the majority of soluble proteins, nucleic acids and endotoxins remained in the supernatant. The supernatant was discarded. The pelleted protein was resuspended by recirculating 55 ± 5°C TED buffer through the rotor. The first high-speed resuspensions were combined and subjected to a second low-speed spin. The second low-speed spin ensured that residual cell debris was removed from the product stream. The second low speed supernatant was subdivided into two or three equal parts. Each fraction was given two consecutive high-speed spins. All high-speed spins were operated under the same conditions and each further purified the Omp protein.

For sterile filtration and final diafiltration, the third high-speed resuspensions were diluted with an equal volume of TED buffer and filtered through a 0.2 micron cellulose acetate filter. When all fractions were permeated, an 8 L TED buffer rinse was used to flush the filtration system. The permeate and rinse were combined and concentrated to 3 liters on a 0.1 micron polysulfone autoclavable hollow fiber membrane. The material then was diafiltered with 15 liters of sterile pyrogen free water. The retentate was collected in a 4-liter bottle along with a 1-L rinse to give the final product. The final aqueous suspension was stored at 2-8°C, as purified Omp.

C. Third Method

Omp is purified from 0.2 M LiCl-0.1M Na Acetate, pH 5.8, extracts by ultracentrifugation, by the method of C.E. Frasch et al. J. Exp. Med. 140, 87-104 (1974), herein incorporated by reference.

EXAMPLE 9

A. Preparation of (EE15-1 Peptide)-Omp conjugate ("EE15-1-Omp" conjugate)

N-acetylhomocystaminylated outer membrane protein (Omp) of N. meningitidis from 59 mg of Omp (purified by Method B of Example 2) is prepared by the centrifugation method described in Marburg, S. et al., J. Am. Chem. Soc. 108:5282 (1986). This material (about 50 mg) is reacted at pH 8 (6.5 mL 0.1M $PO_4$ buffer) with 20 mg of N-$\Omega$-bromoacetylated EE15-1 (lyophilized) under $N_2$ for 18 hours at room temperature.

The reaction mixture is diluted to 10 mL with $H_2O$ and centrifuged for 2h, at 4°C and 43,000 rpm. The supernatant is removed, and the pellet resuspended, using a Dounce tissue homogenizer, in 10 mL of $H_2O$. This suspension is recentrifuged (as above) and the pellet resuspended in 9.5 mL of $H_2O$. A low speed spin for 1 minute in a clinical centrifuge removes a flocculent insoluble material if any. The degree of substitution can be determined and calculated by a variety of methods.

B. Preparation of Other Peptide Conjugates

By the method of Example 9A the following peptide-Omp conjugates are obtained:
$(EE15-1)_5$-Omp,
$(EE164-3)_4$-Omp,
$(EE244-1)_6$-Omp,
$(EE310-2)_8$-Omp,
$(EE311-1)_{10}$-Omp,
$(EE359-2)_{6.5}$-Omp,
$(EE360-1)_{3.3}$-Omp, and
$(EE543-1)_{4.0}$-Omp.

EXAMPLE 10

Protocol for Inoculation of Animals with the $(EE15-1)_5$-Omp Conjugate (hereinafter "EE-15-1-Omp" conjugate)

Alum is used as an adjuvant during the inoculation series. The inoculum is prepared by dissolving the EE15-1-Omp conjugate in physiologic saline at a final conjugate concentration of 100 $\mu$g/ml. Preformed alum (aluminum hydroxide gel) is added to the solution to a final level of 500 $\mu$g/ml aluminum. The conjugate is allowed to adsorb onto the alum gel for two hours at room temperature. Following adsorption, the gel with the conjugate is washed twice with physiologic saline and resuspended in the saline to a protein concentration of about 100 $\mu$g/ml.

African green monkeys are individually inoculated with four 100 mcg doses of the EE15-1-Omp conjugate adsorbed onto alum. Each dose is injected intramuscularly. The doses are delivered one or five months apart (week 0, 4, 8 and 28). The animals are bled at intervals of two or four weeks. Serum samples are prepared from each bleed to assay for the development of specific antibodies as described in the subsequent examples.

EXAMPLE 11

Analysis of Sera for Anti-Peptide IgG Antibodies

Each serum sample is analyzed by enzyme-linked immunoadsorbent assay (ELISA). Polystyrene microtiter plates are coated with 0.5 $\mu$g per well of the synthetic peptide (not conjugated to Omp) in phosphate-buffered physiological saline (PBS) at 4°C. Each well is then washed with PBS containing 0.05% TWEEN-20 (PBS-T). Test serum, diluted serially in PBS-T, is added to the peptide-containing wells and allowed to react with the adsorbed peptide for one hour at 36°C. After washing with PBS-T, alkaline phosphatase-conjugated goat anti-human IgG is added to the test wells and is allowed to react for one hour at 36°C. The wells are then washed extensively in PBS-T. Each well receives 0.1% p-nitrophenyl phosphate in 10% diethanolamine, pH 9.8, containing 0.5 mM $MgCl_2 6H_2O$. The ensuing reaction is allowed to proceed at room temperature for 30 minutes, at which time it is terminated by the addition of 3.0 N NaOH.

The greater the interaction of antibodies in the test serum with the peptide substrate, the greater is the amount of alkaline phosphatase bound onto the well. The phosphatase enzyme mediates the breakdown of p-nitrophenyl phosphate into a molecular substance which absorbs light at a wavelength of 405 nm. Hence, there exists a direct relationship between the absorbance at 405 nm of light at the end of the ELISA reaction and the amount of peptide-bound antibody.

Titers of anti-(EE15-1-Omp) antibody are thus readily determined.

EXAMPLE 12

Analysis of Sera for Activity which Specifically Neutralizes HIV Infectivity

Virus-neutralizing activity is determined with an assay described by Robertson et al., J. Virol. Methods 20: 195-202 (1988). The assay measures specific HIV-neutralizing activity in test serum. The assay is based on the observation that MT-4 cells, a human T-lymphoid cell line, are readily susceptible to infection with HIV and, after a period of virus replication, are killed as a result of the infection.

The test serum is treated at 56°C for 60 minutes prior to the assay. This treatment is required to eliminate non-specific inhibitors of HIV replication. Heat treated serum, serially diluted in RPMI-1640 cell culture medium, is mixed with a standard infection dose of HIV. The dose is determined prior to the assay as containing the smallest quantity of virus required to kill all the MT-4 cells in the assay culture after a period of 7 days. The serum-virus mixture is allowed to interact for one hour at 37°C. It then is added to $1.0 \times 10^5$ MT-4 cells suspended in RPMI-1640 growth medium supplemented with 10% fetal bovine serum. The cultures are incubated at 37°C in a 5% $CO_2$ atmosphere for 7 days.

At the end of the incubation period, a metabolic dye, DTT, is added to each culture. This dye is yellow in color upon visual inspection. In the presence of live cells, the dye is metabolically processed to a molecular species which yields a blue visual color. Neutralized HIV cannot replicate in the target MT-4 cells and therefore does not kill the cells. Hence, positive neutralization is assessed by the development of blue color following addition of the metabolic dye.

All the monkeys inoculated with the EE15-1-Omp conjugate are bled for specific HIV infectivity-neutralizing activity. Further follow-up evaluation of the same monkeys is also performed. Booster shots are also administered to ascertain renewed neutralizing titer.

While the foregoing specification teaches the principles of the present invention, with examples provided for the purpose of illustration, it will be understood that the practice of the invention encompasses all of the usual variations, adaptations, modifications, deletions or additions of procedures and protocols described herein, as come within the scope of the following claims and its equivalents.

SEQUENCE LISTING
(1) GENERAL INFORMATION:
    (i)  APPLICANT:  J.A. LEWIS ET AL.
    (ii) TITLE OF INVENTION:  NEW EMBODIMENTS OF THE
        HIV PRINCIPAL NEUTRALIZING DETERMINANT
    (iii)CORRESPONDENCE ADDRESS:  MERCK & CO., INC.
        (A)  STREET:  P.O. BOX 2000, EAST LINCOLN AVE.
        (B)  CITY:  RAHWAY
        (C)  STATE:  NEW JERSEY
        (D)  COUNTRY:  USA
        (E)  ZIP:  07065
    (iv) COMPUTER READABLE FORM:
        (A)  MEDIUM TYPE:  Diskette, 5.25 in., 360 Kb storage
        (B)  COMPUTER:  Wang PC 381
        (C)  OPERATING SYSTEM:  MS-DOS 3.30.10
        (D)  SOFTWARE:  Microsoft WORD 5.0
    (v)  CURRENT APPLICATION DATA:
        (A)  APPLICATION NUMBER:  NA
        (B)  FILING DATE:  NA
        (C)  CLASSIFICATION:  NA
    (vi) PRIOR APPLICATION DATA: NONE
        (A)  DOCUMENT NUMBER:  _____
        (B)  COUNTRY:  _____
        (C)  FILING DATE:  _____
        (D)  PUBLICATION DATE:  _____
    (vii)    ATTORNEY/AGENT INFORMATION:
        (A)  NAME:  R.D. MEREDITH
        (B)  REGISTRATION NUMBER:  30,777
        (C)  REFERENCE/DOCKET NUMBER:  18114Y
    (viii)    TELECOMMUNICATION INFORMATION:
        (A)  TELEPHONE:  201-594-4678
        (B)  TELEFAX:  201-594-4720
        (C)  TELEX:  _____
    (ix) PUBLICATION STATUS: NOT KNOWN
        (A)  AUTHORS:  _____
        (B)  TITLE:  _____
        (C)  JOURNAL:  _____
        (D)  VOLUME:  _____
        (E)  ISSUE:  _____
        (F)  PAGES:  _____
        (G)  DATE:  _____
        (H)  RELEVANT RESIDUES:
            (1)  START:  _____
            (2)  END:  _____
            (3)  BASE PAIRS:  _____
            (4)  AMINO ACIDS:  _____

(2) INFORMATION FOR SEQ ID NO: EE15-1
   (i)   SEQUENCE CHARACTERISTICS:
      (A)   LENGTH:  105
      (B)   TYPE:  Nucleic Acid
      (C)   STRANDEDNESS:  Single
      (D)   TOPOLOGY:  Linear
   (ii) KIND:cDNA to genomic RNA
   (ii) KIND (if peptide or protein):
      (A)   SEQUENCE ASSEMBLY METHOD:  Overlap
      (B)   FRAGMENT TYPE:  Internal Fragment
      (C)   HYPOTHETICAL:  _____
   (iii)     ORIGINAL SOURCE: HIV
      (E)   INDIVIDUAL
           ISOLATE:  _____
   (iv) IMMEDIATE SOURCE:
      (C)   CLONE:  _____
   (v)  POSITION IN GENOME: Within Env Gene
   (vi) PROPERTIES OF SEQUENCE:  Expresses conserved
      antigenic determinant
   (viii)   SEQUENCE DESCRIPTION:


SEQ ID NO:  EE15-1


```
1                   5                   10                  15
Cys Thr Arg Pro Ser Asn Asn Thr Arg Arg Gly Ile His Ile Gly
TGT ACA AGA CCC AGC AAC AAT ACA AGA AGA GGT ATA CAT ATA GGA


                    20                  25                  30
Pro Gly Arg Ala Leu Tyr Thr Thr Gly Glu Ile Thr Gly Asp Ile
CCA GGG AGA GCA CTT TAT ACA ACA GGA GAA ATA ACA GGA GAT ATA


                    35
Arg Arg Ala Tyr Cys
AGA CGA GCA TAT TGT
```


(2) INFORMATION FOR SEQ ID NO: EE15-2
   (i)   SEQUENCE CHARACTERISTICS:
      (A)   LENGTH:  105
      (B)   TYPE:  Nucleic Acid
      (C)   STRANDEDNESS:  Single
      (D)   TOPOLOGY:  Linear
   (ii) KIND:cDNA to genomic RNA

```
       (ii) KIND (if peptide or protein):
            (A)   SEQUENCE ASSEMBLY METHOD:  Overlap
            (B)   FRAGMENT TYPE:  Internal Fragment
            (C)   HYPOTHETICAL:  _____
       (iii)      ORIGINAL SOURCE: HIV
            (E)   INDIVIDUAL ISOLATE:  _____
       (iv) IMMEDIATE SOURCE:
            (C)   CLONE:  _____
       (v)  POSITION IN GENOME: Within Env Gene
       (vi) PROPERTIES OF SEQUENCE:  Expresses conserved antigenic
            determinant
       (viii)     SEQUENCE DESCRIPTION:


SEQ ID NO:  EE15-2


1                   5                   10                  15
Cys Thr Arg Pro Ser Asn Asn Thr Arg Arg Ser Ile Pro Ile Gly
TGT ACA AGG CCC AGC AAC AAT ACA AGA AGA AGT ATA CCT ATA GGA



                    20                  25                  30
Pro Gly Arg Ala Phe Tyr Thr Thr Gly Asp Ile Ile Gly Asp Ile
CCA GGG AGA GCC TTT TAT ACA ACA GGA GAC ATA ATA GGA GAT ATA



                    35
Arg Gln Ala His Cys
AGA CAA GCA CAT TGT


(2) INFORMATION FOR SEQ ID NO: EE15-3
    (i)   SEQUENCE CHARACTERISTICS:
          (A)   LENGTH:  105
          (B)   TYPE:  Nucleic Acid
          (C)   STRANDEDNESS:  Single
          (D)   TOPOLOGY:  Linear
    (ii) KIND: cDNA to genomic RNA
    (ii) KIND (if peptide or protein):
          (A)   SEQUENCE ASSEMBLY METHOD:  Overlap
          (B)   FRAGMENT TYPE:  Internal Fragment
          (C)   HYPOTHETICAL:  _____
    (iii)       ORIGINAL SOURCE: HIV
          (E)   INDIVIDUAL ISOLATE:  _____
    (iv) IMMEDIATE SOURCE:
          (C)   CLONE:  _____
```

(v)  POSITION IN GENOME: Within Env Gene
(vi) PROPERTIES OF SEQUENCE:  Expresses conserved antigenic
     determinant
(viii)    SEQUENCE DESCRIPTION: SEQ ID NO: __


SEQ ID NO:  EE15-3


```
 1                  5                  10                 15
Cys Thr Arg Pro Ser Asn Asn Thr Arg Arg Ser Ile Pro Ile Gly
TGT ACA AGG CCC AGC AAC AAT ACA AGA AGA AGT ATA CCT ATA GGA


                   20                 25                 30
Pro Gly Arg Ala Phe Tyr Thr Thr Gly Asp Ile Ile Gly Asp Ile
CCA GGG AGA GCC TTT TAT ACA ACA GGA GAC ATA ATA GGA GAT ATA


                   35
Arg Gln Ala His Cys
AGA CAA GCA CAT TGT
```


(2)      INFORMATION FOR SEQ ID NO: EEE37-1
         (i)      SEQUENCE CHARACTERISTICS:
                  (A)      LENGTH:  105
                  (B)      TYPE:  Nucleic Acid
                  (C)      STRANDEDNESS:  Single
                  (D)      TOPOLOGY:  Linear
         (ii)     KIND: cDNA to genomic RNA
         (ii)     KIND (if peptide or protein):
                  (A)      SEQUENCE ASSEMBLY METHOD:  Overlap
                  (B)      FRAGMENT TYPE:  Internal Fragment
                  (C)      HYPOTHETICAL:  _____
         (iii)    ORIGINAL SOURCE: HIV
                  (E)      INDIVIDUAL ISOLATE:  _____
         (iv)     IMMEDIATE SOURCE:
                  (C)      CLONE:  _____
         (v)      POSITION IN GENOME: Within Env Gene
         (vi)     PROPERTIES OF SEQUENCE:  Expresses conserved antigenic
                  determinant
         (viii)   SEQUENCE DESCRIPTION:

SEQ ID NO:  EEE37-1

```
         1                5                    10                   15
Cys Thr Arg Pro Asn Asn Asn Thr Arg Lys Arg Ile Thr MET Gly
TGT ACA AGA CCC AAC AAC AAT ACA AGA AAA AGG ATA ACT ATG GGA


                          20                   25                   30
Pro Gly Arg Val Phe Tyr Thr Thr Gly Gly Ile Ile Gly Asn Ile
CCA GGG AGA GTA TTT TAT ACA ACA GGA GGA ATA ATA GGA AAT ATA


                          35
Arg Arg Ala His Cys
AGA CGA GCA CAT TGT
```

(2)      INFORMATION FOR SEQ ID NO: EEE37-2
         (i)      SEQUENCE CHARACTERISTICS:
                  (A)      LENGTH:  105
                  (B)      TYPE:  Nucleic Acid
                  (C)      STRANDEDNESS:  Single
                  (D)      TOPOLOGY:  Linear
         (ii)     KIND: cDNA to genomic RNA
         (ii)     KIND (if peptide or protein):
                  (A)      SEQUENCE ASSEMBLY METHOD:  Overlap
                  (B)      FRAGMENT TYPE:  Internal Fragment
                  (C)      HYPOTHETICAL:  _____
         (iii)    ORIGINAL SOURCE: HIV
                  (E)      INDIVIDUAL ISOLATE:  _____
         (iv)     IMMEDIATE SOURCE:
                  (C)      CLONE:  _____
         (v)      POSITION IN GENOME: Within Env Gene
         (vi)     PROPERTIES OF SEQUENCE:  Expresses conserved antigenic
                  determinant
         (viii)   SEQUENCE DESCRIPTION:


SEQ ID NO:  EEE37-2

```
         1                5                    10                   15
Cys Thr Arg Pro Asn Asn Asn Thr Arg Lys Ser Ile Asn Ile Gly
TGT ACA AGA CCC AAC AAC AAT ACA AGA AAA AGT ATA AAT ATA GGA
```

```
                     20                    25                    30
Pro Gly Arg Ala Phe Tyr Thr Thr Gly Glu Ile Ile Gly Asp Ile
CCA GGG AGA GCA TTT TAT ACA ACA GGA GAA ATA ATA GGA GAT ATA


                     35
Arg Gln Ala His Cys
AGA CAA GCA CAT TGT
```

(2)         INFORMATION FOR SEQ ID NO: EE37-3
        (i)        SEQUENCE CHARACTERISTICS:
                   (A)        LENGTH:  105
                   (B)        TYPE:  Nucleic Acid
                   (C)        STRANDEDNESS:  Single
                   (D)        TOPOLOGY:  Linear
        (ii)       KIND: cDNA to genomic RNA
        (ii)       KIND (if peptide or protein):
                   (A)        SEQUENCE ASSEMBLY METHOD:  Overlap
                   (B)        FRAGMENT TYPE:  Internal Fragment
                   (C)        HYPOTHETICAL:  _____
        (iii)      ORIGINAL SOURCE: HIV
                   (E)        INDIVIDUAL ISOLATE:  _____
        (iv)       IMMEDIATE SOURCE:
                   (C)        CLONE:  _____
        (v)        POSITION IN GENOME: Within Env Gene
        (vi)       PROPERTIES OF SEQUENCE:  Expresses conserved antigenic
                   determinant
        (viii)     SEQUENCE DESCRIPTION:


SEQ ID NO:  EE37-3


```
    1                    5                    10                    15
Cys Thr Arg Pro Asn Asn Asn Thr Arg Lys Ser Ile Asn Ile Gly
TGT ACA AGA CCC AAC AAC AAT ACA AGA AAA AGT ATA AAT ATA GGA


                     20                    25                    30
Pro Gly Pro Gly Arg Ala Phe Tyr Thr Thr Gly Glu Ile Gly Asp
CCA GGA CCA GGG AGA GCA TTT TAT ACA ACA GGA GAA ATA GGA GAT


                     35
Ile Arg Gln Ala His Cys
ATA AGA CAA GCA CAT TGT
```

```
(2)     INFORMATION FOR SEQ ID NO: EE54-1
        (i)     SEQUENCE CHARACTERISTICS:
                (A)     LENGTH:  105
                (B)     TYPE:  Nucleic Acid
                (C)     STRANDEDNESS:  Single
                (D)     TOPOLOGY:  Linear
        (ii)    KIND: cDNA to genomic RNA
        (ii)    KIND (if peptide or protein):
                (A)     SEQUENCE ASSEMBLY METHOD:  Overlap
                (B)     FRAGMENT TYPE:  Internal Fragment
                (C)     HYPOTHETICAL:  _____
        (iii)   ORIGINAL SOURCE: HIV
                (E)     INDIVIDUAL ISOLATE:  _____
        (iv)    IMMEDIATE SOURCE:
                (C)     CLONE:  _____
        (v)     POSITION IN GENOME: Within Env Gene
        (vi)    PROPERTIES OF SEQUENCE:  Expresses conserved antigenic
                determinant
        (viii)  SEQUENCE DESCRIPTION:
```

SEQ ID NO:   EE54-1

```
 1                    5                         10                        15
Cys Thr Arg Pro Asn Asn Asn Thr Arg Lys Ser Ile Asn Ile Gly
TGT ACA AGA CCC AAC AAC AAT ACA AGA AAA AGT ATC AAT ATA GGA


                      20                        25                        30
Pro Gly Arg Ala Phe Tyr Thr Thr Gly Ala Ile Ile Gly Asp Ile
CCA GGG AGA GCA TTT TAT ACA ACA GGA GCA ATA ATA GGA GAT ATA


                      35
Arg Gln Ala His Cys
AGA CAA GCA CAT TGT


(2)     INFORMATION FOR SEQ ID NO: EEE69-1
        (i)     SEQUENCE CHARACTERISTICS:
                (A)     LENGTH:  105
                (B)     TYPE:  Nucleic Acid
                (C)     STRANDEDNESS:  Single
                (D)     TOPOLOGY:  Linear
        (ii)    KIND: cDNA to genomic RNA
```

```
(ii)    KIND (if peptide or protein):
        (A)     SEQUENCE ASSEMBLY METHOD:  Overlap
        (B)     FRAGMENT TYPE:  Internal Fragment
        (C)     HYPOTHETICAL:  _____
(iii)   ORIGINAL SOURCE: HIV
        (E)     INDIVIDUAL ISOLATE:  _____
(iv)    IMMEDIATE SOURCE:
        (C)     CLONE:  _____
(v)     POSITION IN GENOME: Within Env Gene
(vi)    PROPERTIES OF SEQUENCE:  Expresses conserved antigenic
        determinant
(viii)  SEQUENCE DESCRIPTION:
```

SEQ ID NO:  EEE69-1

```
1                  5                    10                   15
Cys Thr Arg Leu Asn Asn Asn Thr Arg Lys Ser Ile His Ile Gly
TGT ACA AGG CTC AAC AAC AAT ACA AGA AAA AGT ATA CAT ATA GGA


                   20                   25                   30
Pro Gly Arg Ala Phe Tyr Ala Thr Gly Glu Ile Ile Gly Asp Ile
CCA GGG AGA GCA TTT TAT GCA ACA GGA GAA ATA ATA GGA GAT ATA


                   35
Arg Gln Ala His Cys
AGA CAA GCA CAT TGT
```

```
(2)     INFORMATION FOR SEQ ID NO: EEE69-2
        (i)     SEQUENCE CHARACTERISTICS:
                (A)     LENGTH:  105
                (B)     TYPE:  Nucleic Acid
                (C)     STRANDEDNESS:  Single
                (D)     TOPOLOGY:  Linear
        (ii)    KIND: cDNA to genomic RNA
        (ii)    KIND (if peptide or protein):
                (A)     SEQUENCE ASSEMBLY METHOD:  Overlap
                (B)     FRAGMENT TYPE:  Internal Fragment
                (C)     HYPOTHETICAL:  _____
        (iii)   ORIGINAL SOURCE: HIV
                (E)     INDIVIDUAL ISOLATE:  _____
        (iv)    IMMEDIATE SOURCE:
                (C)     CLONE:  _____
        (v)     POSITION IN GENOME: Within Env Gene
        (vi)    PROPERTIES OF SEQUENCE:  Expresses conserved antigenic
                determinant
        (viii)  SEQUENCE DESCRIPTION:
```

SEQ ID NO:  EEE69-2

| 1 | | | | 5 | | | | | 10 | | | | | 15 |
Cys Thr Arg Leu Asn Asn Asn Thr Arg Lys Ser Ile His Ile Gly
TGT ACA AGA CTC AAC AAC AAT ACA AGA AAA AGT ATA CAT ATA GGA

| | | | | 20 | | | | | 25 | | | | | 30 |
Pro Gly Arg Ala Phe Tyr Ala Thr Gly Glu Ile Ile Gly Asp Ile
CCA GGG AGA GCA TTT TAT GCA ACA GGA GAA ATA ATA GGA GAT ATA

| | | | | 35 |
Arg Gln Ala Gln Cys
AGA CAA GCA CAG TGT

(2)      INFORMATION FOR SEQ ID NO: EE74-1
         (i)      SEQUENCE CHARACTERISTICS:
                  (A)      LENGTH:  105
                  (B)      TYPE:  Nucleic Acid
                  (C)      STRANDEDNESS:  Single
                  (D)      TOPOLOGY:  Linear
         (ii)     KIND: cDNA to genomic RNA
         (ii)     KIND (if peptide or protein):
                  (A)      SEQUENCE ASSEMBLY METHOD:  Overlap
                  (B)      FRAGMENT TYPE:  Internal Fragment
                  (C)      HYPOTHETICAL:  _____
         (iii)    ORIGINAL SOURCE: HIV
                  (E)      INDIVIDUAL ISOLATE:  _____
         (iv)     IMMEDIATE SOURCE:
                  (C)      CLONE:  _____
         (v)      POSITION IN GENOME: Within Env Gene
         (vi)     PROPERTIES OF SEQUENCE:  Expresses conserved antigenic
                  determinant
         (viii)   SEQUENCE DESCRIPTION:

SEQ ID NO:  EE74-1

| 1 | | | | 5 | | | | | 10 | | | | | 15 |
Cys Thr Arg Pro Asn Asn Asn Thr Arg Lys Ser Ile Asn Ile Gly
TGT ACA AGA CCC AAC AAC AAT ACA AGA AAA AGT ATA AAT ATA GGA

34

```
                    20                      25                      30
Pro Gly Arg Ala Phe Tyr Thr Thr Gly Asp Ile Ile Gly Asp Ile
CCA GGG AGA GCA TTT TAT ACA ACA GGA GAC ATA ATA GGA GAT ATA


                    35
Arg Gln Ala His Cys
AGA CAA GCA CAT TGT
```

(2)        INFORMATION FOR SEQ ID NO: EE74-2
    (i)        SEQUENCE CHARACTERISTICS:
        (A)        LENGTH:  105
        (B)        TYPE:  Nucleic Acid
        (C)        STRANDEDNESS:  Single
        (D)        TOPOLOGY:  Linear
    (ii)    KIND: cDNA to genomic RNA
    (ii)    KIND (if peptide or protein):
        (A)        SEQUENCE ASSEMBLY METHOD:  Overlap
        (B)        FRAGMENT TYPE:  Internal Fragment
        (C)        HYPOTHETICAL:  _____
    (iii)    ORIGINAL SOURCE: HIV
        (E)        INDIVIDUAL ISOLATE:  _____
    (iv)    IMMEDIATE SOURCE:
        (C)        CLONE:  _____
    (v)        POSITION IN GENOME: Within Env Gene
    (vi)    PROPERTIES OF SEQUENCE:  Expresses conserved antigenic
                determinant
    (viii)  SEQUENCE DESCRIPTION:


SEQ ID NO:  EE74-2


```
    1                   5                      10                      15
Cys Thr Arg Pro Ser Asn Asn Thr Arg Lys Ser Ile Asn Ile Gly
TGT ACA AGA CCC AGC AAC AAT ACA AGA AAA AGT ATA AAT ATA GGA


                    20                      25                      30
Pro Gly Arg Ala Phe Tyr Thr Thr Gly Asp Ile Ile Gly Asp Ile
CCA GGG AGA GCA TTT TAT ACC ACA GGA GAC ATA ATA GGA GAT ATA


                    35
Arg Gln Ala His Cys
AGA CAA GCA CAT TGT
```

```
(2)      INFORMATION FOR SEQ ID NO: EE74-3
         (i)       SEQUENCE CHARACTERISTICS:
                   (A)      LENGTH:  105
                   (B)      TYPE:  Nucleic Acid
                   (C)      STRANDEDNESS:  Single
                   (D)      TOPOLOGY:  Linear
         (ii)      KIND: cDNA to genomic RNA
         (ii)      KIND (if peptide or protein):
                   (A)      SEQUENCE ASSEMBLY METHOD:  Overlap
                   (B)      FRAGMENT TYPE:  Internal Fragment
                   (C)      HYPOTHETICAL:  _____
         (iii)     ORIGINAL SOURCE: HIV
                   (E)      INDIVIDUAL ISOLATE:  _____
         (iv)      IMMEDIATE SOURCE:
                   (C)      CLONE:  _____
         (v)       POSITION IN GENOME: Within Env Gene
         (vi)      PROPERTIES OF SEQUENCE:  Expresses conserved antigenic
                   determinant
         (viii)    SEQUENCE DESCRIPTION:


SEQ ID NO:   EE74-3


     1                 5                      10                    15
Cys Thr Arg Pro Asn Asn Asn Thr Arg Lys Ser Ile Asn Ile Gly
TGT ACA AGA CCC AAC AAC AAT ACA AGA AAA AGT ATA AAT ATA GGA


                      20                     25                    30
Pro Gly Arg Ala Phe Tyr Thr Thr Gly Asp Ile Ile Gly Asp Ile
CCA GGG AGA GCA TTT TAT ACA ACA GGA GAC ATA ATA GGA GAT ATA


                      35
Arg Gln Ala His Cys
AGA CAA GCA CAT TGT


(2)      INFORMATION FOR SEQ ID NO: EEE90-1
         (i)       SEQUENCE CHARACTERISTICS:
                   (A)      LENGTH:  105
                   (B)      TYPE:  Nucleic Acid
                   (C)      STRANDEDNESS:  Single
                   (D)      TOPOLOGY:  Linear
         (ii)      KIND: cDNA to genomic RNA
```

```
(ii)     KIND (if peptide or protein):
         (A)     SEQUENCE ASSEMBLY METHOD:  Overlap
         (B)     FRAGMENT TYPE:  Internal Fragment
         (C)     HYPOTHETICAL:  _____
(iii)    ORIGINAL SOURCE: HIV
         (E)     INDIVIDUAL ISOLATE:  _____
(iv)     IMMEDIATE SOURCE:
         (C)     CLONE:  _____
(v)      POSITION IN GENOME: Within Env Gene
(vi)     PROPERTIES OF SEQUENCE:  Expresses conserved antigenic
         determinant
(viii)   SEQUENCE DESCRIPTION:
```

SEQ ID NO:  EEE90-1

```
1                 5                      10                    15
Cys Thr Arg Pro Asn Asn Asn Thr Arg Lys Ser Ile His Ile Ala
TGT ACA AGA CCC AAC AAC AAT ACA AGA AAA AGT ATA CAT ATA GCA


                  20                     25                    30
Pro Gly Arg Ala Phe Tyr Ala Thr Gly Glu Ile Ile Gly Asp Ile
CCA GGG AGA GCA TTT TAC GCA ACA GGA GAA ATA ATA GGA GAT ATA


                  35
Arg Gln Ala His Cys
AGA CAA GCA CAT TGT
```

```
(2)      INFORMATION FOR SEQ ID NO: EE90-2
         (i)     SEQUENCE CHARACTERISTICS:
                 (A)     LENGTH:  105
                 (B)     TYPE:  Nucleic Acid
                 (C)     STRANDEDNESS:  Single
                 (D)     TOPOLOGY:  Linear
         (ii)    KIND: cDNA to genomic RNA
         (ii)    KIND (if peptide or protein):
                 (A)     SEQUENCE ASSEMBLY METHOD:  Overlap
                 (B)     FRAGMENT TYPE:  Internal Fragment
                 (C)     HYPOTHETICAL:  _____
         (iii)   ORIGINAL SOURCE: HIV
                 (E)     INDIVIDUAL ISOLATE:  _____
         (iv)    IMMEDIATE SOURCE:
                 (C)     CLONE:  _____
         (v)     POSITION IN GENOME: Within Env Gene
         (vi)    PROPERTIES OF SEQUENCE:  Expresses conserved antigenic
                 determinant
         (viii)  SEQUENCE DESCRIPTION:
```

SEQ ID NO: EE90-2

| 1 | | | | 5 | | | | | 10 | | | | | 15 |
Cys Thr Arg Pro Asn Asn Asn Thr Arg Lys Ser Ile His Ile Ala
TGT ACA AGA CCC AAC AAC AAT ACA AGA AAA AGT ATA CAT ATA GCA

| | | | | 20 | | | | | 25 | | | | | 30 |
Pro Gly Arg Ala Phe Tyr Ala Thr Gly Glu Ile Ile Gly Asp Ile
CCA GGG AGA GCA TTT TAC GCA ACA GGA GAA ATA ATA GGA GAT ATA

| | | | | 35 |
Arg Gln Ala His Cys
AGA CAA GCA CAT TGT

(2)     INFORMATION FOR SEQ ID NO: EE90-3
        (i)     SEQUENCE CHARACTERISTICS:
                (A)     LENGTH:  105
                (B)     TYPE:  Nucleic Acid
                (C)     STRANDEDNESS:  Single
                (D)     TOPOLOGY:  Linear
        (ii)    KIND: cDNA to genomic RNA
        (ii)    KIND (if peptide or protein):
                (A)     SEQUENCE ASSEMBLY METHOD:  Overlap
                (B)     FRAGMENT TYPE:  Internal Fragment
                (C)     HYPOTHETICAL:  _____
        (iii)   ORIGINAL SOURCE: HIV
                (E)     INDIVIDUAL ISOLATE:  _____
        (iv)    IMMEDIATE SOURCE:
                (C)     CLONE:  _____
        (v)     POSITION IN GENOME: Within Env Gene
        (vi)    PROPERTIES OF SEQUENCE:  Expresses conserved antigenic
                determinant
        (viii)  SEQUENCE DESCRIPTION:

SEQ ID NO:  EE90-3

| 1 | | | | 5 | | | | | 10 | | | | | 15 |
Cys Thr Arg Pro Asn Asn Asn Thr Arg Lys Ser Ile His Ile Ala
TGT ACA AGA CCC AAC AAC AAT ACA AGA AAA AGT ATA CAT ATA GCA

38

```
                        20                      25                      30
Pro Gly Arg Ala Phe Tyr Ala Thr Gly Glu Ile Ile Gly Asp Ile
CCA GGG AGA GCA TTT TAC GCA ACA GGA GAA ATA ATA GGA GAT ATA


                        35
Arg Gln Ala His Cys
AGA CAA GCA CAT TGT
```

```
(2)       INFORMATION FOR SEQ ID NO: EE100-1
          (i)      SEQUENCE CHARACTERISTICS:
                   (A)     LENGTH:  105
                   (B)     TYPE:  Nucleic Acid
                   (C)     STRANDEDNESS:  Single
                   (D)     TOPOLOGY:  Linear
          (ii)     KIND: cDNA to genomic RNA
          (ii)     KIND (if peptide or protein):
                   (A)     SEQUENCE ASSEMBLY METHOD:  Overlap
                   (B)     FRAGMENT TYPE:  Internal Fragment
                   (C)     HYPOTHETICAL:  _____
          (iii)    ORIGINAL SOURCE: HIV
                   (E)     INDIVIDUAL ISOLATE:  _____
          (iv)     IMMEDIATE SOURCE:
                   (C)     CLONE:  _____
          (v)      POSITION IN GENOME: Within Env Gene
          (vi)     PROPERTIES OF SEQUENCE:  Expresses conserved antigenic
                   determinant
          (viii)   SEQUENCE DESCRIPTION:
```

```
SEQ ID NO:  EE100-1


1                   5                       10                      15
Cys Thr Arg Pro His Asn Asn Thr Arg Lys Ser Ile His Ile Gly
TGC ACA AGA CCC CAC AAC AAT ACA AGG AAA AGT ATA CAT ATA GGA


                        20                      25                      30
Pro Gly Arg Ala Phe Tyr Thr Thr Gly Ala Ile Ile Gly Asp Ile
CCA GGG AGA GCA TTT TAT ACA ACA GGA GCA ATA ATA GGA GAT ATA


                        35
Arg Gln Ala Tyr Cys
AGA CAA GCA TAT TGT
```

(2)    INFORMATION FOR SEQ ID NO: EEE100-2
    (i)    SEQUENCE CHARACTERISTICS:
        (A)    LENGTH: 105
        (B)    TYPE: Nucleic Acid
        (C)    STRANDEDNESS: Single
        (D)    TOPOLOGY: Linear
    (ii)    KIND: cDNA to genomic RNA
    (ii)    KIND (if peptide or protein):
        (A)    SEQUENCE ASSEMBLY METHOD: Overlap
        (B)    FRAGMENT TYPE: Internal Fragment
        (C)    HYPOTHETICAL: _____
    (iii)    ORIGINAL SOURCE: HIV
        (E)    INDIVIDUAL ISOLATE: _____
    (iv)    IMMEDIATE SOURCE:
        (C)    CLONE: _____
    (v)    POSITION IN GENOME: Within Env Gene
    (vi)    PROPERTIES OF SEQUENCE: Expresses conserved antigenic
        determinant
    (viii) SEQUENCE DESCRIPTION:


SEQ ID NO: EEE100-2


```
 1                   5                       10                      15
Cys Thr Arg Pro Gly Asn Asn Thr Arg Lys Ser Ile His Ile Gly
TGC ACA AGA CCC GGC AAC AAT ACA AGG AAA AGT ATA CAT ATA GGA


                     20                      25                      30
Pro Gly Arg Ala Phe Tyr Thr Thr Gly Asp Ile Ile Gly Asp Ile
CCA GGG AGA GCA TTT TAT ACA ACA GGA GAT ATA ATA GGA GAT ATA


                     35
Arg Gln Ala His Cys
AGA CAA GCA CAT TGT
```


(2)    INFORMATION FOR SEQ ID NO: EE100-3
    (i)    SEQUENCE CHARACTERISTICS:
        (A)    LENGTH: 105
        (B)    TYPE: Nucleic Acid
        (C)    STRANDEDNESS: Single
        (D)    TOPOLOGY: Linear
    (ii)    KIND: cDNA to genomic RNA

```
(ii)     KIND (if peptide or protein):
         (A)     SEQUENCE ASSEMBLY METHOD:  Overlap
         (B)     FRAGMENT TYPE:  Internal Fragment
         (C)     HYPOTHETICAL:  _____
(iii)    ORIGINAL SOURCE: HIV
         (E)     INDIVIDUAL ISOLATE:  _____
(iv)     IMMEDIATE SOURCE:
         (C)     CLONE:  _____
(v)      POSITION IN GENOME: Within Env Gene
(vi)     PROPERTIES OF SEQUENCE:  Expresses conserved antigenic
         determinant
(viii)   SEQUENCE DESCRIPTION:
```

SEQ ID NO:  EE100-3

```
1                5                    10                   15
Cys Thr Arg Pro His Asn Asn Thr Arg Lys Ser Ile His Ile Gly
TGC ACA AGA CCC CAC AAC AAT ACA AGG AAA AGT ATA CAT ATA GGA


1                5                    10                   15
Pro Gly Arg Ala Trp Tyr Thr Thr Gly Ala Ile Ile Gly Asp Ile
CCA GGG AGA GCA TGG TAT ACA ACA GGA GCA ATA ATA GGA GAT ATA


                 35
Arg Gln Ala Tyr Cys
AGA CAA GCA TAT TGT
```

```
(2)      INFORMATION FOR SEQ ID NO: EE125-1
         (i)      SEQUENCE CHARACTERISTICS:
                  (A)     LENGTH:  105
                  (B)     TYPE:  Nucleic Acid
                  (C)     STRANDEDNESS:  Single
                  (D)     TOPOLOGY:  Linear
         (ii)     KIND: cDNA to genomic RNA
         (ii)     KIND (if peptide or protein):
                  (A)     SEQUENCE ASSEMBLY METHOD:  Overlap
                  (B)     FRAGMENT TYPE:  Internal Fragment
                  (C)     HYPOTHETICAL:  _____
         (iii)    ORIGINAL SOURCE: HIV
                  (E)     INDIVIDUAL ISOLATE:  _____
         (iv)     IMMEDIATE SOURCE:
                  (C)     CLONE:  _____
         (v)      POSITION IN GENOME: Within Env Gene
         (vi)     PROPERTIES OF SEQUENCE:  Expresses conserved antigenic
                  determinant
         (viii)   SEQUENCE DESCRIPTION:
```

SEQ ID NO:  EE125-1

```
1                 5                      10                    15
Cys Thr Arg Pro Asn Asn Asn Thr Arg Lys Gly Ile His Leu Gly
TGT ACA AGA CCC AAC AAC AAT ACA AGA AAA GGT ATA CAT CTA GGA


                  20                     25                    30
Pro Gly Arg Ala Phe Tyr Ala Thr Gly Glu Ile Ile Gly Asp Ile
CCA GGG AGA GCA TTT TAT GCA ACA GGA GAA ATA ATA GGA GAT ATA


                  35
Arg Gln Ala His Cys
AGA CAA GCA CAT TGT
```

(2)      INFORMATION FOR SEQ ID NO: EE125-2
         (i)      SEQUENCE CHARACTERISTICS:
                  (A)      LENGTH:  105
                  (B)      TYPE:  Nucleic Acid
                  (C)      STRANDEDNESS:  Single
                  (D)      TOPOLOGY:  Linear
         (ii)     KIND: cDNA to genomic RNA
         (ii)     KIND (if peptide or protein):
                  (A)      SEQUENCE ASSEMBLY METHOD:  Overlap
                  (B)      FRAGMENT TYPE:  Internal Fragment
                  (C)      HYPOTHETICAL:  _____
         (iii)    ORIGINAL SOURCE: HIV
                  (E)      INDIVIDUAL ISOLATE:  _____
         (iv)     IMMEDIATE SOURCE:
                  (C)      CLONE:  _____
         (v)      POSITION IN GENOME: Within Env Gene
         (vi)     PROPERTIES OF SEQUENCE:  Expresses conserved antigenic
                  determinant
         (viii)   SEQUENCE DESCRIPTION:


SEQ ID NO:  EE125-2

```
1                 5                      10                    15
Cys Thr Arg Pro Asn Asn Asn Thr Arg Lys Gly Ile His Leu Gly
TGT ACA AGA CCC AAC AAC AAT ACA AGA AAA GGT ATA CAT CTA GGA
```

42

```
                        20                      25                      30
Pro Gly Lys Ala Phe Tyr Ala Thr Gly Glu Ile Ile Gly Asp Ile
CCA GGA AAA GCA TTT TAT GCA ACA GGA GAA ATA ATA GGA GAT ATA


                        35
Arg Gln Ala His Cys
AGA CAA GCA CAT TGT
```

(2)      INFORMATION FOR SEQ ID NO: EEE125-3
         (i)      SEQUENCE CHARACTERISTICS:
                  (A)      LENGTH:  105
                  (B)      TYPE:  Nucleic Acid
                  (C)      STRANDEDNESS:  Single
                  (D)      TOPOLOGY:  Linear
         (ii)     KIND: cDNA to genomic RNA
         (ii)     KIND (if peptide or protein):
                  (A)      SEQUENCE ASSEMBLY METHOD:  Overlap
                  (B)      FRAGMENT TYPE:  Internal Fragment
                  (C)      HYPOTHETICAL:  _____
         (iii)    ORIGINAL SOURCE: HIV
                  (E)      INDIVIDUAL ISOLATE:  _____
         (iv)     IMMEDIATE SOURCE:
                  (C)      CLONE:  _____
         (v)      POSITION IN GENOME: Within Env Gene
         (vi)     PROPERTIES OF SEQUENCE:  Expresses conserved antigenic
                  determinant
         (viii)   SEQUENCE DESCRIPTION:


SEQ ID NO:  EEE125-3


```
1                       5                       10                      15
Cys Thr Arg Pro Asn Asn Asn Thr Arg Lys Gly Ile His Leu Gly
TGT ACA AGA CCC AAC AAC AAT ACA AGA AAA GGT ATA CAT CTA GGA


                        20                      25                      30
Pro Gly Arg Ala Phe Tyr Ala Thr Gly Glu Ile Ile Gly Asp Ile
CCA GGG AGA GCA TTT TAT GCA ACA GGA GAA ATA ATA GGA GAT ATA


                        35
Arg Gln Ala His Cys
AGA CAA GCA CAT TGT
```

(2)    INFORMATION FOR SEQ ID NO: EE131-1
   (i)     SEQUENCE CHARACTERISTICS:
        (A)     LENGTH:  105
        (B)     TYPE:  Nucleic Acid
        (C)     STRANDEDNESS:  Single
        (D)     TOPOLOGY:  Linear
   (ii)   KIND: cDNA to genomic RNA
   (ii)   KIND (if peptide or protein):
        (A)     SEQUENCE ASSEMBLY METHOD:  Overlap
        (B)     FRAGMENT TYPE:  Internal Fragment
        (C)     HYPOTHETICAL:  _____
   (iii)  ORIGINAL SOURCE: HIV
        (E)     INDIVIDUAL ISOLATE:  _____
   (iv)   IMMEDIATE SOURCE:
        (C)     CLONE:  _____
   (v)    POSITION IN GENOME: Within Env Gene
   (vi)   PROPERTIES OF SEQUENCE:  Expresses conserved antigenic
        determinant
   (viii) SEQUENCE DESCRIPTION:


SEQ ID NO:  EE131-1


```
 1                 5                       10                      15
Cys Thr Arg Pro Asn Asn Asn Thr Ser Lys Arg Ile Ser Ile Gly
TGT ACA AGA CCC AAC AAC AAT ACA AGC AAA AGA ATA TCT ATA GGA


                   20                      25                      30
Pro Gly Arg Ala Phe Arg Ala Thr Arg Ile Ile Gly Asp Ile Arg
CCA GGG AGA GCT TTT CGT GCA ACA AGA ATA ATA GGA GAT ATA AGA


                   35
Gln Ala His Cys
CAA GCA CAT TGT
```


(2)    INFORMATION FOR SEQ ID NO: EE131-2
   (i)     SEQUENCE CHARACTERISTICS:
        (A)     LENGTH:  105
        (B)     TYPE:  Nucleic Acid
        (C)     STRANDEDNESS:  Single
        (D)     TOPOLOGY:  Linear
   (ii)   KIND: cDNA to genomic RNA
   (ii)   KIND (if peptide or protein):
        (A)     SEQUENCE ASSEMBLY METHOD:  Overlap
        (B)     FRAGMENT TYPE:  Internal Fragment

```
                    (C)      HYPOTHETICAL:  _____
          (iii)     ORIGINAL SOURCE: HIV
                    (E)      INDIVIDUAL ISOLATE:  _____
          (iv)      IMMEDIATE SOURCE:
                    (C)      CLONE:  _____
          (v)       POSITION IN GENOME: Within Env Gene
          (vi)      PROPERTIES OF SEQUENCE:  Expresses conserved antigenic
                    determinant
          (viii)    SEQUENCE DESCRIPTION:
```

SEQ ID NO:  EE131-2

```
   1                5                        10                      15
Cys Thr Arg Pro Asn Asn Asn Thr Ser Lys Arg Ile Ser Ile Gly
TGT ACA AGA CCC AAC AAC AAT ACA AGT AAA AGA ATA TCT ATA GGA


                    20                       25                      30
Pro Gly MET Ala Phe Arg Ala Thr Arg Ile Ile Gly Asp Ile Arg
CCA GGG ATG GCA TTT CGT GCA ACA AGA ATA ATA GGA GAT ATA AGA


                    35
Gln Ala His Cys
CAA GCA CAT TGT
```

```
(2)       INFORMATION FOR SEQ ID NO: EE131-3
          (i)       SEQUENCE CHARACTERISTICS:
                    (A)      LENGTH:  105
                    (B)      TYPE:  Nucleic Acid
                    (C)      STRANDEDNESS:  Single
                    (D)      TOPOLOGY:  Linear
          (ii)      KIND: cDNA to genomic RNA
          (ii)      KIND (if peptide or protein):
                    (A)      SEQUENCE ASSEMBLY METHOD:  Overlap
                    (B)      FRAGMENT TYPE:  Internal Fragment
                    (C)      HYPOTHETICAL:  _____
          (iii)     ORIGINAL SOURCE: HIV
                    (E)      INDIVIDUAL ISOLATE:  _____
          (iv)      IMMEDIATE SOURCE:
                    (C)      CLONE:  _____
          (v)       POSITION IN GENOME: Within Env Gene
          (vi)      PROPERTIES OF SEQUENCE:  Expresses conserved antigenic
                    determinant
          (viii)    SEQUENCE DESCRIPTION:
```

SEQ ID NO:  EE131-3

```
         1              5                   10                  15
         Cys Thr Arg Pro Asn Asn Asn Thr Ser Lys Arg Ile Ser Ile Gly
         TGT ACA AGA CCC AAC AAC AAT ACA AGC AAA AGA ATA TCT ATA GGA


                        20                  25                  30
         Pro Gly Arg Ala Phe Arg Ala Thr Arg Ile Ile Gly Asp Ile Arg
         CCA GGG AGA GCA TTT CGT GCA ACA AGA ATA ATA GGA GAT ATA AGA


                        35
         Gln Ala His Cys
         CAA GCA CAT TGT
```

(2)      INFORMATION FOR SEQ ID NO: EEE149-1
         (i)      SEQUENCE CHARACTERISTICS:
                  (A)      LENGTH:  105
                  (B)      TYPE:  Nucleic Acid
                  (C)      STRANDEDNESS:  Single
                  (D)      TOPOLOGY:  Linear
         (ii)     KIND: cDNA to genomic RNA
         (ii)     KIND (if peptide or protein):
                  (A)      SEQUENCE ASSEMBLY METHOD:  Overlap
                  (B)      FRAGMENT TYPE:  Internal Fragment
                  (C)      HYPOTHETICAL:  _____
         (iii)    ORIGINAL SOURCE: HIV
                  (E)      INDIVIDUAL ISOLATE:  _____
         (iv)     IMMEDIATE SOURCE:
                  (C)      CLONE:  _____
         (v)      POSITION IN GENOME: Within Env Gene
         (vi)     PROPERTIES OF SEQUENCE:  Expresses conserved antigenic
                  determinant
         (viii)   SEQUENCE DESCRIPTION:


SEQ ID NO:  EEE149-1

```
         1              5                   10                  15
         Cys Thr Arg Pro Asn Asn Asn Thr Arg Arg Gly Ile Ser Ile Gly
         TGT ACA AGA CCC AAC AAC AAT ACA AGA AGG GGT ATA AGT ATA GGA
```

```
                        20                  25                  30
Pro Gly Arg Ala Phe Val Tyr Ala Thr Lys Ile Ile Gly Asp Ile
CCA GGG AGA GCA TTT GTT TAT GCA ACA AAA ATA ATA GGA GAT ATA



                        35
Arg Gln Ala His Cys
AGA CAA GCA CAT TGT
```

(2)        INFORMATION FOR SEQ ID NO: EE149-2
           (i)      SEQUENCE CHARACTERISTICS:
                    (A)      LENGTH:  105
                    (B)      TYPE:  Nucleic Acid
                    (C)      STRANDEDNESS:  Single
                    (D)      TOPOLOGY:  Linear
           (ii)     KIND: cDNA to genomic RNA
           (ii)     KIND (if peptide or protein):
                    (A)      SEQUENCE ASSEMBLY METHOD:  Overlap
                    (B)      FRAGMENT TYPE:  Internal Fragment
                    (C)      HYPOTHETICAL:  _____
           (iii)    ORIGINAL SOURCE: HIV
                    (E)      INDIVIDUAL ISOLATE:  _____
           (iv)     IMMEDIATE SOURCE:
                    (C)      CLONE:  _____
           (v)      POSITION IN GENOME: Within Env Gene
           (vi)     PROPERTIES OF SEQUENCE:  Expresses conserved antigenic
                    determinant
           (viii)   SEQUENCE DESCRIPTION:

SEQ ID NO:  EE149-2


```
 1                   5                  10                  15
Cys Thr Arg Pro Asn Asn Asn Thr Arg Arg Gly Ile Ser Ile Gly
TGT ACA AGA CCC AAC AAC AAT ACA AGA AGG GGT ATA AGT ATA GGA



                        20                  25                  30
Pro Gly Arg Ala Phe Val Tyr Ala Thr Lys Ile Ile Gly Asp Ile
CCA GGG AGA GCA TTT GTT TAT GCA ACA AAA ATA ATA GGA GAT ATA



                        35
Arg Gln Ala His Cys
AGA CAA GCA CAT TGT
```

(2)     INFORMATION FOR SEQ ID NO: EE149-3
        (i)     SEQUENCE CHARACTERISTICS:
                (A)     LENGTH:  105
                (B)     TYPE:  Nucleic Acid
                (C)     STRANDEDNESS:  Single
                (D)     TOPOLOGY:  Linear
        (ii)    KIND: cDNA to genomic RNA
        (ii)    KIND (if peptide or protein):
                (A)     SEQUENCE ASSEMBLY METHOD:  Overlap
                (B)     FRAGMENT TYPE:  Internal Fragment
                (C)     HYPOTHETICAL:  _____
        (iii)   ORIGINAL SOURCE: HIV
                (E)     INDIVIDUAL ISOLATE:  _____
        (iv)    IMMEDIATE SOURCE:
                (C)     CLONE:  _____
        (v)     POSITION IN GENOME: Within Env Gene
        (vi)    PROPERTIES OF SEQUENCE:  Expresses conserved antigenic
                determinant
        (viii)  SEQUENCE DESCRIPTION:


SEQ ID NO:  EE149-3


    1                   5                   10                  15
Cys Thr Arg Pro Asn Asn Asn Thr Arg Arg Gly Ile Ser Ile Gly
TGT ACA AGA CCC AAC AAC AAT ACA AGA AGG GGT ATA AGT ATA GGA


                    20                  25                  30
Pro Gly Arg Ala Phe Val Tyr Ala Thr Lys Ile Ile Gly Asp Ile
CCA GGG AGA GCA TTT GTT TAT GCA ACA AAA ATA ATA GGA GAT ATA


                    35
Arg Gln Ala His Cys
AGA CAA GCA CAT TGT


(2)     INFORMATION FOR SEQ ID NO: EEE159-1
        (i)     SEQUENCE CHARACTERISTICS:
                (A)     LENGTH:  105
                (B)     TYPE:  Nucleic Acid
                (C)     STRANDEDNESS:  Single
                (D)     TOPOLOGY:  Linear
        (ii)    KIND: cDNA to genomic RNA
        (ii)    KIND (if peptide or protein):
                (A)     SEQUENCE ASSEMBLY METHOD:  Overlap
                (B)     FRAGMENT TYPE:  Internal Fragment

```
            (C)      HYPOTHETICAL: _____
     (iii)  ORIGINAL SOURCE: HIV
            (E)      INDIVIDUAL ISOLATE: _____
     (iv)   IMMEDIATE SOURCE:
            (C)      CLONE: _____
     (v)    POSITION IN GENOME: Within Env Gene
     (vi)   PROPERTIES OF SEQUENCE:  Expresses conserved antigenic
            determinant
     (viii) SEQUENCE DESCRIPTION:
```

SEQ ID NO:  EEE159-1

```
  1                   5                   10                    15
Cys Thr Arg Pro Ser Asn Asn Thr Arg Lys Ser Ile His Ile Gly
TGT ACA AGA CCC AGC AAC AAT ACA AGA AAA AGT ATA CAT ATA GGA


                    20                  25                    30
Pro Gly Arg Ala Phe Tyr Ala Thr Gly Glu Ile Ile Gly Asp Ile
CCA GGG AGA GCA TTT TAT GCA ACA GGA GAA ATA ATA GGA GAT ATA


                    35
Arg Gln Ala His Cys
AGA CAA GCA CAT TGT
```

```
(2)      INFORMATION FOR SEQ ID NO: EEE159-2
         (i)      SEQUENCE CHARACTERISTICS:
                  (A)      LENGTH:  105
                  (B)      TYPE:  Nucleic Acid
                  (C)      STRANDEDNESS:  Single
                  (D)      TOPOLOGY:  Linear
         (ii)     KIND: cDNA to genomic RNA
         (ii)     KIND (if peptide or protein):
                  (A)      SEQUENCE ASSEMBLY METHOD:  Overlap
                  (B)      FRAGMENT TYPE:  Internal Fragment
                  (C)      HYPOTHETICAL: _____
         (iii)    ORIGINAL SOURCE: HIV
                  (E)      INDIVIDUAL ISOLATE: _____
         (iv)     IMMEDIATE SOURCE:
                  (C)      CLONE: _____
         (v)      POSITION IN GENOME: Within Env Gene
         (vi)     PROPERTIES OF SEQUENCE:  Expresses conserved antigenic
                  determinant
         (viii)   SEQUENCE DESCRIPTION:
```

49

SEQ ID NO: EEE159-2

```
     1                   5                        10                       15
Cys Thr Arg Pro Asn Asn Asn Thr Arg Lys Ser Ile Pro Ile Gly
TGT ACA AGA CCC AAC AAC AAT ACA AGG AAA AGT ATA CCT ATA GGA


                         20                       25                       30
Pro Gly Arg Ala Phe Tyr Ala Thr Gly Asp Ile Ile Gly Asp Ile
CCA GGG AGA GCA TTT TAT GCA ACA GGA GAC ATA ATA GGA GAT ATA


                         35
Arg Gln Ala His Cys
AGA CAA GCA CAT TGT
```

```
(2)        INFORMATION FOR SEQ ID NO: EE159-3
           (i)      SEQUENCE CHARACTERISTICS:
                    (A)      LENGTH:  105
                    (B)      TYPE:  Nucleic Acid
                    (C)      STRANDEDNESS:  Single
                    (D)      TOPOLOGY:  Linear
           (ii)     KIND: cDNA to genomic RNA
           (ii)     KIND (if peptide or protein):
                    (A)      SEQUENCE ASSEMBLY METHOD:  Overlap
                    (B)      FRAGMENT TYPE:  Internal Fragment
                    (C)      HYPOTHETICAL:  _____
           (iii)    ORIGINAL SOURCE: HIV
                    (E)      INDIVIDUAL ISOLATE:  _____
           (iv)     IMMEDIATE SOURCE:
                    (C)      CLONE:  _____
           (v)      POSITION IN GENOME: Within Env Gene
           (vi)     PROPERTIES OF SEQUENCE:  Expresses conserved antigenic
                    determinant
           (viii)   SEQUENCE DESCRIPTION:
```

SEQ ID NO: EE159-3

```
     1                   5                        10                       15
TGT ACA AGA CCC AGC AAC AAT ACA AGA AAA AGT ATA CAT ATA GGA
Cys Thr Arg Pro Ser Asn Asn Thr Arg Lys Ser Ile His Ile Gly


                         20                       25                       30
CCA GGG AGA GCA TTT TAT GCA ACA GGA GAA ATA ATA GGA GAT ATA
Pro Gly Arg Ala Phe Tyr Ala Thr Gly Glu Ile Ile Gly Asp Ile
```

50

```
                    35
AGA CAA GCA CAT TGT
Arg Gln Ala His Cys
```

(2)      INFORMATION FOR SEQ ID NO: EE164-1
         (i)      SEQUENCE CHARACTERISTICS:
                  (A)      LENGTH:  105
                  (B)      TYPE:  Nucleic Acid
                  (C)      STRANDEDNESS:  Single
                  (D)      TOPOLOGY:  Linear
         (ii)     KIND: cDNA to genomic RNA
         (ii)     KIND (if peptide or protein):
                  (A)      SEQUENCE ASSEMBLY METHOD:  Overlap
                  (B)      FRAGMENT TYPE:  Internal Fragment
                  (C)      HYPOTHETICAL:  _____
         (iii)    ORIGINAL SOURCE: HIV
                  (E)      INDIVIDUAL ISOLATE:  _____
         (iv)     IMMEDIATE SOURCE:
                  (C)      CLONE:  _____
         (v)      POSITION IN GENOME: Within Env Gene
         (vi)     PROPERTIES OF SEQUENCE:  Expresses conserved antigenic
                  determinant
         (viii)   SEQUENCE DESCRIPTION:


SEQ ID NO:  EE164-1


```
1                   5                        10                       15
Cys Thr Arg Pro Ser Asn Asn Thr Ser Lys Gly Ile His Ile Gly
TGT ACA AGA CCC AGC AAC AAT ACA AGC AAA GGT ATA CAT ATA GGA


                    20                       25                       30
Pro Gly Arg Ala Phe Tyr Thr Thr Gly Asn Ile Ile Gly Asn Ile
CCA GGG AGA GCA TTT TAT ACA ACA GGA AAT ATA ATA GGA AAT ATA


                    35
Arg Gln Ala His Cys
AGA CAA GCA CAT TGT
```


(2)      INFORMATION FOR SEQ ID NO: EE164-2
         (i)      SEQUENCE CHARACTERISTICS:
                  (A)      LENGTH:  105
                  (B)      TYPE:  Nucleic Acid

```
              (C)      STRANDEDNESS:  Single
              (D)      TOPOLOGY:  Linear
      (ii)    KIND: cDNA to genomic RNA
      (ii)    KIND (if peptide or protein):
              (A)      SEQUENCE ASSEMBLY METHOD:  Overlap
              (B)      FRAGMENT TYPE:  Internal Fragment
              (C)      HYPOTHETICAL:  _____
      (iii)   ORIGINAL SOURCE: HIV
              (E)      INDIVIDUAL ISOLATE:  _____
      (iv)    IMMEDIATE SOURCE:
              (C)      CLONE:  _____
      (v)     POSITION IN GENOME: Within Env Gene
      (vi)    PROPERTIES OF SEQUENCE:  Expresses conserved antigenic
              determinant
      (viii)  SEQUENCE DESCRIPTION:


  SEQ ID NO:  EE164-2


  1                 5                      10                     15
  Cys Thr Arg Pro Asn Asn Asn Thr Ser Arg Gly Ile His Ile Gly
  TGT ACA AGA CCC AAC AAC AAT ACA AGC AGA GGT ATA CAT ATA GGA


                    20                     25                     30
  Pro Gly Arg Ala Phe Tyr Ala Thr Gly Asn Ile Ile Gly Asp Ile
  CCA GGG AGA GCA TTT TAT GCA ACA GGA AAT ATA ATA GGA GAT ATA


                    35
  Arg Arg Ala His Cys
  AGA CGA GCA CAT TGT


  (2)     INFORMATION FOR SEQ ID NO: EEE164-3
          (i)      SEQUENCE CHARACTERISTICS:
                   (A)      LENGTH:  105
                   (B)      TYPE:  Nucleic Acid
                   (C)      STRANDEDNESS:  Single
                   (D)      TOPOLOGY:  Linear
          (ii)     KIND: cDNA to genomic RNA
          (ii)     KIND (if peptide or protein):
                   (A)      SEQUENCE ASSEMBLY METHOD:  Overlap
                   (B)      FRAGMENT TYPE:  Internal Fragment
                   (C)      HYPOTHETICAL:  _____
```

```
(iii)    ORIGINAL SOURCE: HIV
         (E)      INDIVIDUAL ISOLATE: _____
(iv)     IMMEDIATE SOURCE:
         (C)      CLONE: _____
(v)      POSITION IN GENOME: Within Env Gene
(vi)     PROPERTIES OF SEQUENCE:  Expresses conserved antigenic
         determinant
(viii)   SEQUENCE DESCRIPTION:
```

SEQ ID NO: EEE164-3

```
 1                5                        10                       15
Cys Thr Arg Pro Ser Asn Asn Thr Arg Lys Gly Ile His Ile Gly
TGT ACA AGA CCC AGC AAC AAT ACA AGA AAA GGT ATA CAT ATA GGA


                 20                       25                       30
Pro Gly Arg Ala Phe Tyr Thr Thr Gly Gln Ile Ile Gly Asp Ile
CCA GGG AGA GCA TTT TAT ACA ACA GGA CAA ATA ATA GGA GAT ATA


                 35
Arg Gln Ala His Cys
AGA CAA GCA CAT TGT
```

```
(2)      INFORMATION FOR SEQ ID NO: EE179-1
         (i)      SEQUENCE CHARACTERISTICS:
                  (A)      LENGTH:  105
                  (B)      TYPE:  Nucleic Acid
                  (C)      STRANDEDNESS:  Single
                  (D)      TOPOLOGY:  Linear
         (ii)     KIND: cDNA to genomic RNA
         (ii)     KIND (if peptide or protein):
                  (A)      SEQUENCE ASSEMBLY METHOD:  Overlap
                  (B)      FRAGMENT TYPE:  Internal Fragment
                  (C)      HYPOTHETICAL: _____
         (iii)    ORIGINAL SOURCE: HIV
                  (E)      INDIVIDUAL ISOLATE: _____
         (iv)     IMMEDIATE SOURCE:
                  (C)      CLONE: _____
         (v)      POSITION IN GENOME: Within Env Gene
         (vi)     PROPERTIES OF SEQUENCE:  Expresses conserved antigenic
                  determinant
         (viii)   SEQUENCE DESCRIPTION:
```

SEQ ID NO: EE179-1

```
         1                     5                        10                        15
       Cys Thr Arg Pro Asn Asn Asn Thr Arg Lys Ser Ile His Ile Gly
       TGT ACA AGA CCC AAC AAC AAT ACA AGA AAA AGT ATA CAT ATA GGA


                               20                       25                        30
       Pro Gly Arg Ala Phe Tyr Thr Thr Gly Glu Ile Ile Gly Asn Ile
       CCA GGG AGA GCA TTT TAT ACA ACA GGA GAA ATA ATA GGA AAT ATA


                               35
       Arg Gln Ala His Cys
       AGA CAA GCA CAC TGT
```

(2)      INFORMATION FOR SEQ ID NO: EE179-2
         (i)      SEQUENCE CHARACTERISTICS:
                  (A)      LENGTH:  105
                  (B)      TYPE:  Nucleic Acid
                  (C)      STRANDEDNESS:  Single
                  (D)      TOPOLOGY:  Linear
         (ii)     KIND: cDNA to genomic RNA
         (ii)     KIND (if peptide or protein):
                  (A)      SEQUENCE ASSEMBLY METHOD:  Overlap
                  (B)      FRAGMENT TYPE:  Internal Fragment
                  (C)      HYPOTHETICAL:  _____
         (iii)    ORIGINAL SOURCE: HIV
                  (E)      INDIVIDUAL ISOLATE:  _____
         (iv)     IMMEDIATE SOURCE:
                  (C)      CLONE:  _____
         (v)      POSITION IN GENOME: Within Env Gene
         (vi)     PROPERTIES OF SEQUENCE:  Expresses conserved antigenic
                  determinant
         (viii)   SEQUENCE DESCRIPTION:


SEQ ID NO: EE179-2

```
         1                     5                        10                        15
       Cys Thr Arg Pro Ser Asn Asn Thr Arg Lys Ser Ile His Ile Gly
       TGT ACA AGA CCC AGC AAC AAT ACA AGA AAA AGT ATA CAT ATA GGA
```

```
                        20                      25                      30
     Pro Gly Arg Ala Phe Tyr Thr Thr Gly Glu Ile Ile Glu Asn Ile
     CCA GGG AGA GCA TTT TAT ACA ACA GGA GAA ATA ATA GAA AAT ATA


                        35
     Arg Gln Ala His Cys
     AGA CAA GCA CAC TGT
```

(2)       INFORMATION FOR SEQ ID NO: EE179-3
          (i)      SEQUENCE CHARACTERISTICS:
                   (A)      LENGTH:  105
                   (B)      TYPE:  Nucleic Acid
                   (C)      STRANDEDNESS:  Single
                   (D)      TOPOLOGY:  Linear
          (ii)     KIND: cDNA to genomic RNA
          (ii)     KIND (if peptide or protein):
                   (A)      SEQUENCE ASSEMBLY METHOD:  Overlap
                   (B)      FRAGMENT TYPE:  Internal Fragment
                   (C)      HYPOTHETICAL:  _____
          (iii)    ORIGINAL SOURCE: HIV
                   (E)      INDIVIDUAL ISOLATE:  _____
          (iv)     IMMEDIATE SOURCE:
                   (C)      CLONE:  _____
          (v)      POSITION IN GENOME: Within Env Gene
          (vi)     PROPERTIES OF SEQUENCE:  Expresses conserved antigenic
                   determinant
          (viii)   SEQUENCE DESCRIPTION:


     SEQ ID NO:   EE179-3


```
     1                       5                      10                      15
     Cys Thr Arg Pro Asn Asn Asn Thr Arg Lys Ser Ile His Ile Gly
     TGT ACA AGA CCC AAC AAC AAT ACA AGA AAA AGT ATA CAT ATA GGA


                        20                      25                      30
     Pro Gly Arg Ala Phe Tyr Thr Thr Gly Glu Ile Ile Gly Asn Ile
     CCA GGG AGA GCA TTT TAT ACA ACA GGA GAA ATA ATA GGA AAT ATA


                        35
     Arg Gln Ala His Cys
     AGA CAA GCA CAC TGT
```

```
(2)        INFORMATION FOR SEQ ID NO: EEE181-1
           (i)      SEQUENCE CHARACTERISTICS:
                    (A)      LENGTH:  105
                    (B)      TYPE:  Nucleic Acid
                    (C)      STRANDEDNESS:  Single
                    (D)      TOPOLOGY:  Linear
           (ii)    KIND: cDNA to genomic RNA
           (ii)    KIND (if peptide or protein):
                    (A)      SEQUENCE ASSEMBLY METHOD:  Overlap
                    (B)      FRAGMENT TYPE:  Internal Fragment
                    (C)      HYPOTHETICAL:  _____
           (iii)   ORIGINAL SOURCE: HIV
                    (E)      INDIVIDUAL ISOLATE:  _____
           (iv)    IMMEDIATE SOURCE:
                    (C)      CLONE:  _____
           (v)     POSITION IN GENOME: Within Env Gene
           (vi)    PROPERTIES OF SEQUENCE:  Expresses conserved antigenic
                   determinant
           (viii)  SEQUENCE DESCRIPTION:


SEQ ID NO:  EEE181-1


      1                 5                   10                  15
      Cys Thr Arg Pro Asn Asn Asn Thr Arg Lys Ser Ile His Ile Gly
      TGT ACA AGA CCC AAC AAT AAT ACA AGA AAA AGT ATA CAT ATA GGA


                        20                  25                  30
      Pro Gly Arg Ala Phe Tyr Thr Thr Gly Glu Ile Ile Gly Asn Ile
      CCA GGG AGA GCA TTT TAT ACA ACG GGA GAA ATA ATA GGA AAT ATA


                        35
      Arg Gln Ala His Cys
      AGA CAA GCA CAT TGT


(2)        INFORMATION FOR SEQ ID NO: EE181-2
           (i)      SEQUENCE CHARACTERISTICS:
                    (A)      LENGTH:  105
                    (B)      TYPE:  Nucleic Acid
                    (C)      STRANDEDNESS:  Single
                    (D)      TOPOLOGY:  Linear
           (ii)    KIND: cDNA to genomic RNA
```

(ii)     KIND (if peptide or protein):
         (A)     SEQUENCE ASSEMBLY METHOD:  Overlap
         (B)     FRAGMENT TYPE:  Internal Fragment
         (C)     HYPOTHETICAL:  _____
(iii)    ORIGINAL SOURCE: HIV
         (E)     INDIVIDUAL ISOLATE:  _____
(iv)     IMMEDIATE SOURCE:
         (C)     CLONE:  _____
(v)      POSITION IN GENOME: Within Env Gene
(vi)     PROPERTIES OF SEQUENCE:  Expresses conserved antigenic
         determinant
(viii)   SEQUENCE DESCRIPTION:


SEQ ID NO:  EE181-2


```
1                   5                   10                  15
Cys Thr Arg Pro Asn Asn Asn Thr Arg Lys Ser Ile His Ile Gly
TGT ACA AGA CCC AAC AAC AAT ACA AGA AAA AGT ATA CAT ATA GGA


                    20                  25                  30
Pro Gly Arg Ala Phe Tyr Thr Thr Gly Glu Ile Ile Gly Asn Ile
CCA GGG AGA GCA TTT TAT ACA ACG GGA GAA ATA ATA GGA AAT ATA


                    35
Arg Gln Ala His Cys
AGA CAA GCA CAT TGT
```


(2)      INFORMATION FOR SEQ ID NO: EE181-3
         (i)     SEQUENCE CHARACTERISTICS:
                 (A)     LENGTH:  105
                 (B)     TYPE:  Nucleic Acid
                 (C)     STRANDEDNESS:  Single
                 (D)     TOPOLOGY:  Linear
         (ii)    KIND: cDNA to genomic RNA
         (ii)    KIND (if peptide or protein):
                 (A)     SEQUENCE ASSEMBLY METHOD:  Overlap
                 (B)     FRAGMENT TYPE:  Internal Fragment
                 (C)     HYPOTHETICAL:  _____
         (iii)   ORIGINAL SOURCE: HIV
                 (E)     INDIVIDUAL ISOLATE:  _____
         (iv)    IMMEDIATE SOURCE:
                 (C)     CLONE:  _____
         (v)     POSITION IN GENOME: Within Env Gene
         (vi)    PROPERTIES OF SEQUENCE:  Expresses conserved antigenic
                 determinant
         (viii)  SEQUENCE DESCRIPTION:

SEQ ID NO:  EE181-3

```
1              5                        10                       15
Cys Thr Arg Pro Asn Asn Asn Thr Arg Lys Ser Ile His Ile Gly
TGT ACA AGA CCC AAC AAT AAT ACA AGA AAA AGT ATA CAT ATA GGA


                20                       25                       30
Pro Gly Arg Ala Phe Tyr Thr Thr Gly Gly Ile Ile Gly Asp Ile
CCA GGG AGA GCA TTT TAT ACA ACG GGA GGA ATA ATA GGA GAT ATA


                35
Arg Gln Ala His Cys
AGA CAA GCA CAT TGT
```

(2)      INFORMATION FOR SEQ ID NO: EE211-1
         (i)      SEQUENCE CHARACTERISTICS:
                  (A)      LENGTH:  105
                  (B)      TYPE:  Nucleic Acid
                  (C)      STRANDEDNESS:  Single
                  (D)      TOPOLOGY:  Linear
         (ii)     KIND: cDNA to genomic RNA
         (ii)     KIND (if peptide or protein):
                  (A)      SEQUENCE ASSEMBLY METHOD:  Overlap
                  (B)      FRAGMENT TYPE:  Internal Fragment
                  (C)      HYPOTHETICAL:  _____
         (iii)    ORIGINAL SOURCE: HIV
                  (E)      INDIVIDUAL ISOLATE:  _____
         (iv)     IMMEDIATE SOURCE:
                  (C)      CLONE:  _____
         (v)      POSITION IN GENOME: Within Env Gene
         (vi)     PROPERTIES OF SEQUENCE:  Expresses conserved antigenic
                  determinant
         (viii)   SEQUENCE DESCRIPTION:


SEQ ID NO:  EE211-1

```
1              5                        10                       15
Cys Thr Arg Pro Asn Asp Asn Thr Arg Arg Ser Ile Asn Ile Gly
TGT ACA AGA CCC AAC GAC AAT ACA AGA AGA AGT ATA AAT ATA GGA
```

```
                  20                    25                    30
Pro Gly Arg Ala Phe Tyr Ala Thr Gly Glu Ile Ile Gly Asn Ile
CCA GGG AGA GCC TTT TAT GCA ACA GGA GAA ATA ATA GGA AAT ATA


                  35
Arg Gln Ala His Cys
AGA CAA GCA CAT TGT
```

(2)      INFORMATION FOR SEQ ID NO: EEE211-2
         (i)      SEQUENCE CHARACTERISTICS:
                  (A)      LENGTH:  105
                  (B)      TYPE:  Nucleic Acid
                  (C)      STRANDEDNESS:  Single
                  (D)      TOPOLOGY:  Linear
         (ii)     KIND: cDNA to genomic RNA
         (ii)     KIND (if peptide or protein):
                  (A)      SEQUENCE ASSEMBLY METHOD:  Overlap
                  (B)      FRAGMENT TYPE:  Internal Fragment
                  (C)      HYPOTHETICAL:  _____
         (iii)    ORIGINAL SOURCE: HIV
                  (E)      INDIVIDUAL ISOLATE:  _____
         (iv)     IMMEDIATE SOURCE:
                  (C)      CLONE:  _____
         (v)      POSITION IN GENOME: Within Env Gene
         (vi)     PROPERTIES OF SEQUENCE:  Expresses conserved antigenic
                  determinant
         (viii)   SEQUENCE DESCRIPTION:


SEQ ID NO:  EEE211-2


```
 1                   5                    10                    15
Cys Thr Arg Pro Asn Asn Asn Thr Arg Lys Ser Ile Ser Leu Gly
TGT ACA AGA CCC AAC AAC AAT ACA AGA AAA AGT ATA TCT CTA GGA


                  20                    25                    30
Pro Gly Ser Ala Ile Tyr Ala Thr Gly Asp Ile Ile Gly Asp Ile
CCA GGG AGT GCA ATT TAT GCA ACA GGA GAC ATA ATA GGA GAT ATA


                  35
Arg Gln Ala His Cys
AGA CAA GCA CAT TGT
```

59

```
(2)      INFORMATION FOR SEQ ID NO: EE215-1
         (i)      SEQUENCE CHARACTERISTICS:
                  (A)      LENGTH:  105
                  (B)      TYPE:  Nucleic Acid
                  (C)      STRANDEDNESS:  Single
                  (D)      TOPOLOGY:  Linear
         (ii)     KIND: cDNA to genomic RNA
         (ii)     KIND (if peptide or protein):
                  (A)      SEQUENCE ASSEMBLY METHOD:  Overlap
                  (B)      FRAGMENT TYPE:  Internal Fragment
                  (C)      HYPOTHETICAL:  _____
         (iii)    ORIGINAL SOURCE: HIV
                  (E)      INDIVIDUAL ISOLATE:  _____
         (iv)     IMMEDIATE SOURCE:
                  (C)      CLONE:  _____
         (v)      POSITION IN GENOME: Within Env Gene
         (vi)     PROPERTIES OF SEQUENCE:  Expresses conserved antigenic
                  determinant
         (viii)   SEQUENCE DESCRIPTION:
```

SEQ ID NO:   EE215-1

```
1                 5                      10                     15
Cys Ile Arg Pro Asn Asn Asn Thr Arg Lys Ser Ile His Ile Gly
TGT ATA AGA CCC AAC AAC AAT ACA AGA AAA AGT ATA CAT ATA GGA


                  20                     25                     30
Pro Gly Arg Ala Phe Tyr Thr Thr Gly Asp Ile Ile Gly Asp Ile
CCA GGG AGA GCA TTT TAT ACA ACA GGA GAT ATA ATA GGA GAT ATA


                  35
Arg Gln Ala His Cys
AGA CAA GCG CAT TGT
```

```
(2)      INFORMATION FOR SEQ ID NO: EE215-2
         (i)      SEQUENCE CHARACTERISTICS:
                  (A)      LENGTH:  105
                  (B)      TYPE:  Nucleic Acid
                  (C)      STRANDEDNESS:  Single
                  (D)      TOPOLOGY:  Linear
         (ii)     KIND: cDNA to genomic RNA
```

```
(ii)    KIND (if peptide or protein):
        (A)     SEQUENCE ASSEMBLY METHOD:  Overlap
        (B)     FRAGMENT TYPE:  Internal Fragment
        (C)     HYPOTHETICAL:  _____
(iii)   ORIGINAL SOURCE: HIV
        (E)     INDIVIDUAL ISOLATE:  _____
(iv)    IMMEDIATE SOURCE:
        (C)     CLONE:  _____
(v)     POSITION IN GENOME: Within Env Gene
(vi)    PROPERTIES OF SEQUENCE:  Expresses conserved antigenic
        determinant
(viii)  SEQUENCE DESCRIPTION:
```

SEQ ID NO:  EE215-2

```
1               5                       10                  15
Cys Ile Arg Pro Asn Asn Asn Thr Arg Lys Ser Ile His Ile Gly
TGT ATA AGA CCC AAC AAC AAT ACA AGA AAA AGT ATA CAT ATA GGA


                20                      25                  30
Pro Gly Arg Ala Phe Tyr Thr Thr Gly Asp Ile Ile Gly Asp Ile
CCA GGG AGA GCA TTT TAT ACA ACA GGA GAT ATA ATA GGA GAT ATA


                35
Arg Gln Ala His Cys
AGA CAA GCA CAT TGT
```

```
(2)     INFORMATION FOR SEQ ID NO: EE215-3
        (i)     SEQUENCE CHARACTERISTICS:
                (A)     LENGTH:  105
                (B)     TYPE:  Nucleic Acid
                (C)     STRANDEDNESS:  Single
                (D)     TOPOLOGY:  Linear
        (ii)    KIND: cDNA to genomic RNA
        (ii)    KIND (if peptide or protein):
                (A)     SEQUENCE ASSEMBLY METHOD:  Overlap
                (B)     FRAGMENT TYPE:  Internal Fragment
                (C)     HYPOTHETICAL:  _____
        (iii)   ORIGINAL SOURCE: HIV
                (E)     INDIVIDUAL ISOLATE:  _____
        (iv)    IMMEDIATE SOURCE:
                (C)     CLONE:  _____
        (v)     POSITION IN GENOME: Within Env Gene
        (vi)    PROPERTIES OF SEQUENCE:  Expresses conserved antigenic
                determinant
        (viii)  SEQUENCE DESCRIPTION:
```

SEQ ID NO:  EE215-3


```
1                   5                        10                       15
Cys Ile Arg Pro Asn Asn Asn Thr Arg Lys Ser Ile His Ile Gly
TGT ATA AGA CCC AAC AAC AAT ACA AGA AAA AGT ATA CAT ATA GGA


                    20                       25                       30
Pro Gly Arg Ala Phe Tyr Thr Thr Gly Thr Ile Ile Gly Asp Ile
CCA GGG AGA GCA TTT TAT ACA ACA GGA ACA ATA ATA GGA GAT ATA


                    35
Arg Gln Ala His Cys
AGA CAA GCA CAT TGT
```


(2)      INFORMATION FOR SEQ ID NO: EEE217-1
         (i)     SEQUENCE CHARACTERISTICS:
                 (A)     LENGTH:  105
                 (B)     TYPE:  Nucleic Acid
                 (C)     STRANDEDNESS:  Single
                 (D)     TOPOLOGY:  Linear
         (ii)    KIND: cDNA to genomic RNA
         (ii)    KIND (if peptide or protein):
                 (A)     SEQUENCE ASSEMBLY METHOD:  Overlap
                 (B)     FRAGMENT TYPE:  Internal Fragment
                 (C)     HYPOTHETICAL:  _____
         (iii)   ORIGINAL SOURCE: HIV
                 (E)     INDIVIDUAL ISOLATE:  _____
         (iv)    IMMEDIATE SOURCE:
                 (C)     CLONE:  _____
         (v)     POSITION IN GENOME: Within Env Gene
         (vi)    PROPERTIES OF SEQUENCE:  Expresses conserved antigenic
                 determinant
         (viii)  SEQUENCE DESCRIPTION:


SEQ ID NO:  EEE217-1


```
1                   5                        10                       15
Cys Thr Arg Pro Asn Asn Asn Thr Arg Arg Gly Ile Ser Ile Gly
TGT ACA AGA CCC AAC AAC AAT ACA AGA AGG GGT ATA AGT ATA GGA
```


62

```
                        20                    25                    30
Pro Gly Arg Ala Phe Val Tyr Ala Thr Lys Ile Ile Gly Asp Ile
CCA GGG AGA GCA TTT GTT TAT GCA ACA AAA ATA ATA GGA GAT ATA


                        35
Arg Gln Ala His Cys
AGA CAA GCA CAT TGT
```

(2)      INFORMATION FOR SEQ ID NO: EE217-2
         (i)     SEQUENCE CHARACTERISTICS:
                 (A)      LENGTH:  105
                 (B)      TYPE:  Nucleic Acid
                 (C)      STRANDEDNESS:  Single
                 (D)      TOPOLOGY:  Linear
         (ii)    KIND: cDNA to genomic RNA
         (ii)    KIND (if peptide or protein):
                 (A)      SEQUENCE ASSEMBLY METHOD:  Overlap
                 (B)      FRAGMENT TYPE:  Internal Fragment
                 (C)      HYPOTHETICAL:  _____
         (iii)   ORIGINAL SOURCE: HIV
                 (E)      INDIVIDUAL ISOLATE:  _____
         (iv)    IMMEDIATE SOURCE:
                 (C)      CLONE:  _____
         (v)     POSITION IN GENOME: Within Env Gene
         (vi)    PROPERTIES OF SEQUENCE:  Expresses conserved antigenic
                 determinant
         (viii)  SEQUENCE DESCRIPTION:


SEQ ID NO:  EE217-2


```
 1                   5                    10                    15
Cys Thr Arg Pro Asn Asn Asn Thr Arg Lys Ser Ile Thr Ile Gly
TGT ACA AGA CCC AAT AAC AAT ACA AGA AAA AGT ATA ACT ATA GGA


                        20                    25                    30
Pro Gly Arg Ala Phe Tyr Ala Thr Gly Glu Ile Ile Gly Asp Ile
CCA GGG AGA GCA TTT TAT GCA ACA GGA GAA ATA ATA GGA GAT ATA


                        35
Arg Gln Ala His Cys
AGA CAA GCA CAT TGT
```

(2)      INFORMATION FOR SEQ ID NO: EE228-1
       (i)      SEQUENCE CHARACTERISTICS:
            (A)     LENGTH:  105
            (B)     TYPE:  Nucleic Acid
            (C)     STRANDEDNESS:  Single
            (D)     TOPOLOGY:  Linear
       (ii)   KIND: cDNA to genomic RNA
       (ii)   KIND (if peptide or protein):
            (A)     SEQUENCE ASSEMBLY METHOD:  Overlap
            (B)     FRAGMENT TYPE:  Internal Fragment
            (C)     HYPOTHETICAL:  _____
       (iii)  ORIGINAL SOURCE: HIV
            (E)     INDIVIDUAL ISOLATE:  _____
       (iv)   IMMEDIATE SOURCE:
            (C)     CLONE:  _____
       (v)    POSITION IN GENOME: Within Env Gene
       (vi)   PROPERTIES OF SEQUENCE:  Expresses conserved antigenic
            determinant
       (viii) SEQUENCE DESCRIPTION:


SEQ ID NO:  EE228-1


```
 1               5                    10                      15
Cys Thr Arg Pro Asn Asn Asn Thr Arg Lys Ser Ile Pro Ile Gly
TGT ACA AGA CCC AAC AAC AAT ACA AGA AAA AGT ATA CCT ATA GGA


                20                   25                      30
Pro Gly Arg Ala Phe Tyr Thr Thr Gly Asp Ile Ile Gly Asp Ile
CCA GGG AGA GCA TTT TAT ACA ACA GGA GAT ATA ATA GGA GAT ATA


                35
Arg Gln Ala His Cys
AGA CAA GCA CAT TGT
```


(2)      INFORMATION FOR SEQ ID NO: EE228-2
       (i)      SEQUENCE CHARACTERISTICS:
            (A)     LENGTH:  105
            (B)     TYPE:  Nucleic Acid
            (C)     STRANDEDNESS:  Single
            (D)     TOPOLOGY:  Linear
       (ii)   KIND: cDNA to genomic RNA

```
(ii)    KIND (if peptide or protein):
        (A)     SEQUENCE ASSEMBLY METHOD:  Overlap
        (B)     FRAGMENT TYPE:  Internal Fragment
        (C)     HYPOTHETICAL:   _____
(iii)   ORIGINAL SOURCE: HIV
        (E)     INDIVIDUAL ISOLATE:  _____
(iv)    IMMEDIATE SOURCE:
        (C)     CLONE:  _____
(v)     POSITION IN GENOME: Within Env Gene
(vi)    PROPERTIES OF SEQUENCE:  Expresses conserved antigenic
        determinant
(viii)  SEQUENCE DESCRIPTION:
```

SEQ ID NO:  EE228-2

```
1               5                   10                  15
Cys Thr Arg Pro Asn Asn Asn Thr Arg Lys Ser Ile Pro Ile Gly
TGT ACA AGA CCC AAC AAC AAT ACA AGA AAA AGT ATA CCT ATA GGA


                20                  25                  30
Pro Gly Arg Ala Phe Tyr Thr Thr Gly Asp Ile Ile Gly Asp Ile
CCA GGG AGA GCA TTT TAT ACA ACA GGA GAT ATA ATA GGA GAT ATA


                35
Arg Gln Ala His Cys
AGA CAA GCA CAT TGT
```

```
(2)     INFORMATION FOR SEQ ID NO: EE228-3
        (i)     SEQUENCE CHARACTERISTICS:
                (A)     LENGTH:  105
                (B)     TYPE:  Nucleic Acid
                (C)     STRANDEDNESS:  Single
                (D)     TOPOLOGY:  Linear
        (ii)    KIND: cDNA to genomic RNA
        (ii)    KIND (if peptide or protein):
                (A)     SEQUENCE ASSEMBLY METHOD:  Overlap
                (B)     FRAGMENT TYPE:  Internal Fragment
                (C)     HYPOTHETICAL:   _____
        (iii)   ORIGINAL SOURCE: HIV
                (E)     INDIVIDUAL ISOLATE:  _____
        (iv)    IMMEDIATE SOURCE:
                (C)     CLONE:  _____
        (v)     POSITION IN GENOME: Within Env Gene
        (vi)    PROPERTIES OF SEQUENCE:  Expresses conserved antigenic
                determinant
        (viii)  SEQUENCE DESCRIPTION:
```

SEQ ID NO: EE228-3

```
          1                    5                    10                   15
          Cys Thr Arg Pro Asn Asn Asn Thr Arg Lys Ser Ile Pro Ile Gly
          TGT ACA AGA CCC AAC AAC AAT ACA AGA AAA AGT ATA CCT ATA GGA


                               20                   25                   30
          Pro Gly Arg Ala Phe Tyr Thr Thr Gly Asp Ile Ile Gly Asp Ile
          CCA GGG AGA GCA TTT TAT ACA ACA GGA GAT ATA ATA GGA GAT ATA


                               35
          Arg Gln Ala His Cys
          AGA CAA GCA CAT TGT
```

```
(2)       INFORMATION FOR SEQ ID NO: EE229-1
          (i)       SEQUENCE CHARACTERISTICS:
                    (A)       LENGTH:  102
                    (B)       TYPE:  Nucleic Acid
                    (C)       STRANDEDNESS:  Single
                    (D)       TOPOLOGY:  Linear
          (ii)      KIND: cDNA to genomic RNA
          (ii)      KIND (if peptide or protein):
                    (A)       SEQUENCE ASSEMBLY METHOD:  Overlap
                    (B)       FRAGMENT TYPE:  Internal Fragment
                    (C)       HYPOTHETICAL:  _____
          (iii)     ORIGINAL SOURCE: HIV
                    (E)       INDIVIDUAL ISOLATE:  _____
          (iv)      IMMEDIATE SOURCE:
                    (C)       CLONE:  _____
          (v)       POSITION IN GENOME: Within Env Gene
          (vi)      PROPERTIES OF SEQUENCE:  Expresses conserved antigenic
                    determinant
          (viii)    SEQUENCE DESCRIPTION:
```

SEQ ID NO: EE229-1

```
          1                    5                    10                   15
          Cys Thr Arg Pro Asn Asn Asn Thr Arg Arg Ser Ile His Ile Gly
          TGT ACA AGA CCC AAT AAC AAT ACA AGA AGA AGT ATA CAT ATA GGA
```

```
                        20                  25                  30
Pro Gly Arg Ala Phe Tyr Ala Thr Asp Ile Ile Gly Asn Ile Arg
CCA GGG AGA GCA TTT TAT GCA ACA GAT ATA ATA GGA AAT ATA AGA


                        35
Gln Ala His Cys
CAA GCA CAT TGT
```

(2)        INFORMATION FOR SEQ ID NO: EE229-2
           (i)      SEQUENCE CHARACTERISTICS:
                    (A)      LENGTH:  102
                    (B)      TYPE:  Nucleic Acid
                    (C)      STRANDEDNESS:  Single
                    (D)      TOPOLOGY:  Linear
           (ii)     KIND: cDNA to genomic RNA
           (ii)     KIND (if peptide or protein):
                    (A)      SEQUENCE ASSEMBLY METHOD:  Overlap
                    (B)      FRAGMENT TYPE:  Internal Fragment
                    (C)      HYPOTHETICAL:  _____
           (iii)    ORIGINAL SOURCE: HIV
                    (E)      INDIVIDUAL ISOLATE:  _____
           (iv)     IMMEDIATE SOURCE:
                    (C)      CLONE:  _____
           (v)      POSITION IN GENOME: Within Env Gene
           (vi)     PROPERTIES OF SEQUENCE:  Expresses conserved antigenic
                    determinant
           (viii)   SEQUENCE DESCRIPTION:


SEQ ID NO:  EE229-2

```
1                   5                   10                  15
Cys Thr Arg Pro Gly Asn Asn Thr Arg Lys Gly Ile His Ile Gly
TGT ACA AGA CCC GGC AAC AAT ACA AGA AAA GGT ATA CAT ATA GGA


                        20                  25                  30
Pro Gly Arg Ala Ile Tyr Ala Thr Asp Ile Ile Gly Asp Ile Arg
CCA GGG AGA GCA ATT TAT GCA ACA GAT ATA ATA GGA GAT ATA AGA


                        35
Gln Ala His Cys
CAA GCA CAT TGT
```

67

```
(2)      INFORMATION FOR SEQ ID NO: EE229-3
         (i)      SEQUENCE CHARACTERISTICS:
                  (A)      LENGTH:  102
                  (B)      TYPE:  Nucleic Acid
                  (C)      STRANDEDNESS:  Single
                  (D)      TOPOLOGY:  Linear
         (ii)   KIND: cDNA to genomic RNA
         (ii)   KIND (if peptide or protein):
                  (A)      SEQUENCE ASSEMBLY METHOD:  Overlap
                  (B)      FRAGMENT TYPE:  Internal Fragment
                  (C)      HYPOTHETICAL:  _____
         (iii)  ORIGINAL SOURCE: HIV
                  (E)      INDIVIDUAL ISOLATE:  _____
         (iv)   IMMEDIATE SOURCE:
                  (C)      CLONE:  _____
         (v)    POSITION IN GENOME: Within Env Gene
         (vi)   PROPERTIES OF SEQUENCE:  Expresses conserved antigenic
                determinant
         (viii) SEQUENCE DESCRIPTION:
```

SEQ ID NO:  EE229-3

```
1                5                          10                       15
Cys Thr Arg Pro Gly Asn Asn Thr Arg Lys Gly Ile His Ile Gly
TGT ACA AGA CCC GGC AAC AAT ACA AGA AAA GGT ATA CAT ATA GGA


                 20                         25                       30
Pro Gly Arg Ala Ile Tyr Ala Thr Asp Ile Ile Gly Asp Ile Arg
CCA GGG AGA GCA ATT TAT GCA ACA GAT ATA ATA GGA GAT ATA AGA


                 35
Gln Ala His Cys
CAA GCA CAT TGT
```

```
(2)      INFORMATION FOR SEQ ID NO: EEE244-1
         (i)      SEQUENCE CHARACTERISTICS:
                  (A)      LENGTH:  102
                  (B)      TYPE:  Nucleic Acid
                  (C)      STRANDEDNESS:  Single
                  (D)      TOPOLOGY:  Linear
         (ii)   KIND: cDNA to genomic RNA
```

```
(ii)     KIND (if peptide or protein):
         (A)     SEQUENCE ASSEMBLY METHOD:  Overlap
         (B)     FRAGMENT TYPE:  Internal Fragment
         (C)     HYPOTHETICAL:  _____
(iii)    ORIGINAL SOURCE: HIV
         (E)     INDIVIDUAL ISOLATE:  _____
(iv)     IMMEDIATE SOURCE:
         (C)     CLONE:  _____
(v)      POSITION IN GENOME: Within Env Gene
(vi)     PROPERTIES OF SEQUENCE:  Expresses conserved antigenic
         determinant
(viii)   SEQUENCE DESCRIPTION:
```

SEQ ID NO:  EE244-1

```
1               5                   10                  15
Cys Thr Arg Pro Asn Asn Asn Ile Lys Ile Arg Ser Ile His Ile
TGT ACA AGG CCC AAC AAC AAT ATA AAA ATA AGA AGT ATA CAT ATA


                20                  25                  30
Gly Pro Gly Arg Pro Phe Tyr Thr Thr Lys Ile Gly Asp Ile Arg
GGA CCA GGG AGA CCA TTT TAT ACA ACA AAA ATA GGA GAT ATA AGA


                35
Gln Ala Tyr Cys
CAA GCA TAT TGT
```

```
(2)      INFORMATION FOR SEQ ID NO: EE244-2
         (i)      SEQUENCE CHARACTERISTICS:
                  (A)     LENGTH:  102
                  (B)     TYPE:  Nucleic Acid
                  (C)     STRANDEDNESS:  Single
                  (D)     TOPOLOGY:  Linear
         (ii)     KIND: cDNA to genomic RNA
         (ii)     KIND (if peptide or protein):
                  (A)     SEQUENCE ASSEMBLY METHOD:  Overlap
                  (B)     FRAGMENT TYPE:  Internal Fragment
                  (C)     HYPOTHETICAL:  _____
         (iii)    ORIGINAL SOURCE: HIV
                  (E)     INDIVIDUAL ISOLATE:  _____
         (iv)     IMMEDIATE SOURCE:
                  (C)     CLONE:  _____
         (v)      POSITION IN GENOME: Within Env Gene
         (vi)     PROPERTIES OF SEQUENCE:  Expresses conserved antigenic
                  determinant
         (viii)   SEQUENCE DESCRIPTION:
```

SEQ ID NO:  EE244-2

```
          1                 5                      10                          15
Cys Thr Arg Pro Asn Asn Asn Ile Lys Ile Arg Ser Ile His Ile
TGT ACA AGG CCC AAC AAC AAT ATA AAA ATA AGA AGT ATA CAT ATA


                            20                  25                      30
Gly Pro Gly Arg Pro Phe Tyr Thr Thr Lys Ile Gly Asp Ile Arg
GGA CCA GGG AGA CCA TTT TAT ACA ACA AAA ATA GGA GAT ATA AGA


                            35
Gln Ala Tyr Cys
CAA GCA TAT TGT
```

(2)        INFORMATION FOR SEQ ID NO: EE244-3
           (i)       SEQUENCE CHARACTERISTICS:
                     (A)       LENGTH:  102
                     (B)       TYPE:  Nucleic Acid
                     (C)       STRANDEDNESS:  Single
                     (D)       TOPOLOGY:  Linear
           (ii)      KIND: cDNA to genomic RNA
           (ii)      KIND (if peptide or protein):
                     (A)       SEQUENCE ASSEMBLY METHOD:  Overlap
                     (B)       FRAGMENT TYPE:  Internal Fragment
                     (C)       HYPOTHETICAL:  _____
           (iii)     ORIGINAL SOURCE: HIV
                     (E)       INDIVIDUAL ISOLATE:  _____
           (iv)      IMMEDIATE SOURCE:
                     (C)       CLONE:  _____
           (v)       POSITION IN GENOME: Within Env Gene
           (vi)      PROPERTIES OF SEQUENCE:  Expresses conserved antigenic
                     determinant
           (viii)    SEQUENCE DESCRIPTION:


SEQ ID NO:  EE244-3

```
          1                 5                      10                          15
Cys Thr Arg Pro Asn Asn Asn Ile Lys Ile Arg Ser Ile His Ile
TGT ACA AGG CCC AAC AAC AAT ATA AAA ATA AGA AGT ATA CAT ATA
```

70

```
                     20                    25                    30
Gly Pro Gly Arg Pro Phe Tyr Thr Thr Lys Ile Gly Asp Ile Arg
GGA CCA GGG AGA CCA TTT TAT ACA ACA AAA ATA GGA GAT ATA AGA


                     35
Gln Ala Tyr Cys
CAA GCA TAT TGT
```

(2)        INFORMATION FOR SEQ ID NO: EE289-1
           (i)      SEQUENCE CHARACTERISTICS:
                    (A)      LENGTH:  105
                    (B)      TYPE:  Nucleic Acid
                    (C)      STRANDEDNESS:  Single
                    (D)      TOPOLOGY:  Linear
           (ii)     KIND: cDNA to genomic RNA
           (ii)     KIND (if peptide or protein):
                    (A)      SEQUENCE ASSEMBLY METHOD:  Overlap
                    (B)      FRAGMENT TYPE:  Internal Fragment
                    (C)      HYPOTHETICAL:  _____
           (iii)    ORIGINAL SOURCE: HIV
                    (E)      INDIVIDUAL ISOLATE:  _____
           (iv)     IMMEDIATE SOURCE:
                    (C)      CLONE:  _____
           (v)      POSITION IN GENOME: Within Env Gene
           (vi)     PROPERTIES OF SEQUENCE:  Expresses conserved antigenic
                    determinant
           (viii)   SEQUENCE DESCRIPTION:


SEQ ID NO:  EE289-1


```
 1                   5                    10                   15
Cys Thr Arg Pro Asn Asn Asn Thr Arg Lys Gly Ile His Ile Gly
TGT ACA AGA CCC AAC AAC AAT ACA AGA AAA GGT ATA CAT ATA GGA


                     20                    25                    30
Pro Gly Arg Ala Phe Tyr Thr Thr Gly Glu Ile Ile Gly Asp Ile
CCA GGG AGA GCA TTT TAT ACT ACA GGA GAA ATA ATA GGA GAT ATA


                     35
Arg Gln Ala His Cys
AGA CAA GCA CAT TGT
```

(2)     INFORMATION FOR SEQ ID NO: EE289-2
        (i)     SEQUENCE CHARACTERISTICS:
                (A)     LENGTH:  105
                (B)     TYPE:  Nucleic Acid
                (C)     STRANDEDNESS:  Single
                (D)     TOPOLOGY:  Linear
        (ii)    KIND: cDNA to genomic RNA
        (ii)    KIND (if peptide or protein):
                (A)     SEQUENCE ASSEMBLY METHOD:  Overlap
                (B)     FRAGMENT TYPE:  Internal Fragment
                (C)     HYPOTHETICAL:  _____
        (iii)   ORIGINAL SOURCE: HIV
                (E)     INDIVIDUAL ISOLATE:  _____
        (iv)    IMMEDIATE SOURCE:
                (C)     CLONE:  _____
        (v)     POSITION IN GENOME: Within Env Gene
        (vi)    PROPERTIES OF SEQUENCE:  Expresses conserved antigenic
                determinant
        (viii)  SEQUENCE DESCRIPTION:


SEQ ID NO:  EE289-2


```
1                   5                       10                      15
Cys Thr Arg Pro Asn Asn Asn Thr Arg Lys Gly Ile His Ile Gly
TGT ACA AGA CCC AAC AAC AAT ACA AGA AAA GGT ATA CAT ATA GGA


                    20                      25                      30
Pro Gly Arg Ala Phe Tyr Thr Thr Gly Glu Ile Ile Gly Asp Ile
CCA GGG AGA GCA TTT TAT ACT ACA GGA GAA ATA ATA GGA GAT ATA


                    35
Arg Gln Ala His Cys
AGA CAA GCA CAT TGT
```


(2)     INFORMATION FOR SEQ ID NO: EE290-1
        (i)     SEQUENCE CHARACTERISTICS:
                (A)     LENGTH:  105
                (B)     TYPE:  Nucleic Acid
                (C)     STRANDEDNESS:  Single
                (D)     TOPOLOGY:  Linear
        (ii)    KIND: cDNA to genomic RNA

```
(ii)     KIND (if peptide or protein):
         (A)     SEQUENCE ASSEMBLY METHOD:  Overlap
         (B)     FRAGMENT TYPE:  Internal Fragment
         (C)     HYPOTHETICAL:  _____
(iii)    ORIGINAL SOURCE: HIV
         (E)     INDIVIDUAL ISOLATE:  _____
(iv)     IMMEDIATE SOURCE:
         (C)     CLONE:  _____
(v)      POSITION IN GENOME: Within Env Gene
(vi)     PROPERTIES OF SEQUENCE:  Expresses conserved antigenic
         determinant
(viii)   SEQUENCE DESCRIPTION:
```

SEQ ID NO:  EE290-1

```
1               5                    10                 15
Cys Thr Arg Pro Asn Asn Asn Thr Arg Lys Ser Ile His Leu Gly
TGT ACA AGA CCC AAC AAC AAT ACA AGA AAA AGT ATA CAT CTA GGG


               20                   25                 30
Pro Gly Arg Ala Phe Tyr Thr Thr Gly Asp Ile Ile Gly Asp Ile
CCA GGG AGA GCA TTT TAT ACA ACA GGA GAC ATA ATA GGA GAT ATA


               35
Arg Gln Ala His Cys
AGA CAA GCA CAT TGT
```

```
(2)      INFORMATION FOR SEQ ID NO: EE293-1
         (i)      SEQUENCE CHARACTERISTICS:
                  (A)     LENGTH:  105
                  (B)     TYPE:  Nucleic Acid
                  (C)     STRANDEDNESS:  Single
                  (D)     TOPOLOGY:  Linear
         (ii)     KIND: cDNA to genomic RNA
         (ii)     KIND (if peptide or protein):
                  (A)     SEQUENCE ASSEMBLY METHOD:  Overlap
                  (B)     FRAGMENT TYPE:  Internal Fragment
                  (C)     HYPOTHETICAL:  _____
         (iii)    ORIGINAL SOURCE: HIV
                  (E)     INDIVIDUAL ISOLATE:  _____
         (iv)     IMMEDIATE SOURCE:
                  (C)     CLONE:  _____
         (v)      POSITION IN GENOME: Within Env Gene
         (vi)     PROPERTIES OF SEQUENCE:  Expresses conserved antigenic
                  determinant
         (viii)   SEQUENCE DESCRIPTION:
```

SEQ ID NO:   EE293-1

```
         1                 5                      10                    15
Cys Thr Arg Pro Asn Asn Asn Thr Arg Lys Ser Ile His Ile Gly
TGT ACA AGA CCC AAC AAC AAT ACA AGA AAA AGT ATA CAT ATA GGA


                          20                     25                    30
Pro Gly Arg Ala Phe Tyr Thr Thr Gly Glu Ile Ile Gly Asn Ile
CCA GGG AGA GCA TTT TAT ACA ACA GGA GAA ATA ATA GGA AAT ATA


                          35
Arg Gln Ala His Cys
AGA CAA GCA CAT TGT
```

```
(2)        INFORMATION FOR SEQ ID NO: EE293-2
           (i)      SEQUENCE CHARACTERISTICS:
                    (A)      LENGTH:  105
                    (B)      TYPE:  Nucleic Acid
                    (C)      STRANDEDNESS:  Single
                    (D)      TOPOLOGY:  Linear
           (ii)     KIND: cDNA to genomic RNA
           (ii)     KIND (if peptide or protein):
                    (A)      SEQUENCE ASSEMBLY METHOD:  Overlap
                    (B)      FRAGMENT TYPE:  Internal Fragment
                    (C)      HYPOTHETICAL:  _____
           (iii)    ORIGINAL SOURCE: HIV
                    (E)      INDIVIDUAL ISOLATE:  _____
           (iv)     IMMEDIATE SOURCE:
                    (C)      CLONE:  _____
           (v)      POSITION IN GENOME: Within Env Gene
           (vi)     PROPERTIES OF SEQUENCE:  Expresses conserved antigenic
                    determinant
           (viii)   SEQUENCE DESCRIPTION:
```

SEQ ID NO:   EE293-2

```
         1                 5                      10                    15
Cys Thr Arg Pro Asn Asn Asn Thr Arg Lys Ser Ile His Ile Gly
TGT ACA AGA CCC AAC AAC AAT ACA AGA AAA AGT ATA CAT ATA GGA
```

74

```
                    20                  25                  30
Pro Gly Arg Ala Phe Tyr Thr Thr Gly Glu Ile Ile Gly Asn Ile
CCA GGG AGA GCA TTT TAT ACA ACA GGA GAA ATA ATA GGA AAT ATA


                    35
Arg Gln Ala His Cys
AGA CAA GCA CAT TGT
```

(2)      INFORMATION FOR SEQ ID NO: EE293-3
         (i)      SEQUENCE CHARACTERISTICS:
                  (A)      LENGTH:  105
                  (B)      TYPE:  Nucleic Acid
                  (C)      STRANDEDNESS:  Single
                  (D)      TOPOLOGY:  Linear
         (ii)     KIND: cDNA to genomic RNA
         (ii)     KIND (if peptide or protein):
                  (A)      SEQUENCE ASSEMBLY METHOD:  Overlap
                  (B)      FRAGMENT TYPE:  Internal Fragment
                  (C)      HYPOTHETICAL:  _____
         (iii)    ORIGINAL SOURCE: HIV
                  (E)      INDIVIDUAL ISOLATE:  _____
         (iv)     IMMEDIATE SOURCE:
                  (C)      CLONE:  _____
         (v)      POSITION IN GENOME: Within Env Gene
         (vi)     PROPERTIES OF SEQUENCE:  Expresses conserved antigenic
                  determinant
         (viii)   SEQUENCE DESCRIPTION:


SEQ ID NO:  EE293-3


```
1                   5                   10                  15
Cys Thr Arg Pro Asn Asn Asn Thr Arg Lys Ser Ile His Ile Gly
TGT ACA AGA CCC AAC AAC AAT ACA AGA AAA AGT ATA CAT ATA GGA


                    20                  25                  30
Pro Gly Arg Ala Phe Tyr Thr Thr Gly Glu Ile Ile Gly Asn Ile
CCA GGG AGA GCA TTT TAT ACA ACA GGA GAA ATA ATA GGA AAT ATA


                    35
Arg Gln Ala His Cys
AGA CAA GCA CAT TGT
```

(2)      INFORMATION FOR SEQ ID NO: EE295-1
      (i)      SEQUENCE CHARACTERISTICS:
            (A)      LENGTH:  105
            (B)      TYPE:  Nucleic Acid
            (C)      STRANDEDNESS:  Single
            (D)      TOPOLOGY:  Linear
      (ii)   KIND: cDNA to genomic RNA
      (ii)   KIND (if peptide or protein):
            (A)      SEQUENCE ASSEMBLY METHOD:  Overlap
            (B)      FRAGMENT TYPE:  Internal Fragment
            (C)      HYPOTHETICAL:  _____
      (iii)  ORIGINAL SOURCE: HIV
            (E)      INDIVIDUAL ISOLATE:  _____
      (iv)   IMMEDIATE SOURCE:
            (C)      CLONE:  _____
      (v)    POSITION IN GENOME: Within Env Gene
      (vi)   PROPERTIES OF SEQUENCE:  Expresses conserved antigenic
            determinant
      (viii) SEQUENCE DESCRIPTION:


SEQ ID NO:  EE295-1


```
1                5                10                15
Cys Thr Arg Pro Asn Asn Asn Thr Arg Lys Gly Ile His Ile Gly
TGT ACA AGA CCC AAC AAC AAT ACA AGA AAA GGT ATA CAT ATA GGA


                20                25                30
Pro Gly Arg Ala Phe Tyr Ala Thr Lys Asp Ile Ile Gly Asp Ile
CCA GGG AGA GCA TTT TAT GCA ACA AAA GAC ATA ATA GGA GAT ATA


                35
Arg Gln Ala His Cys
AGA CAA GCA CAT TGT
```


(2)      INFORMATION FOR SEQ ID NO: EE295-2
      (i)      SEQUENCE CHARACTERISTICS:
            (A)      LENGTH:  105
             (B)      TYPE:  Nucleic Acid
             (C)      STRANDEDNESS:  Single
             (D)      TOPOLOGY:  Linear
      (ii)   KIND: cDNA to genomic RNA

```
(ii)    KIND (if peptide or protein):
        (A)     SEQUENCE ASSEMBLY METHOD:  Overlap
        (B)     FRAGMENT TYPE:  Internal Fragment
        (C)     HYPOTHETICAL:  _____
(iii)   ORIGINAL SOURCE: HIV
        (E)     INDIVIDUAL ISOLATE:  _____
(iv)    IMMEDIATE SOURCE:
        (C)     CLONE:  _____
(v)     POSITION IN GENOME: Within Env Gene
(vi)    PROPERTIES OF SEQUENCE:  Expresses conserved antigenic
        determinant
(viii)  SEQUENCE DESCRIPTION:
```

SEQ ID NO:  EE295-2


```
1               5                   10                  15
Cys Thr Arg Pro Asn Asn Asn Thr Arg Lys Gly Ile His Ile Gly
TGT ACA AGA CCC AAC AAC AAT ACA AGA AAA GGT ATA CAT ATA GGA


                20                  25                  30
Pro Gly Arg Ala Phe Tyr Ala Thr Lys Asp Ile Ile Gly Asp Ile
CCA GGG AGA GCA TTT TAT GCA ACA AAA GAC ATA ATA GGA GAT ATA


                35
Arg Gln Ala His Cys
AGA CAA GCA CAT TGT
```


```
(2)     INFORMATION FOR SEQ ID NO: EE297-1
        (i)     SEQUENCE CHARACTERISTICS:
                (A)     LENGTH:  105
                (B)     TYPE:  Nucleic Acid
                (C)     STRANDEDNESS:  Single
                (D)     TOPOLOGY:  Linear
        (ii)    KIND: cDNA to genomic RNA
        (ii)    KIND (if peptide or protein):
                (A)     SEQUENCE ASSEMBLY METHOD:  Overlap
                (B)     FRAGMENT TYPE:  Internal Fragment
                (C)     HYPOTHETICAL:  _____
        (iii)   ORIGINAL SOURCE: HIV
                (E)     INDIVIDUAL ISOLATE:  _____
        (iv)    IMMEDIATE SOURCE:
                (C)     CLONE:  _____
        (v)     POSITION IN GENOME: Within Env Gene
        (vi)    PROPERTIES OF SEQUENCE:  Expresses conserved antigenic
                determinant
        (viii)  SEQUENCE DESCRIPTION:
```

SEQ ID NO:  EE297-1

|  | 1 | | | 5 | | | | | 10 | | | | | 15 |
|---|---|---|---|---|---|---|---|---|---|---|---|---|---|---|
| | Cys | Ile | Arg | Pro | Asn | Asn | Asn | Thr | Arg | Lys | Ser | Ile | Asn | Ile | Gly |
| | TGT | ATA | AGA | CCC | AAC | AAC | AAT | ACA | AGA | AAA | AGT | ATA | AAT | ATA | GGA |

|  | | | | 20 | | | | | 25 | | | | | 30 |
|---|---|---|---|---|---|---|---|---|---|---|---|---|---|---|
| Pro | Gly | Arg | Ala | Phe | Tyr | Thr | Thr | Gly | Glu | Ile | Ile | Gly | Asn | Ile |
| CCA | GGG | AGA | GCA | TTT | TAT | ACA | ACA | GGA | GAA | ATA | ATA | GGA | AAT | ATA |

|  | | | | 35 |
|---|---|---|---|---|
| Arg | Gln | Ala | His | Cys |
| AGA | CAA | GCA | CAT | TGT |

(2)       INFORMATION FOR SEQ ID NO: EE297-2
          (i)      SEQUENCE CHARACTERISTICS:
                   (A)      LENGTH:  105
                   (B)      TYPE:  Nucleic Acid
                   (C)      STRANDEDNESS:  Single
                   (D)      TOPOLOGY:  Linear
          (ii)     KIND: cDNA to genomic RNA
          (ii)     KIND (if peptide or protein):
                   (A)      SEQUENCE ASSEMBLY METHOD:  Overlap
                   (B)      FRAGMENT TYPE:  Internal Fragment
                   (C)      HYPOTHETICAL:  _____
          (iii)    ORIGINAL SOURCE: HIV
                   (E)      INDIVIDUAL ISOLATE:  _____
          (iv)     1MMEDIATE SOURCE:
                   (C)      CLONE:  _____
          (v)      POSITION IN GENOME: Within Env Gene
          (vi)     PROPERTIES OF SEQUENCE:  Expresses conserved antigenic
                   determinant
          (viii)   SEQUENCE DESCRIPTION:

SEQ ID NO:  EE297-2

|  | 1 | | | 5 | | | | | 10 | | | | | 15 |
|---|---|---|---|---|---|---|---|---|---|---|---|---|---|---|
| | Cys | Ile | Arg | Pro | Asn | Asn | Asn | Thr | Arg | Lys | Ser | Ile | Asn | Ile | Gly |
| | TGT | ATA | AGA | CCC | AAC | AAC | AAT | ACA | AGA | AAA | AGT | ATA | AAT | ATA | GGA |

```
                    20                      25                      30
Pro Gly Arg Ala Phe Tyr Thr Thr Gly Glu Ile Ile Gly Asn Ile
CCA GGG AGA GCA TTT TAT ACA ACA GGA GAA ATA ATA GGA AAT ATA


                    35
Arg Gln Ala His Cys
AGA CAA GCA CAT TGT
```

(2)       INFORMATION FOR SEQ ID NO: EE297-3
          (i)     SEQUENCE CHARACTERISTICS:
                  (A)     LENGTH:  105
                  (B)     TYPE:  Nucleic Acid
                  (C)     STRANDEDNESS:  Single
                  (D)     TOPOLOGY:  Linear
          (ii)    KIND: cDNA to genomic RNA
          (ii)    KIND (if peptide or protein):
                  (A)     SEQUENCE ASSEMBLY METHOD:  Overlap
                  (B)     FRAGMENT TYPE:  Internal Fragment
                  (C)     HYPOTHETICAL:  _____
          (iii)   ORIGINAL SOURCE: HIV
                  (E)     INDIVIDUAL ISOLATE:  _____
          (iv)    IMMEDIATE SOURCE:
                  (C)     CLONE:  _____
          (v)     POSITION IN GENOME: Within Env Gene
          (vi)    PROPERTIES OF SEQUENCE:  Expresses conserved antigenic
                  determinant
          (viii)  SEQUENCE DESCRIPTION:


SEQ ID NO:   EE297-3


```
1                   5                       10                      15
Cys Ile Arg Pro Asn Asn Asn Thr Arg Lys Ser Ile Asn Ile Gly
TGT ATA AGA CCC AAC AAC AAT ACA AGG AAA AGT ATA AAT ATA GGA


                    20                      25                      30
Pro Gly Arg Ala Phe Tyr Thr Thr Gly Glu Ile Ile Gly Asn Ile
CCA GGG AGA GCA TTT TAT ACA ACA GGA GAA ATA ATA GGA AAT ATA


                    35
Arg Gln Ala His Cys
AGA CAA GCA CAT TGT
```

(2)     INFORMATION FOR SEQ ID NO: EE304-1
        (i)     SEQUENCE CHARACTERISTICS:
                (A)     LENGTH:  105
                (B)     TYPE:  Nucleic Acid
                (C)     STRANDEDNESS:  Single
                (D)     TOPOLOGY:  Linear
        (ii)    KIND: cDNA to genomic RNA
        (ii)    KIND (if peptide or protein):
                (A)     SEQUENCE ASSEMBLY METHOD:  Overlap
                (B)     FRAGMENT TYPE:  Internal Fragment
                (C)     HYPOTHETICAL:  _____
        (iii)   ORIGINAL SOURCE: HIV
                (E)     INDIVIDUAL ISOLATE:  _____
        (iv)    IMMEDIATE SOURCE:
                (C)     CLONE:  _____
        (v)     POSITION IN GENOME: Within Env Gene
        (vi)    PROPERTIES OF SEQUENCE:  Expresses conserved antigenic
                determinant
        (viii)  SEQUENCE DESCRIPTION:


SEQ ID NO:  EE304-1


```
1                   5                   10                  15
Cys Thr Arg Pro Asn Asn Asn Thr Arg Lys Ser Ile Asn Ile Gly
TGT ACA AGA CCC AAC AAC AAT ACA AGG AAA AGT ATA AAT ATA GGA


                    20                  25                  30
Pro Gly Arg Ala Phe Tyr Thr Thr Gly Glu Ile Ile Gly Asp Ile
CCA GGG AGA GCA TTT TAT ACA ACA GGA GAA ATA ATA GGA GAT ATA


                    35
Arg Gln Ala His Cys
AGA CAA GCA CAT TGT
```


(2)     INFORMATION FOR SEQ ID NO: EE304-2
        (i)     SEQUENCE CHARACTERISTICS:
                (A)     LENGTH:  105
                (B)     TYPE:  Nucleic Acid
                (C)     STRANDEDNESS:  Single
                (D)     TOPOLOGY:  Linear
        (ii)    KIND: cDNA to genomic RNA

```
(ii)     KIND (if peptide or protein):
         (A)     SEQUENCE ASSEMBLY METHOD:  Overlap
         (B)     FRAGMENT TYPE:  Internal Fragment
         (C)     HYPOTHETICAL:  _____
(iii)    ORIGINAL SOURCE: HIV
         (E)     INDIVIDUAL ISOLATE:  _____
(iv)     IMMEDIATE SOURCE:
         (C)     CLONE:  _____
(v)      POSITION IN GENOME: Within Env Gene
(vi)     PROPERTIES OF SEQUENCE:  Expresses conserved antigenic
         determinant
(viii)   SEQUENCE DESCRIPTION:
```

SEQ ID NO:  EE304-2


```
 1                 5                 10                15
Cys Thr Arg Pro Asn Asn Asn Thr Arg Arg Ser Ile Asn Ile Gly
TGT ACA AGA CCC AAC AAC AAT ACA AGG AGA AGT ATA AAT ATA GGA


                  20                25                30
Pro Gly Arg Ala Phe Tyr Thr Thr Gly Glu Ile Ile Gly Asp Ile
CCA GGG AGA GCA TTT TAT ACA ACA GGA GAA ATA ATA GGA GAT ATA


                  35
Arg Gln Ala His Cys
AGA CAA GCA CAT TGT
```

```
(2)      INFORMATION FOR SEQ ID NO: EE304-3
         (i)     SEQUENCE CHARACTERISTICS:
                 (A)     LENGTH:  105
                 (B)     TYPE:  Nucleic Acid
                 (C)     STRANDEDNESS:  Single
                 (D)     TOPOLOGY:  Linear
         (ii)    KIND: cDNA to genomic RNA
         (ii)    KIND (if peptide or protein):
                 (A)     SEQUENCE ASSEMBLY METHOD:  Overlap
                 (B)     FRAGMENT TYPE:  Internal Fragment
                 (C)     HYPOTHETICAL:  _____
         (iii)   ORIGINAL SOURCE: HIV
                 (E)     INDIVIDUAL ISOLATE:  _____
         (iv)    IMMEDIATE SOURCE:
                 (C)     CLONE:  _____
         (v)     POSITION IN GENOME: Within Env Gene
         (vi)    PROPERTIES OF SEQUENCE:  Expresses conserved antigenic
                 determinant
         (viii)  SEQUENCE DESCRIPTION:
```

SEQ ID NO:  EE304-3

```
       1                   5                       10                      15
      Cys Thr Arg Pro Asn Asn Asn Thr Arg Arg Ser Ile Asn Ile Gly
      TGT ACA AGA CCC AAC AAC AAT ACA AGG AGA AGT ATA AAT ATA GGA


                          20                      25                      30
      Pro Gly Arg Ala Phe Tyr Thr Thr Gly Glu Ile Ile Gly Asp Ile
      CCA GGG AGA GCA TTT TAT ACA ACA GGA GAA ATA ATA GGA GAT ATA


                          35
      Arg Gln Ala His Cys
      AGA CAA GCA CAT TGT
```

(2) INFORMATION FOR SEQ ID NO: EE308-1
   (i)   SEQUENCE CHARACTERISTICS:
      (A)   LENGTH:  105
      (B)   TYPE:  Nucleic Acid
      (C)   STRANDEDNESS:  Single
      (D)   TOPOLOGY:  Linear
  (ii) KIND: cDNA to genomic RNA
  (ii) KIND (if peptide or protein):
      (A)   SEQUENCE ASSEMBLY METHOD:  Overlap
      (B)   FRAGMENT TYPE:  Internal Fragment
      (C)   HYPOTHETICAL:  _____
  (iii)    ORIGINAL SOURCE: HIV
      (E)   INDIVIDUAL
          ISOLATE:  _____
  (iv) IMMEDIATE SOURCE:
      (C)   CLONE:  _____
  (v)  POSITION IN GENOME: Within Env Gene
  (vi) PROPERTIES OF SEQUENCE:  Expresses conserved
      antigenic determinant
  (viii)    SEQUENCE DESCRIPTION:


SEQ ID NO:  EE308-1


```
 1                 5                      10                      15
Cys Thr Arg Pro Asn Asn Asn Thr Arg Lys Ser Ile His Ile Gly
TGT ACA AGA CCC AAC AAC AAT ACA AGA AAA AGT ATA CAT ATA GGA


                   20                      25                      30
Pro Gly Arg Ala Phe Tyr Thr Thr Gly Glu Ile Ile Gly Asp Ile
CCA GGC AGA GCA TTT TAT ACA ACA GGA GAA ATA ATA GGA GAT ATA


                   35
Arg Gln Ala His Cys
AGA CAA GCA CAT TGT
```


(2) INFORMATION FOR SEQ ID NO: EE308-2
   (i)   SEQUENCE CHARACTERISTICS:
      (A)   LENGTH:  105
      (B)   TYPE:  Nucleic Acid
      (C)   STRANDEDNESS:  Single
      (D)   TOPOLOGY:  Linear
  (ii) KIND: cDNA to genomic RNA

```
(ii) KIND (if peptide or protein):
     (A)   SEQUENCE ASSEMBLY METHOD:  Overlap
     (B)   FRAGMENT TYPE:  Internal Fragment
     (C)   HYPOTHETICAL:  _____
(iii)      ORIGINAL SOURCE: HIV
     (E)   INDIVIDUAL ISOLATE:  _____
(iv) IMMEDIATE SOURCE:
     (C)   CLONE:  _____
(v)  POSITION IN GENOME: Within Env Gene
(vi) PROPERTIES OF SEQUENCE:  Expresses conserved antigenic
     determinant
(viii)     SEQUENCE DESCRIPTION:
```

SEQ ID NO:   EE308-2

```
   1             5                  10                 15
Cys Thr Arg Pro Asn Asn Asn Thr Arg Lys Ser Ile His Ile Gly
TGT ACA AGA CCC AAC AAC AAT ACA AGA AAA AGT ATA CAT ATA GGA


                 20                 25                 30
Pro Gly Arg Pro Phe Tyr Thr Thr Gly Glu Ile Ile Gly Asp Ile
CCA GGC AGA CCA TTT TAT ACA ACA GGA GAA ATA ATA GGA GAT ATA


                 35
Arg Gln Ala His Cys
AGA CAA GCA CAT TGT
```

```
(2) INFORMATION FOR SEQ ID NO: EE310-1
    (i)   SEQUENCE CHARACTERISTICS:
          (A)   LENGTH:  105
          (B)   TYPE:  Nucleic Acid
          (C)   STRANDEDNESS:  Single
          (D)   TOPOLOGY:  Linear
    (ii) KIND: cDNA to genomic RNA
    (ii) KIND (if peptide or protein):
          (A)   SEQUENCE ASSEMBLY METHOD:  Overlap
          (B)   FRAGMENT TYPE:  Internal Fragment
          (C)   HYPOTHETICAL:  _____
    (iii)      ORIGINAL SOURCE: HIV
          (E)   INDIVIDUAL ISOLATE:  _____
    (iv) IMMEDIATE SOURCE:
          (C)   CLONE:  _____
    (v)  POSITION IN GENOME: Within Env Gene
    (vi) PROPERTIES OF SEQUENCE:  Expresses conserved antigenic
         determinant
    (viii)     SEQUENCE DESCRIPTION:
```

SEQ ID NO: EE310-1

```
     1                    5                      10                       15
    Cys Thr Arg Pro Ser Asn Asn Thr Arg Arg Gly Ile His Ile Gly
    TGT ACA AGA CCC AGC AAC AAT ACC AGA AGA GGT ATA CAT ATA GGA


                          20                     25                       30
    Pro Gly Arg Ala Phe Tyr Thr Thr Gly Glu Ile Thr Gly Asp Ile
    CCA GGG AGA GCA TTT TAT ACA ACA GGA GAA ATA ACA GGA GAT ATA


                          35
    Arg Gln Ala His Cys
    AGA CAA GCA CAT TGT
```

```
(2)        INFORMATION FOR SEQ ID NO: EE310-2
           (i)       SEQUENCE CHARACTERISTICS:
                     (A)      LENGTH:  105
                     (B)      TYPE:  Nucleic Acid
                     (C)      STRANDEDNESS:  Single
                     (D)      TOPOLOGY:  Linear
           (ii)      KIND: cDNA to genomic RNA
           (ii)      KIND (if peptide or protein):
                     (A)      SEQUENCE ASSEMBLY METHOD:  Overlap
                     (B)      FRAGMENT TYPE:  Internal Fragment
                     (C)      HYPOTHETICAL:  _____
           (iii)     ORIGINAL SOURCE: HIV
                     (E)      INDIVIDUAL ISOLATE:  _____
           (iv)      IMMEDIATE SOURCE:
                     (C)      CLONE:  _____
           (v)       POSITION IN GENOME: Within Env Gene
           (vi)      PROPERTIES OF SEQUENCE:  Expresses conserved antigenic
                     determinant
           (viii)    SEQUENCE DESCRIPTION:
```

SEQ ID NO: EE310-2

```
     1                    5                      10                       15
    Cys Thr Arg Pro Ser Asn Asn Thr Arg Arg Gly Ile His Ile Gly
    TGT ACA AGA CCC AGC AAC AAT ACA AGA AGA GGT ATA CAT ATA GGA
```

```
                    20                    25                    30
Pro Gly Arg Ala Phe Tyr Thr Thr Gly Glu Ile Thr Gly Asp Ile
CCA GGG AGA GCA TTT TAT ACA ACA GGA GAA ATA ACA GGA GAT ATA


                    35
Arg Gln Ala His Cys
AGA CAA GCA CAT TGT
```

(2)     INFORMATION FOR SEQ ID NO: EE310-3
    (i)     SEQUENCE CHARACTERISTICS:
        (A)     LENGTH:  105
        (B)     TYPE:  Nucleic Acid
        (C)     STRANDEDNESS:  Single
        (D)     TOPOLOGY:  Linear
    (ii)    KIND: cDNA to genomic RNA
    (ii)    KIND (if peptide or protein):
        (A)     SEQUENCE ASSEMBLY METHOD:  Overlap
        (B)     FRAGMENT TYPE:  Internal Fragment
        (C)     HYPOTHETICAL:  _____
    (iii)   ORIGINAL SOURCE: HIV
        (E)     INDIVIDUAL ISOLATE:  _____
    (iv)    IMMEDIATE SOURCE:
        (C)     CLONE:  _____
    (v)     POSITION IN GENOME: Within Env Gene
    (vi)    PROPERTIES OF SEQUENCE:  Expresses conserved antigenic
        determinant
    (viii)  SEQUENCE DESCRIPTION:


SEQ ID NO:  EE310-3


```
1                   5                    10                    15
Cys Thr Arg Pro Ser Asn Asn Thr Arg Lys Gly Ile His Ile Gly
TGT ACA AGA CCC AGC AAC AAT ACA AGA AAA GGT ATA CAT ATA GGA


                    20                    25                    30
Pro Gly Arg Ala Phe Tyr Thr Thr Gly Glu Ile Thr Gly Asp Ile
CCA GGG AGA GCA TTT TAT ACA ACA GGA GAA ATA ACA GGA GAT ATA


                    35
Arg Gln Ala His Cys
AGA CAA GCA CAT TGT
```

(2)      INFORMATION FOR SEQ ID NO: EE311-1
     (i)      SEQUENCE CHARACTERISTICS:
          (A)      LENGTH:  105
          (B)      TYPE:  Nucleic Acid
          (C)      STRANDEDNESS:  Single
          (D)      TOPOLOGY:  Linear
    (ii)   KIND: cDNA to genomic RNA
    (ii)   KIND (if peptide or protein):
          (A)      SEQUENCE ASSEMBLY METHOD:  Overlap
          (B)      FRAGMENT TYPE:  Internal Fragment
          (C)      HYPOTHETICAL:  _____
    (iii)  ORIGINAL SOURCE: HIV
          (E)      INDIVIDUAL ISOLATE:  _____
    (iv)   IMMEDIATE SOURCE:
          (C)      CLONE:  _____
    (v)    POSITION IN GENOME: Within Env Gene
    (vi)   PROPERTIES OF SEQUENCE:  Expresses conserved antigenic
          determinant
    (viii) SEQUENCE DESCRIPTION:


SEQ ID NO:  EE311-1


```
1                   5                   10                  15
Cys Thr Arg Pro Asn Asn Asn Thr Arg Arg Ser Ile His Ile Gly
TGT ACA AGA CCC AAC AAC AAT ACC AGA AGA AGT ATA CAT ATA GGA


                    20                  25                  30
Pro Gly Arg Ala Phe Tyr Ala Thr Gly Ala Ile Ile Gly Asp Ile
CCA GGG AGA GCA TTT TAT GCA ACA GGA GCT ATA ATA GGA GAT ATA


                    35
Arg Arg Ala Tyr Cys
AGA CGA GCA TAT TGT
```


(2)      INFORMATION FOR SEQ ID NO: EE312-1
     (i)      SEQUENCE CHARACTERISTICS:
          (A)      LENGTH:  102
          (B)      TYPE:  Nucleic Acid
          (C)      STRANDEDNESS:  Single
          (D)      TOPOLOGY:  Linear
    (ii)   KIND: cDNA to genomic RNA

87

```
(ii)    KIND (if peptide or protein):
        (A)    SEQUENCE ASSEMBLY METHOD:  Overlap
        (B)    FRAGMENT TYPE:  Internal Fragment
        (C)    HYPOTHETICAL:  _____
(iii)   ORIGINAL SOURCE: HIV
        (E)    INDIVIDUAL ISOLATE:  _____
(iv)    IMMEDIATE SOURCE:
        (C)    CLONE:  _____
(v)     POSITION IN GENOME: Within Env Gene
(vi)    PROPERTIES OF SEQUENCE:  Expresses conserved antigenic
        determinant
(viii)  SEQUENCE DESCRIPTION:
```

SEQ ID NO:  EE312-1

```
1               5                       10                      15
Cys Thr Arg Pro Asn Asn Asn Thr Arg Lys Ser Ile Thr Ile Gly
TGT ACA AGG CCC AAC AAC AAT ACA AGA AAA AGT ATA ACT ATA GGA


                20                      25                      30
Pro Glu Arg Ala Phe Tyr Ala Thr Asp Ile Ile Gly Asn Ile Arg
CCA GAG AGA GCA TTT TAT GCA ACA GAT ATA ATA GGA AAT ATA AGA


                35
Gln Ala His Cys
CAA GCA CAT TGT
```

```
(2)     INFORMATION FOR SEQ ID NO: EE312-2
        (i)     SEQUENCE CHARACTERISTICS:
                (A)    LENGTH:    99
                (B)    TYPE:  Nucleic Acid
                (C)    STRANDEDNESS:  Single
                (D)    TOPOLOGY:  Linear
        (ii)    KIND: cDNA to genomic RNA
        (ii)    KIND (if peptide or protein):
                (A)    SEQUENCE ASSEMBLY METHOD:  Overlap
                (B)    FRAGMENT TYPE:  Internal Fragment
                (C)    HYPOTHETICAL:  _____
        (iii)   ORIGINAL SOURCE: HIV
                (E)    INDIVIDUAL ISOLATE:  _____
        (iv)    IMMEDIATE SOURCE:
                (C)    CLONE:  _____
        (v)     POSITION IN GENOME: Within Env Gene
        (vi)    PROPERTIES OF SEQUENCE:  Expresses conserved antigenic
                determinant
        (viii)  SEQUENCE DESCRIPTION:
```

SEQ ID NO:  EEE312-2

| 1 | | | | 5 | | | | | 10 | | | | | 15 |
|---|---|---|---|---|---|---|---|---|---|---|---|---|---|---|
Cys Thr Arg Pro Asn Asn Asn Thr Arg Lys Ser Ile Thr Ile Gly
TGT ACA AGG CCC AAC AAC AAT ACA AGA AAA AGT ATA ACT ATA GGA

| | | | | 20 | | | | | 25 | | | | | 30 |
Pro Gly Arg Ala Phe Tyr Ala Thr Asp Ile Ile Gly Asn Ile Arg
CCA GGG AGA GCA TTT TAT GCA ACA GAT ATA ATA GGA AAT ATA AGA

| | | | | 35 |
Gln Ala His
CAA GCA CAT

```
(2)      INFORMATION FOR SEQ ID NO: EE313-1
         (i)      SEQUENCE CHARACTERISTICS:
                  (A)      LENGTH:  105
                  (B)      TYPE:  Nucleic Acid
                  (C)      STRANDEDNESS:  Single
                  (D)      TOPOLOGY:  Linear
         (ii)     KIND: cDNA to genomic RNA
         (ii)     KIND (if peptide or protein):
                  (A)      SEQUENCE ASSEMBLY METHOD:  Overlap
                  (B)      FRAGMENT TYPE:  Internal Fragment
                  (C)      HYPOTHETICAL:  _____
         (iii)    ORIGINAL SOURCE: HIV
                  (E)      INDIVIDUAL ISOLATE:  _____
         (iv)     IMMEDIATE SOURCE:
                  (C)      CLONE:  _____
         (v)      POSITION IN GENOME: Within Env Gene
         (vi)     PROPERTIES OF SEQUENCE:  Expresses conserved antigenic
                  determinant
         (viii)   SEQUENCE DESCRIPTION:
```

SEQ ID NO:  EE313-1

| 1 | | | | 5 | | | | | 10 | | | | | 15 |
|---|---|---|---|---|---|---|---|---|---|---|---|---|---|---|
Cys Thr Arg Pro Asn Asn His Thr Glu Lys Arg Ile Thr Leu Gly
TGT ACA AGA CCC AAC AAC CAT ACA GAA AAA CGT ATA ACT CTA GGA

```
                     20                      25                      30
Pro Gly Arg Val Leu Tyr Thr Thr Gly Arg Ile Ile Gly Asp Ile
CCG GGG AGA GTA CTT TAT ACA ACA GGA AGA ATA ATA GGA GAT ATA


                     35
Arg Arg Ala His Cys
AGA CGA GCA CAT TGT
```

(2)      INFORMATION FOR SEQ ID NO: EE317-1
         (i)      SEQUENCE CHARACTERISTICS:
                  (A)      LENGTH:  105
                  (B)      TYPE:  Nucleic Acid
                  (C)      STRANDEDNESS:  Single
                  (D)      TOPOLOGY:  Linear
         (ii)     KIND: cDNA to genomic RNA
         (ii)     KIND (if peptide or protein):
                  (A)      SEQUENCE ASSEMBLY METHOD:  Overlap
                  (B)      FRAGMENT TYPE:  Internal Fragment
                  (C)      HYPOTHETICAL:    _____
         (iii)    ORIGINAL SOURCE: HIV
                  (E)      INDIVIDUAL ISOLATE:    _____
         (iv)     IMMEDIATE SOURCE:
                  (C)      CLONE:    _____
         (v)      POSITION IN GENOME: Within Env Gene
         (vi)     PROPERTIES OF SEQUENCE:  Expresses conserved antigenic
                  determinant
         (viii)   SEQUENCE DESCRIPTION:


SEQ ID NO:  EE317-1


```
 1                   5                      10                      15
Cys Thr Arg Pro Asn Asn Asn Thr Arg Lys Ser Ile Thr Ile Gly
TGT ACA AGA CCC AAT AAC AAT ACA AGA AAA AGT ATA ACT ATA GGA


                     20                      25                      30
Pro Gly Arg Ala Phe Tyr Ala Thr Gly Glu Ile Ile Gly Asp Ile
CCA GGG AGA GCA TTT TAT GCA ACA GGA GAA ATA ATA GGA GAT ATA


                     35
Arg Gln Ala His Cys
AGA CAA GCA CAT TGT
```

```
(2)       INFORMATION FOR SEQ ID NO: EE320-1
          (i)    SEQUENCE CHARACTERISTICS:
                 (A)    LENGTH:  105
                 (B)    TYPE:  Nucleic Acid
                 (C)    STRANDEDNESS:  Single
                 (D)    TOPOLOGY:  Linear
          (ii)   KIND: cDNA to genomic RNA
          (ii)   KIND (if peptide or protein):
                 (A)    SEQUENCE ASSEMBLY METHOD:  Overlap
                 (B)    FRAGMENT TYPE:  Internal Fragment
                 (C)    HYPOTHETICAL:  _____
          (iii)  ORIGINAL SOURCE: HIV
                 (E)    INDIVIDUAL ISOLATE:  _____
          (iv)   IMMEDIATE SOURCE:
                 (C)    CLONE:  _____
          (v)    POSITION IN GENOME: Within Env Gene
          (vi)   PROPERTIES OF SEQUENCE:  Expresses conserved antigenic
                 determinant
          (viii) SEQUENCE DESCRIPTION:


SEQ ID NO:  EE320-1


 1             5                  10                  15
Cys Thr Arg Pro Asn Asn Asn Thr Arg Lys Ser Ile His Ile Gly
TGT ACA AGA CCC AAC AAC AAT ACA AGA AAA AGT ATA CAT ATA GGA


              20                 25                  30
Pro Gly Arg Ala Phe Tyr Ala Thr Gly Glu Ile Ile Gly Asp Ile
CCA GGG AGA GCA TTT TAT GCA ACA GGA GAA ATA ATA GGA GAT ATA


              35
Arg Gln Ala His Cys
AGA CAA GCA CAT TGC


2)        INFORMATION FOR SEQ ID NO: EE320-2
          (i)    SEQUENCE CHARACTERISTICS:
                 (A)    LENGTH:  105
                 (B)    TYPE:  Nucleic Acid
                 (C)    STRANDEDNESS:  Single
                 (D)    TOPOLOGY:  Linear
          (ii)   KIND: cDNA to genomic RNA
```

```
(ii)    KIND (if peptide or protein):
        (A)     SEQUENCE ASSEMBLY METHOD:  Overlap
        (B)     FRAGMENT TYPE:  Internal Fragment
        (C)     HYPOTHETICAL:  _____
(iii)   ORIGINAL SOURCE: HIV
        (E)     INDIVIDUAL ISOLATE:  _____
(iv)    IMMEDIATE SOURCE:
        (C)     CLONE:  _____
(v)     POSITION IN GENOME: Within Env Gene
(vi)    PROPERTIES OF SEQUENCE:  Expresses conserved antigenic
        determinant
(viii)  SEQUENCE DESCRIPTION:
```

SEQ ID NO:  EE320-2

```
1                5                    10                   15
Cys Thr Arg Pro Asn Asn Asn Thr Arg Lys Ser Ile His Ile Gly
TGT ACA AGA CCC AAC AAC AAT ACA AGA AAA AGT ATA CAT ATA GGA


                 20                   25                   30
Pro Gly Arg Ala Phe Tyr Thr Thr Gly Glu Ile Ile Gly Asp Ile
CCA GGC AGA GCA TTT TAT ACA ACA GGA GAA ATA ATA GGA GAT ATA


                 35
Arg Gln Ala His Cys
AGA CAA GCA CAT TGT
```

```
(2)     INFORMATION FOR SEQ ID NO: EE322-1
        (i)     SEQUENCE CHARACTERISTICS:
                (A)     LENGTH:    99
                (B)     TYPE:  Nucleic Acid
                (C)     STRANDEDNESS:  Single
                (D)     TOPOLOGY:  Linear
        (ii)    KIND: cDNA to genomic RNA
        (ii)    KIND (if peptide or protein):
                (A)     SEQUENCE ASSEMBLY METHOD:  Overlap
                (B)     FRAGMENT TYPE:  Internal Fragment
                (C)     HYPOTHETICAL:  _____
        (iii)   ORIGINAL SOURCE: HIV
                (E)     INDIVIDUAL ISOLATE:  _____
        (iv)    IMMEDIATE SOURCE:
                (C)     CLONE:  _____
        (v)     POSITION IN GENOME: Within Env Gene
        (vi)    PROPERTIES OF SEQUENCE:  Expresses conserved antigenic
                determinant
        (viii)  SEQUENCE DESCRIPTION:
```

SEQ ID NO:  EE322-1

```
    1                 5                    10                   15
Thr Arg Pro Gly Asn Asn Thr Arg Lys Gly Ile His Ile Gly Pro
ACA AGA CCC GGC AAC AAT ACA AGA AAA GGT ATA CAT ATA GGA CCA
```
.

```
                  20                   25                   30
Gly Arg Ala Ile Tyr Ala Thr Asp Ile Ile Gly Asp Ile Arg Gln
GGG AGA GCA ATT TAT GCA ACA GAT ATA ATA GGA GAT ATA AGA CAA
```

```
                  35
Ala His Cys
GCA CAT TGT
```

(2)     INFORMATION FOR SEQ ID NO: EE322-2
        (i)      SEQUENCE CHARACTERISTICS:
                 (A)      LENGTH:  105
                 (B)      TYPE:  Nucleic Acid
                 (C)      STRANDEDNESS:  Single
                 (D)      TOPOLOGY:  Linear
        (ii)    KIND: cDNA to genomic RNA
        (ii)    KIND (if peptide or protein):
                 (A)      SEQUENCE ASSEMBLY METHOD:  Overlap
                 (B)      FRAGMENT TYPE:  Internal Fragment
                 (C)      HYPOTHETICAL: _____
        (iii)   ORIGINAL SOURCE: HIV
                 (E)      INDIVIDUAL ISOLATE: _____
        (iv)    IMMEDIATE SOURCE:
                 (C)      CLONE: _____
        (v)     POSITION IN GENOME: Within Env Gene
        (vi)    PROPERTIES OF SEQUENCE:  Expresses conserved antigenic
                determinant
        (viii)  SEQUENCE DESCRIPTION:

SEQ ID NO:  EE322-2

```
    1                 5                    10                   15
Cys Thr Arg Pro Asn Asn Asn Thr Arg Lys Ser Ile Pro Ile Gly
TGT ACA AGA CCC AAC AAC AAT ACA AGA AAA AGT ATA CCT ATA GGA
```

```
                          20                      25                      30
Pro Gly Arg Ala Phe Tyr Thr Thr Gly Glu Ile Ile Gly Asp Ile
CCA GGG AGA GCA TTT TAT ACA ACA GGA GAA ATA ATA GGA GAT ATA


                          35
Arg Gln Ala His Cys
AGA CAA GCA CAT TGT
```

(2)      INFORMATION FOR SEQ ID NO: EE322-3
         (i)      SEQUENCE CHARACTERISTICS:
                  (A)      LENGTH:  105
                  (B)      TYPE:  Nucleic Acid
                  (C)      STRANDEDNESS:  Single
                  (D)      TOPOLOGY:  Linear
         (ii)     KIND: cDNA to genomic RNA
         (ii)     KIND (if peptide or protein):
                  (A)      SEQUENCE ASSEMBLY METHOD:  Overlap
                  (B)      FRAGMENT TYPE:  Internal Fragment
                  (C)      HYPOTHETICAL:  _____
         (iii)    ORIGINAL SOURCE: HIV
                  (E)      INDIVIDUAL ISOLATE:  _____
         (iv)     IMMEDIATE SOURCE:
                  (C)      CLONE:  _____
         (v)      POSITION IN GENOME: Within Env Gene
         (vi)     PROPERTIES OF SEQUENCE:  Expresses conserved antigenic
                  determinant
         (viii)   SEQUENCE DESCRIPTION:


SEQ ID NO:   EE322-3


```
1                     5                      10                      15
Cys Thr Arg Pro Asn Asn Asn Thr Arg Lys Ser Ile Thr Ile Gly
TGT ACA AGA CCC AAT AAC AAT ACA AGA AAA AGT ATA ACT ATA GGA


                          20                      25                      30
Pro Gly Arg Ala Phe Tyr Ala Thr Gly Glu Ile Ile Gly Asp Ile
CCA GGG AGA GCA TTT TAT GCA ACA GGA GAA ATA ATA GGA GAT ATA


                          35
Arg Gln Ala His Cys
AGA CAA GCA CAT TGT
```

```
(2)      INFORMATION FOR SEQ ID NO: EE324-1
         (i)      SEQUENCE CHARACTERISTICS:
                  (A)      LENGTH:  105
                  (B)      TYPE:  Nucleic Acid
                  (C)      STRANDEDNESS:  Single
                  (D)      TOPOLOGY:  Linear
         (ii)     KIND: cDNA to genomic RNA
         (ii)     KIND (if peptide or protein):
                  (A)      SEQUENCE ASSEMBLY METHOD:  Overlap
                  (B)      FRAGMENT TYPE:  Internal Fragment
                  (C)      HYPOTHETICAL:  _____
         (iii)    ORIGINAL SOURCE: HIV
                  (E)      INDIVIDUAL ISOLATE:  _____
         (iv)     IMMEDIATE SOURCE:
                  (C)      CLONE:  _____
         (v)      POSITION IN GENOME: Within Env Gene
         (vi)     PROPERTIES OF SEQUENCE:  Expresses conserved antigenic
                  determinant
         (viii)   SEQUENCE DESCRIPTION:


SEQ ID NO:  EE324-1


  1                    5                    10                   15
Cys Thr Arg Pro Asn Asn Asn Thr Ile Lys Ser Ile His MET Gly
TGT ACA AGA CCC AAC AAC AAT ACA ATA AAA AGT ATA CAT ATG GGA


                       20                   25                   30
Leu Gly Arg Thr Phe Tyr Thr Thr Gly Glu Val Ile Gly Asp Ile
CTA GGG AGG ACA TTT TAT ACA ACA GGA GAA GTA ATA GGA GAT ATA


                       35
Arg Gln Ala His Cys
AGA CAA GCA CAT TGT


(2)      INFORMATION FOR SEQ ID NO: EE324-2
         (i)      SEQUENCE CHARACTERISTICS:
                  (A)      LENGTH:  105
                  (B)      TYPE:  Nucleic Acid
                  (C)      STRANDEDNESS:  Single
                  (D)      TOPOLOGY:  Linear
         (ii)     KIND: cDNA to genomic RNA
```

```
(ii)    KIND (if peptide or protein):
        (A)    SEQUENCE ASSEMBLY METHOD:  Overlap
        (B)    FRAGMENT TYPE:  Internal Fragment
        (C)    HYPOTHETICAL:  _____
(iii)   ORIGINAL SOURCE: HIV
        (E)    INDIVIDUAL ISOLATE:  _____
(iv)    IMMEDIATE SOURCE:
        (C)    CLONE:  _____
(v)     POSITION IN GENOME: Within Env Gene
(vi)    PROPERTIES OF SEQUENCE:  Expresses conserved antigenic
        determinant
(viii)  SEQUENCE DESCRIPTION:
```

SEQ ID NO:  EE324-2


```
1                5                    10                    15
Cys Thr Arg Pro Asn Asn Asn Thr Arg Lys Ser Ile His Leu Gly
TGT ACA AGA CCC AAC AAC AAT ACA AGA AAA AGT ATA CAT CTA GGG


                 20                   25                    30
Pro Gly Arg Ala Phe Tyr Thr Thr Gly Asp Ile Ile Gly Asp Ile
CCA GGG AGA GCA TTT TAT ACA ACA GGA GAC ATA ATA GGA GAT ATA


                 35
Gly Gln Ala His Cys
GGA CAA GCA CAT TGT
```


```
(2)     INFORMATION FOR SEQ ID NO: EE327-1
        (i)    SEQUENCE CHARACTERISTICS:
               (A)    LENGTH:  105
               (B)    TYPE:  Nucleic Acid
               (C)    STRANDEDNESS:  Single
               (D)    TOPOLOGY:  Linear
        (ii)   KIND: cDNA to genomic RNA
        (ii)   KIND (if peptide or protein):
               (A)    SEQUENCE ASSEMBLY METHOD:  Overlap
               (B)    FRAGMENT TYPE:  Internal Fragment
               (C)    HYPOTHETICAL:  _____
        (iii)  ORIGINAL SOURCE: HIV
               (E)    INDIVIDUAL ISOLATE:  _____
        (iv)   IMMEDIATE SOURCE:
               (C)    CLONE:  _____
        (v)    POSITION IN GENOME: Within Env Gene
        (vi)   PROPERTIES OF SEQUENCE:  Expresses conserved antigenic
               determinant
        (viii) SEQUENCE DESCRIPTION:
```

96

SEQ ID NO:   EE327-1

```
            1                  5                        10                       15
Cys Thr Arg Pro Asn Asn Asn Thr Arg Lys Gly Ile His Ile Gly
TGT ACA AGA CCC AAC AAC AAT ACA AGA AAA GGT ATA CAT ATA GGA


                               20                       25                       30
Pro Gly Arg Ala Phe Tyr Ala Thr Gly Asp Ile Ile Gly Asp Ile
CCA GGG AGA GCA TTT TAT GCA ACA GGA GAC ATA ATA GGA GAT ATA


                               35
Arg Gln Ala His Cys
AGA CAA GCA CAT TGT
```

```
(2)       INFORMATION FOR SEQ ID NO: EE327-2
          (i)       SEQUENCE CHARACTERISTICS:
                    (A)     LENGTH:  105
                    (B)     TYPE:  Nucleic Acid
                    (C)     STRANDEDNESS:  Single
                    (D)     TOPOLOGY:  Linear
          (ii)    KIND: cDNA to genomic RNA
          (ii)    KIND (if peptide or protein):
                    (A)     SEQUENCE ASSEMBLY METHOD:  Overlap
                    (B)     FRAGMENT TYPE:  Internal Fragment
                    (C)     HYPOTHETICAL:  _____
          (iii)   ORIGINAL SOURCE: HIV
                    (E)     INDIVIDUAL ISOLATE:  _____
          (iv)    IMMEDIATE SOURCE:
                    (C)     CLONE:  _____
          (v)     POSITION IN GENOME: Within Env Gene
          (vi)    PROPERTIES OF SEQUENCE:  Expresses conserved antigenic
                  determinant
          (viii)  SEQUENCE DESCRIPTION:
```

SEQ ID NO:   EE327-2

```
            1                  5                        10                       15
Cys Thr Arg Pro Asn Asn Asn Thr Arg Lys Gly Ile His Ile Gly
TGT ACA AGA CCC AAC AAC AAT ACA AGA AAA GGT ATA CAT ATA GGA
```

```
                     20                    25                    30
Pro Gly Arg Ala Phe Tyr Ala Thr Gly Asp Ile Ile Gly Asp Ile
CCA GGG AGA GCA TTT TAT GCA ACA GGA GAC ATA ATA GGA GAT ATA


                     35
Arg Gln Ala His Tyr
AGA CAA GCA CAT TAT
```

(2)      INFORMATION FOR SEQ ID NO: EE327-3
    (i)      SEQUENCE CHARACTERISTICS:
        (A)      LENGTH:  105
        (B)      TYPE:  Nucleic Acid
        (C)      STRANDEDNESS:  Single
        (D)      TOPOLOGY:  Linear
    (ii)     KIND: cDNA to genomic RNA
    (ii)     KIND (if peptide or protein):
        (A)      SEQUENCE ASSEMBLY METHOD:  Overlap
        (B)      FRAGMENT TYPE:  Internal Fragment
        (C)      HYPOTHETICAL:  _____
    (iii)    ORIGINAL SOURCE: HIV
        (E)      INDIVIDUAL ISOLATE:  _____
    (iv)     IMMEDIATE SOURCE:
        (C)      CLONE:  _____
    (v)      POSITION IN GENOME: Within Env Gene
    (vi)     PROPERTIES OF SEQUENCE:  Expresses conserved antigenic
        determinant
    (viii)   SEQUENCE DESCRIPTION:


SEQ ID NO:  EE327-3


```
1                    5                    10                   15
Cys Thr Arg Pro Asn Asn Asn Thr Arg Lys Gly Ile His Ile Gly
TGT ACA AGA CCC AAC AAC AAT ACA AGA AAA GGT ATA CAT ATA GGA


                     20                    25                    30
Pro Gly Arg Ala Phe Tyr Ala Thr Gly Asp Ile Ile Gly Asp Ile
CCA GGG AGA GCA TTT TAT GCA ACA GGA GAC ATA ATA GGA GAT ATA


                     35
Arg Gln Ala His Cys
AGA CAA GCA CAT TGT
```

(2)      INFORMATION FOR SEQ ID NO: EE345-1
      (i)      SEQUENCE CHARACTERISTICS:
            (A)      LENGTH:  105
            (B)      TYPE:  Nucleic Acid
            (C)      STRANDEDNESS:  Single
            (D)      TOPOLOGY:  Linear
      (ii)    KIND: cDNA to genomic RNA
      (ii)    KIND (if peptide or protein):
            (A)      SEQUENCE ASSEMBLY METHOD:  Overlap
            (B)      FRAGMENT TYPE:  Internal Fragment
            (C)      HYPOTHETICAL:  _____
      (iii)   ORIGINAL SOURCE: HIV
            (E)      INDIVIDUAL ISOLATE:  _____
      (iv)    IMMEDIATE SOURCE:
            (C)      CLONE:  _____
      (v)     POSITION IN GENOME: Within Env Gene
      (vi)    PROPERTIES OF SEQUENCE:  Expresses conserved antigenic
            determinant
      (viii)  SEQUENCE DESCRIPTION:


SEQ ID NO:  EE345-1


```
1                   5                       10                      15
Cys Thr Arg Pro Ser Asn Asn Thr Arg Lys Gly Ile His Ile Gly
TGT ACA AGA CCC AGC AAT AAT ACA AGA AAA GGT ATA CAT ATA GGG


                    20                      25                      30
Pro Gly Arg Ala Phe Tyr Ala Thr Gly Glu Ile Thr Gly Asp Ile
CCA GGG AGA GCA TTT TAT GCA ACG GGA GAG ATA ACA GGA GAT ATA


                    35
Arg Gln Ala His Cys
AGA CAA GCA CAT TGT
```


(2)      INFORMATION FOR SEQ ID NO: EE345-2
      (i)      SEQUENCE CHARACTERISTICS:
            (A)      LENGTH:  105
             (B)      TYPE:  Nucleic Acid
             (C)      STRANDEDNESS:  Single
             (D)      TOPOLOGY:  Linear
      (ii)    KIND: cDNA to genomic RNA

```
         (ii)    KIND (if peptide or protein):
                 (A)    SEQUENCE ASSEMBLY METHOD:  Overlap
                 (B)    FRAGMENT TYPE:  Internal Fragment
                 (C)    HYPOTHETICAL: _____
         (iii)   ORIGINAL SOURCE: HIV
                 (E)    INDIVIDUAL ISOLATE: _____
         (iv)    IMMEDIATE SOURCE:
                 (C)    CLONE: _____
         (v)     POSITION IN GENOME: Within Env Gene
         (vi)    PROPERTIES OF SEQUENCE:  Expresses conserved antigenic
                 determinant
         (viii)  SEQUENCE DESCRIPTION:
```

SEQ ID NO:  EE345-2

```
   1                 5                      10                     15
Cys Thr Arg Pro Ser Asn Asn Thr Arg Lys Gly Ile His Ile Gly
TGT ACA AGA CCC AGC AAT AAT ACA AGA AAA GGT ATA CAT ATA GGG


                   20                     25                     30
Pro Gly Arg Ala Phe Phe Thr Thr Gly Glu Ile Thr Gly Asp Ile
CCA GGG AGA GCA TTT TTT ACA ACA GGA GAA ATA ACA GGA GAT ATA


                   35
Arg Gln Ala His Cys
AGA CAA GCA CAT TGT
```

```
   (2)    INFORMATION FOR SEQ ID NO: EE345-3
          (i)     SEQUENCE CHARACTERISTICS:
                  (A)    LENGTH:  105
                  (B)    TYPE:  Nucleic Acid
                  (C)    STRANDEDNESS:  Single
                  (D)    TOPOLOGY:  Linear
          (ii)    KIND: cDNA to genomic RNA
          (ii)    KIND (if peptide or protein):
                  (A)    SEQUENCE ASSEMBLY METHOD:  Overlap
                  (B)    FRAGMENT TYPE:  Internal Fragment
                  (C)    HYPOTHETICAL: _____
          (iii)   ORIGINAL SOURCE: HIV
                  (E)    INDIVIDUAL ISOLATE: _____
          (iv)    IMMEDIATE SOURCE:
                  (C)    CLONE: _____
          (v)     POSITION IN GENOME: Within Env Gene
          (vi)    PROPERTIES OF SEQUENCE:  Expresses conserved antigenic
                  determinant
          (viii)  SEQUENCE DESCRIPTION:
```

SEQ ID NO:   EE345-3

```
          1                    5                        10                          15
Cys Thr Arg Pro Ser Asn Asn Thr Arg Lys Ser Ile His Ile Gly
TGT ACA AGA CCC AGC AAT AAT ACA AGA AAA AGT ATA CAT ATA GGG


                               20                       25                          30
Pro Gly Arg Ala Phe Tyr Ala Thr Gly Glu Ile Thr Gly Asp Ile
CCA GGG AGA GCA TTT TAT GCA ACG GGA GAG ATA ACA GGA GAT ATA


                               35
Arg Gln Ala His Cys
AGA CAA GCA CAT TGT
```

(2)       INFORMATION FOR SEQ ID NO: EE356-1
          (i)       SEQUENCE CHARACTERISTICS:
                    (A)      LENGTH:   105
                    (B)      TYPE:  Nucleic Acid
                    (C)      STRANDEDNESS:   Single
                    (D)      TOPOLOGY:   Linear
          (ii)      KIND: cDNA to genomic RNA
          (ii)      KIND (if peptide or protein):
                    (A)      SEQUENCE ASSEMBLY METHOD:  Overlap
                    (B)      FRAGMENT TYPE:   Internal Fragment
                    (C)      HYPOTHETICAL:  _____
          (iii)     ORIGINAL SOURCE: HIV
                    (E)      INDIVIDUAL ISOLATE:  _____
          (iv)      IMMEDIATE SOURCE:
                    (C)      CLONE:  _____
          (v)       POSITION IN GENOME: Within Env Gene
          (vi)      PROPERTIES OF SEQUENCE:  Expresses conserved antigenic
                    determinant
          (viii)    SEQUENCE DESCRIPTION:


SEQ ID NO:   EE356-1


```
          1                    5                        10                          15
Cys Ile Arg Pro Ser Asn Asn Thr Arg Lys Ser Ile Thr Ile Gly
TGT ATA AGA CCC AGC AAC AAT ACA AGA AAA AGT ATA ACT ATA GGA
```

```
                       20                    25                    30
Pro Gly Arg Ala Phe Phe Ala Thr Gly Glu Ile Thr Gly Asp Ile
CCA GGG AGA GCA TTT TTT GCA ACA GGA GAA ATA ACA GGA GAT ATA


                       35
Arg Gln Ala His Cys
AGA CAA GCA CAT TGT
```

(2)     INFORMATION FOR SEQ ID NO: EE356-2
        (i)     SEQUENCE CHARACTERISTICS:
                (A)     LENGTH:  105
                (B)     TYPE:  Nucleic Acid
                (C)     STRANDEDNESS:  Single
                (D)     TOPOLOGY:  Linear
        (ii)    KIND: cDNA to genomic RNA
        (ii)    KIND (if peptide or protein):
                (A)     SEQUENCE ASSEMBLY METHOD:  Overlap
                (B)     FRAGMENT TYPE:  Internal Fragment
                (C)     HYPOTHETICAL:  _____
        (iii)   ORIGINAL SOURCE: HIV
                (E)     INDIVIDUAL ISOLATE:  _____
        (iv)    IMMEDIATE SOURCE:
                (C)     CLONE:  _____
        (v)     POSITION IN GENOME: Within Env Gene
        (vi)    PROPERTIES OF SEQUENCE:  Expresses conserved antigenic
                determinant
        (viii)  SEQUENCE DESCRIPTION:


SEQ ID NO:   EE356-2


```
1                     5                     10                    15
Cys Ile Arg Pro Ser Asn Asn Thr Arg Lys Ser Ile Thr Ile Gly
TGT ATA AGA CCC AGC AAC AAT ACA AGA AAA AGT ATA ACT ATA GGA


                       20                    25                    30
Pro Gly Arg Ala Phe Phe Ala Thr Gly Glu Ile Thr Gly Asp Ile
CCA GGG AGA GCA TTT TTT GCA ACA GGA GAA ATA ACA GGA GAT ATA


                       35
Arg Gln Ala His Cys
AGA CAA GCA CAT TGT
```

(2)      INFORMATION FOR SEQ ID NO: EE356-3
      (i)      SEQUENCE CHARACTERISTICS:
            (A)      LENGTH:  105
            (B)      TYPE:  Nucleic Acid
            (C)      STRANDEDNESS:  Single
            (D)      TOPOLOGY:  Linear
      (ii)    KIND: cDNA to genomic RNA
      (ii)    KIND (if peptide or protein):
            (A)      SEQUENCE ASSEMBLY METHOD:  Overlap
            (B)      FRAGMENT TYPE:  Internal Fragment
            (C)      HYPOTHETICAL: _____
      (iii)   ORIGINAL SOURCE: HIV
            (E)      INDIVIDUAL ISOLATE: _____
      (iv)    IMMEDIATE SOURCE:
            (C)      CLONE: _____
      (v)     POSITION IN GENOME: Within Env Gene
      (vi)    PROPERTIES OF SEQUENCE:  Expresses conserved antigenic
            determinant
      (viii)  SEQUENCE DESCRIPTION:


SEQ ID NO:  EE356-3


```
 1                    5                    10                   15
Cys Ile Arg Pro Ser Asn Asn Thr Arg Lys Ser Ile Thr Ile Gly
TGT ATA AGA CCC AGC AAC AAT ACA AGA AAA AGT ATA ACT ATA GGA


                     20                   25                   30
Pro Gly Arg Ala Phe Phe Ala Thr Gly Glu Ile Thr Gly Asp Ile
CCA GGG AGA GCA TTT TTT GCA ACA GGA GAA ATA ACA GGA GAT ATA


                     35
Arg Gln Ala His Cys
AGA CAA GCA CAT TGT
```


(2)      INFORMATION FOR SEQ ID NO: EE359-1
      (i)      SEQUENCE CHARACTERISTICS:
            (A)      LENGTH:  105
            (B)      TYPE:  Nucleic Acid
            (C)      STRANDEDNESS:  Single
            (D)      TOPOLOGY:  Linear
      (ii)    KIND: cDNA to genomic RNA

```
(ii)     KIND (if peptide or protein):
         (A)     SEQUENCE ASSEMBLY METHOD:  Overlap
         (B)     FRAGMENT TYPE:  Internal Fragment
         (C)     HYPOTHETICAL:  _____
(iii)    ORIGINAL SOURCE: HIV
         (E)     INDIVIDUAL ISOLATE:  _____
(iv)     IMMEDIATE SOURCE:
         (C)     CLONE:  _____
(v)      POSITION IN GENOME: Within Env Gene
(vi)     PROPERTIES OF SEQUENCE:  Expresses conserved antigenic
         determinant
(viii)   SEQUENCE DESCRIPTION:
```

SEQ ID NO:  EE359-1

```
1              5                  10                 15
Cys Thr Arg Pro Asn Asn Asn Thr Arg Arg Ser Ile Asn Ile Gly
TGT ACA AGA CCC AAC AAC AAT ACA AGA AGA AGT ATA AAT ATA GGA


               20                 25                 30
Pro Gly Arg Ala Phe Tyr Ala Thr Gly Asp Ile Ile Gly Asp Ile
CCA GGG AGA GCC TTT TAT GCA ACA GGA GAC ATA ATA GGA GAT ATA


               35
Arg Gln Ala His Cys
AGA CAA GCA CAT TGT
```

```
(2)      INFORMATION FOR SEQ ID NO: EE359-2
         (i)     SEQUENCE CHARACTERISTICS:
                 (A)     LENGTH:  105
                 (B)     TYPE:  Nucleic Acid
                 (C)     STRANDEDNESS:  Single
                 (D)     TOPOLOGY:  Linear
         (ii)    KIND: cDNA to genomic RNA
         (ii)    KIND (if peptide or protein):
                 (A)     SEQUENCE ASSEMBLY METHOD:  Overlap
                 (B)     FRAGMENT TYPE:  Internal Fragment
                 (C)     HYPOTHETICAL:  _____
         (iii)   ORIGINAL SOURCE: HIV
                 (E)     INDIVIDUAL ISOLATE:  _____
         (iv)    IMMEDIATE SOURCE:
                 (C)     CLONE:  _____
         (v)     POSITION IN GENOME: Within Env Gene
         (vi)    PROPERTIES OF SEQUENCE:  Expresses conserved antigenic
                 determinant
         (viii)  SEQUENCE DESCRIPTION:
```

SEQ ID NO:  EE359-2

```
        1                  5                      10                        15
Cys Thr Arg Pro Asn Asp Asn Thr Arg Arg Ser Ile Asn Ile Gly
TGT ACA AGA CCC AAC GAC AAT ACA AGA AGA AGT ATA AAT ATA GGA


                          20                      25                        30
Pro Gly Arg Ala Phe Tyr Ala Thr Gly Asp Ile Ile Gly Asp Ile
CCA GGG AGA GCC TTT TAT GCA ACA GGA GAC ATA ATA GGA GAT ATA


                          35
Arg Gln Ala His Cys
AGA CAA GCA CAT TGT
```

(2)      INFORMATION FOR SEQ ID NO: EE359-3
         (i)      SEQUENCE CHARACTERISTICS:
                  (A)      LENGTH:  105
                  (B)      TYPE:  Nucleic Acid
                  (C)      STRANDEDNESS:  Single
                  (D)      TOPOLOGY:  Linear
         (ii)     KIND: cDNA to genomic RNA
         (ii)     KIND (if peptide or protein):
                  (A)      SEQUENCE ASSEMBLY METHOD:  Overlap
                  (B)      FRAGMENT TYPE:  Internal Fragment
                  (C)      HYPOTHETICAL:  _____
         (iii)    ORIGINAL SOURCE: HIV
                  (E)      INDIVIDUAL ISOLATE:  _____
         (iv)     IMMEDIATE SOURCE:
                  (C)      CLONE:  _____
         (v)      POSITION IN GENOME: Within Env Gene
         (vi)     PROPERTIES OF SEQUENCE:  Expresses conserved antigenic
                  determinant
         (viii)   SEQUENCE DESCRIPTION:


SEQ ID NO:  EE359-3

```
        1                  5                      10                        15
Cys Thr Arg Pro Asn Asp Asn Thr Arg Arg Ser Ile Asn Ile Gly
TGT ACA AGA CCC AAC GAC AAT ACA AGA AGA AGT ATA AAT ATA GGA
```

```
                    20                      25                      30
Pro Gly Arg Ala Phe Tyr Ala Thr Gly Asp Ile Ile Gly Asp Ile
CCA GGG AGA GCC TTT TAT GCA ACA GGA GAC ATA ATA GGA GAT ATA


                    35
Arg Gln Ala His Cys
AGA CAA GCA CAT TGT
```

(2)     INFORMATION FOR SEQ ID NO: EE360-1
        (i)      SEQUENCE CHARACTERISTICS:
                 (A)      LENGTH:  105
                 (B)      TYPE:  Nucleic Acid
                 (C)      STRANDEDNESS:  Single
                 (D)      TOPOLOGY:  Linear
        (ii)     KIND: cDNA to genomic RNA
        (ii)     KIND (if peptide or protein):
                 (A)      SEQUENCE ASSEMBLY METHOD:  Overlap
                 (B)      FRAGMENT TYPE:  Internal Fragment
                 (C)      HYPOTHETICAL:  _____
        (iii)    ORIGINAL SOURCE: HIV
                 (E)      INDIVIDUAL ISOLATE:  _____
        (iv)     IMMEDIATE SOURCE:
                 (C)      CLONE:  _____
        (v)      POSITION IN GENOME: Within Env Gene
        (vi)     PROPERTIES OF SEQUENCE:  Expresses conserved antigenic
                 determinant
        (viii)   SEQUENCE DESCRIPTION:


SEQ ID NO:   EE360-1


```
1                    5                      10                      15
Cys Thr Arg Pro Ser Asn Asn Thr Arg Lys Ser Ile His Ile Ala
TGC ACA AGG CCC AGC AAC AAT ACA AGA AAA AGT ATA CAT ATA GCA


                    20                      25                      30
Pro Gly Arg Ala Phe Tyr Thr Thr Gly Ala Ile Thr Gly Asp Ile
CCA GGG AGA GCA TTT TAT ACA ACA GGA GCA ATA ACA GGA GAT ATA


                    35
Arg Gln Ala His Cys
AGA CAA GCA CAT TGT
```

106

```
(2)      INFORMATION FOR SEQ ID NO: EE360-2
         (i)      SEQUENCE CHARACTERISTICS:
                  (A)      LENGTH:  105
                  (B)      TYPE:  Nucleic Acid
                  (C)      STRANDEDNESS:  Single
                  (D)      TOPOLOGY:  Linear
         (ii)     KIND: cDNA to genomic RNA
         (ii)     KIND (if peptide or protein):
                  (A)      SEQUENCE ASSEMBLY METHOD:  Overlap
                  (B)      FRAGMENT TYPE:  Internal Fragment
                  (C)      HYPOTHETICAL:  _____
         (iii)    ORIGINAL SOURCE: HIV
                  (E)      INDIVIDUAL ISOLATE:  _____
         (iv)     IMMEDIATE SOURCE:
                  (C)      CLONE:  _____
         (v)      POSITION IN GENOME: Within Env Gene
         (vi)     PROPERTIES OF SEQUENCE:  Expresses conserved antigenic
                  determinant
         (viii)   SEQUENCE DESCRIPTION:


SEQ ID NO:  EE360-2


 1                 5                      10                     15
Cys Thr Arg Pro Ser Asn Asn Thr Arg Lys Ser Ile His Ile Ala
TGC ACA AGG CCC AGC AAC AAT ACA AGA AAA AGT ATA CAT ATA GCA


                   20                     25                     30
Pro Gly Arg Ala Phe Tyr Thr Thr Gly Ala Ile Thr Gly Asp Ile
CCA GGG AGA GCA TTT TAT ACA ACA GGA GCA ATA ACA GGA GAT ATA


                   35
Arg Gln Ala His Cys
AGA CAA GCA CAT TGT


(2)      INFORMATION FOR SEQ ID NO: EE360-3
         (i)      SEQUENCE CHARACTERISTICS:
                  (A)      LENGTH:  105
                  (B)      TYPE:  Nucleic Acid
                  (C)      STRANDEDNESS:  Single
                  (D)      TOPOLOGY:  Linear
         (ii)     KIND: cDNA to genomic RNA
```

```
(ii)     KIND (if peptide or protein):
         (A)     SEQUENCE ASSEMBLY METHOD:  Overlap
         (B)     FRAGMENT TYPE:  Internal Fragment
         (C)     HYPOTHETICAL:  _____
(iii)    ORIGINAL SOURCE: HIV
         (E)     INDIVIDUAL ISOLATE:  _____
(iv)     IMMEDIATE SOURCE:
         (C)     CLONE:  _____
(v)      POSITION IN GENOME: Within Env Gene
(vi)     PROPERTIES OF SEQUENCE:  Expresses conserved antigenic
         determinant
(viii)   SEQUENCE DESCRIPTION:
```

SEQ ID NO:  EE360-3

```
 1                   5                   10                  15
Cys Thr Arg Pro Ser Asn Asn Thr Arg Lys Ser Ile His Ile Ala
TGC ACA AGG CCC AGC AAC AAT ACA AGA AAA AGT ATA CAT ATA GCA


                    20                  25                  30
Pro Gly Arg Ala Phe Tyr Thr Thr Gly Ala Ile Thr Gly Asp Ile
CCA GGG AGA GCA TTT TAT ACA ACA GGA GCA ATA ACA GGA GAT ATA


                    35
Arg Gln Ala His Cys
AGA CAA GCA CAT TGT
```

```
(2)     INFORMATION FOR SEQ ID NO: EE367-1
        (i)      SEQUENCE CHARACTERISTICS:
                 (A)     LENGTH:  105
                 (B)     TYPE:  Nucleic Acid
                 (C)     STRANDEDNESS:  Single
                 (D)     TOPOLOGY:  Linear
        (ii)     KIND: cDNA to genomic RNA
        (ii)     KIND (if peptide or protein):
                 (A)     SEQUENCE ASSEMBLY METHOD:  Overlap
                 (B)     FRAGMENT TYPE:  Internal Fragment
                 (C)     HYPOTHETICAL:  _____
        (iii)    ORIGINAL SOURCE: HIV
                 (E)     INDIVIDUAL ISOLATE:  _____
        (iv)     IMMEDIATE SOURCE:
                 (C)     CLONE:  _____
        (v)      POSITION IN GENOME: Within Env Gene
        (vi)     PROPERTIES OF SEQUENCE:  Expresses conserved antigenic
                 determinant
        (viii)   SEQUENCE DESCRIPTION:
```

SEQ ID NO: EE367-1


```
          1              5                   10                  15
Cys Thr Arg Pro Asn Asn Asn Thr Ile Lys Ser Ile His MET Gly
TGT ACA AGA CCC AAC AAC AAT ACA ATA AAA AGT ATA CAT ATG GGA


                         20                  25                  30
Leu Gly Arg Thr Phe Tyr Thr Thr Gly Glu Val Ile Gly Asp Ile
CTA GGG AGG ACA TTT TAT ACA ACA GGA GAA GTA ATA GGA GAT ATA


                         35
Arg Gln Ala His Cys
AGA CAA GCA CAT TGT
```


(2)      INFORMATION FOR SEQ ID NO: EE367-2
         (i)      SEQUENCE CHARACTERISTICS:
                  (A)      LENGTH:  105
                  (B)      TYPE:  Nucleic Acid
                  (C)      STRANDEDNESS:  Single
                  (D)      TOPOLOGY:  Linear
         (ii)     KIND: cDNA to genomic RNA
         (ii)     KIND (if peptide or protein):
                  (A)      SEQUENCE ASSEMBLY METHOD:  Overlap
                  (B)      FRAGMENT TYPE:  Internal Fragment
                  (C)      HYPOTHETICAL:  _____
         (iii)    ORIGINAL SOURCE: HIV
                  (E)      INDIVIDUAL ISOLATE:  _____
         (iv)     IMMEDIATE SOURCE:
                  (C)      CLONE:  _____
         (v)      POSITION IN GENOME: Within Env Gene
         (vi)     PROPERTIES OF SEQUENCE:  Expresses conserved antigenic
                  determinant
         (viii)   SEQUENCE DESCRIPTION:


SEQ ID NO: EE367-2


```
          1              5                   10                  15
Cys Thr Arg Pro Asn Asn Asn Thr Ile Lys Ser Ile His MET Gly
TGT ACA AGA CCC AAC AAC AAT ACA ATA AAA AGT ATA CAT ATG GGA
```

```
                                    20                        25                        30
Leu Gly Arg Thr Phe Tyr Thr Thr Gly Glu Val Ile Gly Asp Ile
CTA GGG AGG ACA TTT TAT ACA ACA GGA GAA GTA ATA GGA GAT ATA


                                    35
Arg Gln Ala His Cys
AGA CAA GCA CAT TGT
```

(2)      INFORMATION FOR SEQ ID NO: EE367-3
         (i)      SEQUENCE CHARACTERISTICS:
                  (A)      LENGTH:  105
                  (B)      TYPE:  Nucleic Acid
                  (C)      STRANDEDNESS:  Single
                  (D)      TOPOLOGY:  Linear
         (ii)     KIND: cDNA to genomic RNA
         (ii)     KIND (if peptide or protein):
                  (A)      SEQUENCE ASSEMBLY METHOD:  Overlap
                  (B)      FRAGMENT TYPE:  Internal Fragment
                  (C)      HYPOTHETICAL:  _____
         (iii)    ORIGINAL SOURCE: HIV
                  (E)      INDIVIDUAL ISOLATE:  _____
         (iv)     IMMEDIATE SOURCE:
                  (C)      CLONE:  _____
         (v)      POSITION IN GENOME: Within Env Gene
         (vi)     PROPERTIES OF SEQUENCE:  Expresses conserved antigenic
                  determinant
         (viii)   SEQUENCE DESCRIPTION:


SEQ ID NO:  EE367-3


```
1                       5                        10                        15
Cys Thr Arg Pro Asn Asn Asn Thr Ile Lys Ser Ile His MET Gly
TGT ACA AGA CCC AAC AAC AAT ACA ATA AAA AGT ATA CAT ATG GGA


                                    20                        25                        30
Leu Gly Arg Thr Phe Tyr Thr Thr Gly Glu Val Ile Gly Asp Ile
CTA GGG AGG ACA TTT TAT ACA ACA GGA GAA GTA ATA GGA GAT ATA


                                    35
Arg Gln Ala His Cys
AGA CAA GCA CAT TGT
```

(2)      INFORMATION FOR SEQ ID NO: EE370-1
         (i)      SEQUENCE CHARACTERISTICS:
                  (A)      LENGTH:  105
                  (B)      TYPE:  Nucleic Acid
                  (C)      STRANDEDNESS:  Single
                  (D)      TOPOLOGY:  Linear
         (ii)    KIND: cDNA to genomic RNA
         (ii)    KIND (if peptide or protein):
                  (A)      SEQUENCE ASSEMBLY METHOD:  Overlap
                  (B)      FRAGMENT TYPE:  Internal Fragment
                  (C)      HYPOTHETICAL:  _____
         (iii)   ORIGINAL SOURCE: HIV
                  (E)      INDIVIDUAL ISOLATE:  _____
         (iv)    IMMEDIATE SOURCE:
                  (C)      CLONE:  _____
         (v)     POSITION IN GENOME: Within Env Gene
         (vi)    PROPERTIES OF SEQUENCE:  Expresses conserved antigenic
                  determinant
         (viii)  SEQUENCE DESCRIPTION:


SEQ ID NO:   EE370-1


```
1                 5                   10                  15
Cys Thr Arg Pro Asn Asn Asn Thr Arg Lys Ser Ile His Ile Gly
TGT ACA AGA CCC AAC AAC AAT ACA AGA AAA AGT ATA CAT ATA GGA


                  20                  25                  30
Pro Gly Arg Ala Phe Tyr Thr Thr Gly Asp Ile Ile Gly Asp Ile
CCA GGA AGA GCA TTT TAT ACA ACA GGA GAC ATA ATA GGA GAT ATA


                  35
Arg Gln Ala His Cys
AGA CAA GCA CAT TGT
```


(2)      INFORMATION FOR SEQ ID NO: EE370-2
         (i)      SEQUENCE CHARACTERISTICS:
                  (A)      LENGTH:  105
                  (B)      TYPE:  Nucleic Acid
                  (C)      STRANDEDNESS:  Single
                  (D)      TOPOLOGY:  Linear
         (ii)    KIND: cDNA to genomic RNA

```
      (ii)    KIND (if peptide or protein):
              (A)    SEQUENCE ASSEMBLY METHOD:  Overlap
              (B)    FRAGMENT TYPE:  Internal Fragment
              (C)    HYPOTHETICAL:  _____
      (iii)   ORIGINAL SOURCE: HIV
              (E)    INDIVIDUAL ISOLATE:  _____
      (iv)    IMMEDIATE SOURCE:
              (C)    CLONE:  _____
      (v)     POSITION IN GENOME: Within Env Gene
      (vi)    PROPERTIES OF SEQUENCE:  Expresses conserved antigenic
              determinant
      (viii)  SEQUENCE DESCRIPTION:
```

SEQ ID NO:  EE370-2

```
   1                 5                      10                      15
Cys Thr Arg Pro Asn Asn Asn Thr Arg Lys Ser Ile His Ile Gly
TGT ACA AGA CCC AAC AAC AAT ACA AGA AAA AGT ATA CAT ATA GGA


                   20                     25                      30
Pro Gly Arg Ala Phe Tyr Thr Thr Gly Asp Ile Ile Gly Asp Ile
CCA GGA AGA GCA TTT TAT ACA ACA GGA GAC ATA ATA GGA GAT ATA


                   35
Arg Gln Ala His Cys
AGA CAA GCA CAT TGT
```

```
   (2)     INFORMATION FOR SEQ ID NO: EE370-3
           (i)     SEQUENCE CHARACTERISTICS:
                   (A)    LENGTH:  105
                   (B)    TYPE:  Nucleic Acid
                   (C)    STRANDEDNESS:  Single
                   (D)    TOPOLOGY:  Linear
           (ii)    KIND: cDNA to genomic RNA
           (ii)    KIND (if peptide or protein):
                   (A)    SEQUENCE ASSEMBLY METHOD:  Overlap
                   (B)    FRAGMENT TYPE:  Internal Fragment
                   (C)    HYPOTHETICAL:  _____
           (iii)   ORIGINAL SOURCE: HIV
                   (E)    INDIVIDUAL ISOLATE:  _____
           (iv)    IMMEDIATE SOURCE:
                   (C)    CLONE:  _____
           (v)     POSITION IN GENOME: Within Env Gene
           (vi)    PROPERTIES OF SEQUENCE:  Expresses conserved antigenic
                   determinant
           (viii)  SEQUENCE DESCRIPTION:
```

SEQ ID NO:  EE370-3

| 1 | | | | 5 | | | | | 10 | | | | | 15 |
|---|---|---|---|---|---|---|---|---|---|---|---|---|---|---|
| Cys | Thr | Arg | Pro | Asn | Asn | Asn | Thr | Arg | Lys | Ser | Ile | His | Ile | Gly |
| TGT | ACA | AGA | CCC | AAC | AAC | AAT | ACA | AGA | AAA | AGT | ATA | CAT | ATA | GGA |

| | | | | 20 | | | | | 25 | | | | | 30 |
|---|---|---|---|---|---|---|---|---|---|---|---|---|---|---|
| Pro | Gly | Arg | Ala | Phe | Tyr | Thr | Thr | Gly | Asp | Ile | Ile | Gly | Asp | Ile |
| CCA | GGA | AGA | GCA | TTT | TAT | ACA | ACA | GGA | GAC | ATA | ATA | GGA | GAT | ATA |

| | | | | 35 |
|---|---|---|---|---|
| Arg | Gln | Ala | His | Cys |
| AGA | CAA | GCA | CAT | TGT |

```
(2)       INFORMATION FOR SEQ ID NO: EE374-1
          (i)      SEQUENCE CHARACTERISTICS:
                   (A)      LENGTH:  105
                   (B)      TYPE:  Nucleic Acid
                   (C)      STRANDEDNESS:  Single
                   (D)      TOPOLOGY:  Linear
          (ii)     KIND: cDNA to genomic RNA
          (ii)     KIND (if peptide or protein):
                   (A)      SEQUENCE ASSEMBLY METHOD:  Overlap
                   (B)      FRAGMENT TYPE:  Internal Fragment
                   (C)      HYPOTHETICAL:  _____
          (iii)    ORIGINAL SOURCE: HIV
                   (E)      INDIVIDUAL ISOLATE:  _____
          (iv)     IMMEDIATE SOURCE:
                   (C)      CLONE:  _____
          (v)      POSITION IN GENOME: Within Env Gene
          (vi)     PROPERTIES OF SEQUENCE:  Expresses conserved antigenic
                   determinant
          (viii)   SEQUENCE DESCRIPTION:
```

SEQ ID NO:  EE374-1

| 1 | | | | 5 | | | | | 10 | | | | | 15 |
|---|---|---|---|---|---|---|---|---|---|---|---|---|---|---|
| Cys | Ile | Arg | Pro | Asn | Asn | Asn | Thr | Arg | Lys | Ser | Ile | His | Ile | Gly |
| TGT | ATA | AGA | CCC | AAC | AAC | AAT | ACA | AGA | AAA | AGT | ATA | CAT | ATA | GGA |

```
                     20                 25                 30
Pro Gly Arg Ala Phe Tyr Thr Thr Gly Thr Ile Ile Gly Asp Ile
CCA GGG AGA GCA TTT TAT ACA ACA GGA ACA ATA ATA GGA GAT ATA


                     35
Arg Gln Ala His Cys
AGA CAA GCA CAT TGT
```

(2)      INFORMATION FOR SEQ ID NO: EE374-2
    (i)      SEQUENCE CHARACTERISTICS:
        (A)      LENGTH:  105
        (B)      TYPE:  Nucleic Acid
        (C)      STRANDEDNESS:  Single
        (D)      TOPOLOGY:  Linear
    (ii)     KIND: cDNA to genomic RNA
    (ii)     KIND (if peptide or protein):
        (A)      SEQUENCE ASSEMBLY METHOD:  Overlap
        (B)      FRAGMENT TYPE:  Internal Fragment
        (C)      HYPOTHETICAL:  _____
    (iii)    ORIGINAL SOURCE: HIV
        (E)      INDIVIDUAL ISOLATE:  _____
    (iv)     IMMEDIATE SOURCE:
        (C)      CLONE:  _____
    (v)      POSITION IN GENOME: Within Env Gene
    (vi)     PROPERTIES OF SEQUENCE:  Expresses conserved antigenic
        determinant
    (viii)   SEQUENCE DESCRIPTION:

SEQ ID NO:  EE374-2

```
     1                 5                 10                15
Cys Ile Arg Pro Asn Asn Asn Thr Arg Lys Ser Ile His Ile Gly
TGT ATA AGA CCC AAC AAC AAT ACA AGA AAA AGT ATA CAT ATA GGA


                     20                 25                 30
Pro Gly Arg Ala Phe Tyr Thr Thr Gly Thr Ile Ile Gly Asp Ile
CCA GGG AGA GCA TTT TAT ACA ACA GGA ACA ATA ATA GGA GAT ATA


                     35
Arg Gln Ala His Cys
AGA CAA GCA CAT TGT
```

(2)      INFORMATION FOR SEQ ID NO: EE374-3
         (i)      SEQUENCE CHARACTERISTICS:
                  (A)      LENGTH:  105
                  (B)      TYPE:  Nucleic Acid
                  (C)      STRANDEDNESS:  Single
                  (D)      TOPOLOGY:  Linear
         (ii)     KIND: cDNA to genomic RNA
         (ii)     KIND (if peptide or protein):
                  (A)      SEQUENCE ASSEMBLY METHOD:  Overlap
                  (B)      FRAGMENT TYPE:  Internal Fragment
                  (C)      HYPOTHETICAL:  _____
         (iii)    ORIGINAL SOURCE: HIV
                  (E)      INDIVIDUAL ISOLATE:  _____
         (iv)     IMMEDIATE SOURCE:
                  (C)      CLONE:  _____
         (v)      POSITION IN GENOME: Within Env Gene
         (vi)     PROPERTIES OF SEQUENCE:  Expresses conserved antigenic
                  determinant
         (viii)   SEQUENCE DESCRIPTION:


SEQ ID NO:  EE374-3


1                    5                      10                    15
Cys Ile Arg Pro Asn Asn Asn Thr Arg Lys Ser Ile His Ile Gly
TGT ATA AGA CCC AAC AAC AAT ACA AGA AAA AGT ATA CAT ATA GGA


                     20                     25                    30
Pro Gly Arg Ala Phe Tyr Thr Thr Gly Thr Ile Ile Gly Asp Ile
CCA GGG AGA GCA TTT TAT ACA ACA GGA ACA ATA ATA GGA GAT ATA


                     35
Arg Gln Ala His Cys
AGA CAA GCA CAT TGT


(2)      INFORMATION FOR SEQ ID NO: EE378-1
         (i)      SEQUENCE CHARACTERISTICS:
                  (A)      LENGTH:  105
                  (B)      TYPE:  Nucleic Acid
                  (C)      STRANDEDNESS:  Single
                  (D)      TOPOLOGY:  Linear
         (ii)     KIND: cDNA to genomic RNA

```
        (ii)    KIND (if peptide or protein):
                (A)     SEQUENCE ASSEMBLY METHOD:  Overlap
                (B)     FRAGMENT TYPE:  Internal Fragment
                (C)     HYPOTHETICAL:  _____
        (iii)   ORIGINAL SOURCE: HIV
                (E)     INDIVIDUAL ISOLATE:  _____
        (iv)    IMMEDIATE SOURCE:
                (C)     CLONE:  _____
        (v)     POSITION IN GENOME: Within Env Gene
        (vi)    PROPERTIES OF SEQUENCE:  Expresses conserved antigenic
                determinant
        (viii)  SEQUENCE DESCRIPTION:
```

SEQ ID NO:   EE378-1


```
 1                   5                   10                  15
Cys Thr Arg Pro Asn Asn Asn Thr Arg Lys Ser Ile His Ile Gly
TGT ACA AGA CCC AAC AAC AAT ACA AGA AAA AGT ATA CAT ATA GGA


                    20                  25                  30
Pro Gly Arg Ala Phe Tyr Thr Thr Gly Glu Ile Ile Gly Asp Ile
CCA GGG AGA GCA TTT TAT ACA ACA GGA GAA ATA ATA GGA GAT ATA


                    35
Arg Gln Ala His Cys
AGA CAA GCA CAT TGT
```


```
(2)     INFORMATION FOR SEQ ID NO: EE378-2
        (i)     SEQUENCE CHARACTERISTICS:
                (A)     LENGTH:  105
                (B)     TYPE:  Nucleic Acid
                (C)     STRANDEDNESS:  Single
                (D)     TOPOLOGY:  Linear
        (ii)    KIND: cDNA to genomic RNA
        (ii)    KIND (if peptide or protein):
                (A)     SEQUENCE ASSEMBLY METHOD:  Overlap
                (B)     FRAGMENT TYPE:  Internal Fragment
                (C)     HYPOTHETICAL:  _____
        (iii)   ORIGINAL SOURCE: HIV
                (E)     INDIVIDUAL ISOLATE:  _____
        (iv)    IMMEDIATE SOURCE:
                (C)     CLONE:  _____
        (v)     POSITION IN GENOME: Within Env Gene
        (vi)    PROPERTIES OF SEQUENCE:  Expresses conserved antigenic
                determinant
        (viii)  SEQUENCE DESCRIPTION:
```

SEQ ID NO:  EE378-2

```
      1               5                   10                  15
Cys Thr Arg Pro Asn Asn Asn Thr Arg Lys Ser Ile His Ile Gly
TGT ACA AGA CCC AAC AAC AAT ACA AGA AAA AGT ATA CAT ATA GGA


                      20                  25                  30
Pro Gly Arg Ala Phe Tyr Thr Thr Gly Glu Ile Ile Gly Asp Ile
CCA GGG AGA GCA TTT TAT ACA ACA GGA GAA ATA ATA GGA GAT ATA


                      35
Arg Gln Ala His Cys
AGA CAA GCA CAT TGT
```

(2)      INFORMATION FOR SEQ ID NO: EE378-3
         (i)      SEQUENCE CHARACTERISTICS:
                  (A)     LENGTH:  105
                  (B)     TYPE:  Nucleic Acid
                  (C)     STRANDEDNESS:  Single
                  (D)     TOPOLOGY:  Linear
         (ii)     KIND: cDNA to genomic RNA
         (ii)     KIND (if peptide or protein):
                  (A)     SEQUENCE ASSEMBLY METHOD:  Overlap
                  (B)     FRAGMENT TYPE:  Internal Fragment
                  (C)     HYPOTHETICAL:  _____
         (iii)    ORIGINAL SOURCE: HIV
                  (E)     INDIVIDUAL ISOLATE:  _____
         (iv)     IMMEDIATE SOURCE:
                  (C)     CLONE:  _____
         (v)      POSITION IN GENOME: Within Env Gene
         (vi)     PROPERTIES OF SEQUENCE:  Expresses conserved antigenic
                  determinant
         (viii)   SEQUENCE DESCRIPTION:


SEQ ID NO:  EE378-3

```
      1               5                   10                  15
Cys Thr Arg Pro Asn Asn Asn Thr Arg Lys Ser Ile Pro Ile Gly
TGT ACA AGA CCC AAC AAC AAT ACA AGA AAA AGT ATA CCT ATA GGA
```

117

```
                     20                      25                      30
Pro Gly Arg Ala Phe Tyr Thr Thr Gly Glu Ile Ile Gly Asp Ile
CCA GGG AGA GCA TTT TAT ACA ACA GGA GAA ATA ATA GGA GAT ATA


                     35
Arg Gln Ala His Cys
AGA CAA GCA CAT TGT
```

(2)      INFORMATION FOR SEQ ID NO: EE380-1
     (i)      SEQUENCE CHARACTERISTICS:
        (A)      LENGTH:  105
        (B)      TYPE:  Nucleic Acid
        (C)      STRANDEDNESS:  Single
        (D)      TOPOLOGY:  Linear
     (ii)     KIND: cDNA to genomic RNA
     (ii)     KIND (if·peptide or protein):
        (A)      SEQUENCE ASSEMBLY METHOD:  Overlap
        (B)      FRAGMENT TYPE:  Internal Fragment
        (C)      HYPOTHETICAL:  _____
     (iii)    ORIGINAL SOURCE: HIV
        (E)      INDIVIDUAL ISOLATE:  _____
     (iv)     IMMEDIATE SOURCE:
        (C)      CLONE:  _____
     (v)      POSITION IN GENOME: Within Env Gene
     (vi)     PROPERTIES OF SEQUENCE:  Expresses conserved antigenic
        determinant
     (viii)   SEQUENCE DESCRIPTION:

SEQ ID NO:  EE380-1

```
 1                   5                      10                      15
Cys Thr Arg Pro Ser Asn Asn Thr Arg Lys Ser Ile His Ile Gly
TGT ACA AGA CCC AGC AAC AAT ACA AGA AAA AGT ATA CAT ATA GGA


                     20                      25                      30
Pro Gly Arg Ala Phe Tyr Thr Thr Gly Glu Ile Ile Gly Asp Ile
CCA GGG AGA GCA TTT TAT ACA ACA GGA GAA ATA ATA GGA GAT ATA


                     35
Arg Gln Ala His Cys
AGA CAA GCA CAT TGT
```

EP 0 471 407 A2

(2)      INFORMATION FOR SEQ ID NO: EE380-2
        (i)       SEQUENCE CHARACTERISTICS:
                  (A)      LENGTH:  105
                  (B)      TYPE:  Nucleic Acid
                  (C)      STRANDEDNESS:  Single
                  (D)      TOPOLOGY:  Linear
        (ii)     KIND: cDNA to genomic RNA
        (ii)     KIND (if peptide or protein):
                  (A)      SEQUENCE ASSEMBLY METHOD:  Overlap
                  (B)      FRAGMENT TYPE:  Internal Fragment
                  (C)      HYPOTHETICAL:  _____
        (iii)    ORIGINAL SOURCE: HIV
                  (E)      INDIVIDUAL ISOLATE:  _____
        (iv)     IMMEDIATE SOURCE:
                  (C)      CLONE:  _____
        (v)      POSITION IN GENOME: Within Env Gene
        (vi)     PROPERTIES OF SEQUENCE:  Expresses conserved antigenic
                  determinant
        (viii)   SEQUENCE DESCRIPTION:


SEQ ID NO:  EE380-2


```
1                5                       10                      15
Cys Thr Arg Pro Ser Asn Asn Thr Arg Lys Ser Ile His Ile Gly
TGT ACA AGA CCC AGC AAC AAT ACA AGA AAA AGT ATA CAT ATA GGA


                 20                      25                      30
Pro Gly Arg Ala Phe Tyr Thr Thr Gly Glu Ile Ile Gly Asp Ile
CCA GGG AGA GCA TTT TAT ACA ACA GGA GAA ATA ATA GGA GAT ATA


                 35
Arg Gln Ala His Cys
AGA CAA GCA CAT TGT
```


(2)      INFORMATION FOR SEQ ID NO: EE397-1
        (i)       SEQUENCE CHARACTERISTICS:
                  (A)      LENGTH:  105
                  (B)      TYPE:  Nucleic Acid
                  (C)      STRANDEDNESS:  Single
                  (D)      TOPOLOGY:  Linear
        (ii)     KIND: cDNA to genomic RNA

119

```
         (ii)    KIND (if peptide or protein):
                 (A)     SEQUENCE ASSEMBLY METHOD:  Overlap
                 (B)     FRAGMENT TYPE:  Internal Fragment
                 (C)     HYPOTHETICAL:  _____
         (iii)   ORIGINAL SOURCE: HIV
                 (E)     INDIVIDUAL ISOLATE:  _____
         (iv)    IMMEDIATE SOURCE:
                 (C)     CLONE:  _____
         (v)     POSITION IN GENOME: Within Env Gene
         (vi)    PROPERTIES OF SEQUENCE:  Expresses conserved antigenic
                 determinant
         (viii)  SEQUENCE DESCRIPTION:
```

SEQ ID NO:  EE397-1

```
  1              5                   10                  15
Cys Thr Arg Pro Asn Asn Asn Thr Arg Arg Ser Ile His Ile Gly
TGT ACA AGA CCC AAC AAC AAT ACA AGA AGA AGT ATA CAC ATA GGA


                20                  25                  30
Pro Gly Arg Ala Phe Tyr Ala Thr Gly Glu Ile Ile Gly Asn Ile
CCA GGG AGA GCA TTT TAT GCA ACA GGA GAA ATA ATA GGA AAT ATA


                35
Arg Gln Ala Tyr Cys
AGA CAA GCA TAT TGC
```

```
(2)      INFORMATION FOR SEQ ID NO: EE399-1
         (i)     SEQUENCE CHARACTERISTICS:
                 (A)     LENGTH:  105
                 (B)     TYPE:  Nucleic Acid
                 (C)     STRANDEDNESS:  Single
                 (D)     TOPOLOGY:  Linear
         (ii)    KIND: cDNA to genomic RNA
         (ii)    KIND (if peptide or protein):
                 (A)     SEQUENCE ASSEMBLY METHOD:  Overlap
                 (B)     FRAGMENT TYPE:  Internal Fragment
                 (C)     HYPOTHETICAL:  _____
         (iii)   ORIGINAL SOURCE: HIV
                 (E)     INDIVIDUAL ISOLATE:  _____
         (iv)    IMMEDIATE SOURCE:
                 (C)     CLONE:  _____
         (v)     POSITION IN GENOME: Within Env Gene
         (vi)    PROPERTIES OF SEQUENCE:  Expresses conserved antigenic
                 determinant
         (viii)  SEQUENCE DESCRIPTION:
```

SEQ ID NO:  EE399-1

```
        1               5                       10                      15
Cys Thr Arg Pro Asn Asn Asn Thr Arg Lys Gly Ile His Ile Gly
TGT ACA AGA CCC AAC AAC AAT ACA AGA AAA GGT ATA CAT ATA GGA


                        20                      25                      30
Pro Gly Arg Ala Phe Tyr Thr Thr Gly Glu Ile Ile Gly Asp Ile
CCA GGG AGA GCA TTT TAT ACA ACA GGA GAA ATA ATA GGA GAT ATA


                        35
Arg Gln Ala His Cys
AGA CAA GCA CAT TGT
```

(2)     INFORMATION FOR SEQ ID NO: EE399-2
        (i)     SEQUENCE CHARACTERISTICS:
                (A)     LENGTH:  105
                (B)     TYPE:  Nucleic Acid
                (C)     STRANDEDNESS:  Single
                (D)     TOPOLOGY:  Linear
        (ii)    KIND: cDNA to genomic RNA
        (ii)    KIND (if peptide or protein):
                (A)     SEQUENCE ASSEMBLY METHOD:  Overlap
                (B)     FRAGMENT TYPE:  Internal Fragment
                (C)     HYPOTHETICAL:  _____
        (iii)   ORIGINAL SOURCE: HIV
                (E)     INDIVIDUAL ISOLATE:  _____
        (iv)    IMMEDIATE SOURCE:
                (C)     CLONE:  _____
        (v)     POSITION IN GENOME: Within Env Gene
        (vi)    PROPERTIES OF SEQUENCE:  Expresses conserved antigenic
                determinant
        (viii)  SEQUENCE DESCRIPTION:


SEQ ID NO:  EE399-2

```
        1               5                       10                      15
Cys Thr Arg Pro Asn Asn Asn Thr Arg Lys Gly Ile His Ile Gly
TGT ACA AGA CCC AAC AAC AAT ACA AGA AAA GGT ATA CAT ATA GGA
```

```
                        20                      25                      30
Pro Gly Arg Ala Phe Tyr Thr Thr Gly Glu Ile Ile Gly Asp Ile
CCA GGG AGA GCA TTT TAT ACA ACA GGA GAA ATA ATA GGA GAT ATA


                        35
Arg Gln Ala His Cys
AGA CAA GCA CAT TGT
```

(2)      INFORMATION FOR SEQ ID NO: EE399-3
         (i)      SEQUENCE CHARACTERISTICS:
                  (A)      LENGTH:  105
                  (B)      TYPE:  Nucleic Acid
                  (C)      STRANDEDNESS:  Single
                  (D)      TOPOLOGY:  Linear
         (ii)     KIND: cDNA to genomic RNA
         (ii)     KIND (if peptide or protein):
                  (A)      SEQUENCE ASSEMBLY METHOD:  Overlap
                  (B)      FRAGMENT TYPE:  Internal Fragment
                  (C)      HYPOTHETICAL:  _____
         (iii)    ORIGINAL SOURCE: HIV
                  (E)      INDIVIDUAL ISOLATE:  _____
         (iv)     IMMEDIATE SOURCE:
                  (C)      CLONE:  _____
         (v)      POSITION IN GENOME: Within Env Gene
         (vi)     PROPERTIES OF SEQUENCE:  Expresses conserved antigenic
                  determinant
         (viii)   SEQUENCE DESCRIPTION:


SEQ ID NO:  EE399-3


```
1                       5                      10                      15
Cys Thr Arg Pro Asn Asn Asn Thr Arg Arg Ser Ile His Ile Gly
TGT ACA AGA CCC AAC AAC AAT ACA AGA AGA AGT ATA CAT ATA GGA


                        20                      25                      30
Pro Gly Arg Ala Phe Tyr Thr Thr Gly Glu Ile Ile Gly Asn Ile
CCA GGG AGA GCA TTT TAT ACA ACA GGA GAA ATA ATA GGA AAT ATA


                        35
Arg Gln Ala His Cys
AGA CAA GCA CAT TGT
```

(2)     INFORMATION FOR SEQ ID NO: EE405-1
        (i)     SEQUENCE CHARACTERISTICS:
                (A)     LENGTH:  105
                (B)     TYPE:  Nucleic Acid
                (C)     STRANDEDNESS:  Single
                (D)     TOPOLOGY:  Linear
        (ii)    KIND: cDNA to genomic RNA
        (ii)    KIND (if peptide or protein):
                (A)     SEQUENCE ASSEMBLY METHOD:  Overlap
                (B)     FRAGMENT TYPE:  Internal Fragment
                (C)     HYPOTHETICAL:  _____
        (iii)   ORIGINAL SOURCE: HIV
                (E)     INDIVIDUAL ISOLATE:  _____
        (iv)    IMMEDIATE SOURCE:
                (C)     CLONE:  _____
        (v)     POSITION IN GENOME: Within Env Gene
        (vi)    PROPERTIES OF SEQUENCE:  Expresses conserved antigenic
                determinant
        (viii)  SEQUENCE DESCRIPTION:


SEQ ID NO:  EE405-1


```
 1               5                      10                     15
Cys Thr Arg Pro Asn Asn Asn Thr Arg Lys Arg Ile Thr Thr Gly
TGT ACA AGA CCC AAC AAC AAT ACA AGA AAA AGA ATA ACT ACG GGA


                 20                     25                     30
Pro Gly Arg Val Tyr Tyr Thr Thr Gly Glu Ile Ile Gly Asp Ile
CCG GGG AGA GTA TAT TAT ACA ACA GGA GAA ATA ATA GGA GAT ATA


                 35
Arg Lys Ala His Cys
AGA AAA GCA CAT TGT
```


(2)     INFORMATION FOR SEQ ID NO: EE405-2
        (i)     SEQUENCE CHARACTERISTICS:
                (A)     LENGTH:  105
                (B)     TYPE:  Nucleic Acid
                (C)     STRANDEDNESS:  Single
                (D)     TOPOLOGY:  Linear
        (ii)    KIND: cDNA to genomic RNA

```
(ii)     KIND (if peptide or protein):
         (A)      SEQUENCE ASSEMBLY METHOD:  Overlap
         (B)      FRAGMENT TYPE:  Internal Fragment
         (C)      HYPOTHETICAL:  _____
(iii)    ORIGINAL SOURCE: HIV
         (E)      INDIVIDUAL ISOLATE:  _____
(iv)     IMMEDIATE SOURCE:
         (C)      CLONE:  _____
(v)      POSITION IN GENOME: Within Env Gene
(vi)     PROPERTIES OF SEQUENCE:  Expresses conserved antigenic
         determinant
(viii)   SEQUENCE DESCRIPTION:
```

SEQ ID NO:  EE405-2

```
  1                 5                     10                    15
Cys Thr Arg Pro Asn Asn Asn Thr Arg Lys Arg Ile Thr Thr Gly
TGT ACA AGA CCC AAC AAC AAT ACA AGA AAA AGA ATA ACT ACG GGA


                    20                    25                    30
Pro Gly Arg Val Tyr Tyr Thr Thr Gly Glu Ile Ile Gly Asp Ile
CCG GGG AGA GTA TAT TAT ACA ACA GGA GAA ATA ATA GGA GAT ATA


                    35
Arg Lys Ala His Cys
AGA AAA GCA CAT TGT
```

```
(2)      INFORMATION FOR SEQ ID NO: EE405-3
         (i)      SEQUENCE CHARACTERISTICS:
                  (A)      LENGTH:  105
                  (B)      TYPE:  Nucleic Acid
                  (C)      STRANDEDNESS:  Single
                  (D)      TOPOLOGY:  Linear
         (ii)     KIND: cDNA to genomic RNA
         (ii)     KIND (if peptide or protein):
                  (A)      SEQUENCE ASSEMBLY METHOD:  Overlap
                  (B)      FRAGMENT TYPE:  Internal Fragment
                  (C)      HYPOTHETICAL:  _____
         (iii)    ORIGINAL SOURCE: HIV
                  (E)      INDIVIDUAL ISOLATE:  _____
         (iv)     IMMEDIATE SOURCE:
                  (C)      CLONE:  _____
         (v)      POSITION IN GENOME: Within Env Gene
         (vi)     PROPERTIES OF SEQUENCE:  Expresses conserved antigenic
                  determinant
         (viii)   SEQUENCE DESCRIPTION:
```

SEQ ID NO:   EE405-3

```
        1               5                    10                   15
        Cys Thr Arg Pro Asn Asn Asn Thr Arg Lys Lys Ile Thr Thr Gly
        TGT ACA AGA CCC AAC AAC AAT ACA AGA AAA AAA ATA ACT ACG GGA


                        20                   25                   30
        Pro Gly Arg Val Tyr Tyr Thr Thr Gly Glu Ile Ile Glu Asp Val
        CCG GGG AGA GTA TAT TAT ACA ACA GGA GAA ATA ATA GAA GAT GTA


                        35
        Arg Lys Ala His Cys
        AGA AAA GCA CAT TGT
```

(2)       INFORMATION FOR SEQ ID NO: EE505-1
          (i)      SEQUENCE CHARACTERISTICS:
                   (A)      LENGTH:  105
                   (B)      TYPE:  Nucleic Acid
                   (C)      STRANDEDNESS:  Single
                   (D)      TOPOLOGY:  Linear
          (ii)     KIND: cDNA to genomic RNA
          (ii)     KIND (if peptide or protein):
                   (A)      SEQUENCE ASSEMBLY METHOD:  Overlap
                   (B)      FRAGMENT TYPE:  Internal Fragment
                   (C)      HYPOTHETICAL:  _____
          (iii)    ORIGINAL SOURCE: HIV
                   (E)      INDIVIDUAL ISOLATE:  _____
          (iv)     IMMEDIATE SOURCE:
                   (C)      CLONE:  _____
          (v)      POSITION IN GENOME: Within Env Gene
          (vi)     PROPERTIES OF SEQUENCE:  Expresses conserved antigenic
                   determinant
          (viii)   SEQUENCE DESCRIPTION:


SEQ ID NO:   EE505-1

```
        1               5                    10                   15
        Cys Thr Arg Pro Asn Asn Asn Thr Arg Arg Ser Ile Asn Ile Gly
        TGT ACA AGG CCC AAC AAC AAT ACA AGA AGA AGT ATA AAT ATA GGA
```

```
                    20                 25                 30
Pro Gly Arg Ala Phe Tyr Ala Thr Gly Asp Ile Thr Gly Asp Ile
CCA GGG AGA GCA TTT TAT GCA ACA GGA GAT ATA ACA GGA GAT ATA


                    35
Arg Gln Ala His Cys
AGA CAA GCA CAT TGT
```

(2)      INFORMATION FOR SEQ ID NO: EE505-2
         (i)      SEQUENCE CHARACTERISTICS:
                  (A)      LENGTH:  105
                  (B)      TYPE:  Nucleic Acid
                  (C)      STRANDEDNESS:  Single
                  (D)      TOPOLOGY:  Linear
         (ii)     KIND: cDNA to genomic RNA
         (ii)     KIND (if peptide or protein):
                  (A)      SEQUENCE ASSEMBLY METHOD:  Overlap
                  (B)      FRAGMENT TYPE:  Internal Fragment
                  (C)      HYPOTHETICAL:  _____
         (iii)    ORIGINAL SOURCE: HIV
                  (E)      INDIVIDUAL ISOLATE:  _____
         (iv)     IMMEDIATE SOURCE:
                  (C)      CLONE:  _____
         (v)      POSITION IN GENOME: Within Env Gene
         (vi)     PROPERTIES OF SEQUENCE:  Expresses conserved antigenic
                  determinant
         (viii)   SEQUENCE DESCRIPTION:


SEQ ID NO:   EE505-2


```
 1                  5                 10                 15
Cys Thr Arg Pro Asn Asn Asn Thr Arg Arg Ser Ile Asn Ile Gly
TGT ACA AGG CCC AAC AAC AAT ACA AGA AGA AGT ATA AAT ATA GGA


                    20                 25                 30
Pro Gly Arg Ala Phe Tyr Ala Thr Gly Asp Ile Thr Gly Asp Ile
CCA GGG AGA GCA TTT TAT GCA ACA GGA GAT ATA ACA GGA GAT ATA


                    35
Arg Gln Ala His Cys
AGA CAA GCA CAT TGT
```

```
(2)       INFORMATION FOR SEQ ID NO: EE505-3
          (i)     SEQUENCE CHARACTERISTICS:
                  (A)     LENGTH:  105
                  (B)     TYPE:  Nucleic Acid
                  (C)     STRANDEDNESS:  Single
                  (D)     TOPOLOGY:  Linear
          (ii)   KIND: cDNA to genomic RNA
          (ii)   KIND (if peptide or protein):
                  (A)     SEQUENCE ASSEMBLY METHOD:  Overlap
                  (B)     FRAGMENT TYPE:  Internal Fragment
                  (C)     HYPOTHETICAL:  _____
          (iii)  ORIGINAL SOURCE: HIV
                  (E)     INDIVIDUAL ISOLATE:  _____
          (iv)   IMMEDIATE SOURCE:
                  (C)     CLONE:  _____
          (v)    POSITION IN GENOME: Within Env Gene
          (vi)   PROPERTIES OF SEQUENCE:  Expresses conserved antigenic
                  determinant
          (viii) SEQUENCE DESCRIPTION:
```

SEQ ID NO:  EE505-3

```
  1                 5                      10                      15
Cys Thr Arg Pro Asn Asn Asn Thr Arg Arg Ser Ile Asn Ile Gly
TGT ACA AGG CCC AAC AAC AAT ACA AGA AGA AGT ATA AAT ATA GGA


                    20                      25                      30
Pro Gly Arg Ala Phe Tyr Ala Thr Gly Asp Ile Thr Gly Asp Ile
CCA GGG AGA GCA TTT TAT GCA ACA GGA GAT ATA ACA GGA GAT ATA


                    35
Arg Gln Ala His Cys
AGA CAA GCA CAT TGT
```

```
(2)       INFORMATION FOR SEQ ID NO: EE507-1
          (i)     SEQUENCE CHARACTERISTICS:
                  (A)     LENGTH:  105
                  (B)     TYPE:  Nucleic Acid
                  (C)     STRANDEDNESS:  Single
                  (D)     TOPOLOGY:  Linear
          (ii)   KIND: cDNA to genomic RNA
```

127

(ii)    KIND (if peptide or protein):
        (A)    SEQUENCE ASSEMBLY METHOD:  Overlap
        (B)    FRAGMENT TYPE:  Internal Fragment
        (C)    HYPOTHETICAL:  _____
(iii)   ORIGINAL SOURCE: HIV
        (E)    INDIVIDUAL ISOLATE:  _____
(iv)    IMMEDIATE SOURCE:
        (C)    CLONE:  _____
(v)     POSITION IN GENOME: Within Env Gene
(vi)    PROPERTIES OF SEQUENCE:  Expresses conserved antigenic
        determinant
(viii)  SEQUENCE DESCRIPTION:


SEQ ID NO:  EE507-1


```
1                    5                    10                   15
Cys Thr Arg Pro Asn Asn Asn Thr Arg Lys Ser Ile Asn Ile Gly
TGT ACA AGA CCC AAC AAC AAT ACA AGA AAA AGT ATA AAT ATA GGA


                     20                   25                   30
Pro Gly Arg Ala Phe Tyr Ala Thr Gly Glu Ile Ile Gly Asp Ile
CCA GGG AGA GCA TTT TAT GCA ACA GGA GAA ATA ATA GGA GAT ATA


                     35
Arg Gln Ala His Cys
AGA CAA GCA CAT TGT
```


(2)     INFORMATION FOR SEQ ID NO: EE509-1
        (i)     SEQUENCE CHARACTERISTICS:
                (A)    LENGTH:  105
                (B)    TYPE:  Nucleic Acid
                (C)    STRANDEDNESS:  Single
                (D)    TOPOLOGY:  Linear
        (ii)    KIND: cDNA to genomic RNA
        (ii)    KIND (if peptide or protein):
                (A)    SEQUENCE ASSEMBLY METHOD:  Overlap
                (B)    FRAGMENT TYPE:  Internal Fragment
                (C)    HYPOTHETICAL:  _____
        (iii)   ORIGINAL SOURCE: HIV
                (E)    INDIVIDUAL ISOLATE:  _____
        (iv)    IMMEDIATE SOURCE:
                (C)    CLONE:  _____
        (v)     POSITION IN GENOME: Within Env Gene
        (vi)    PROPERTIES OF SEQUENCE:  Expresses conserved antigenic
                determinant
        (viii)  SEQUENCE DESCRIPTION:

SEQ ID NO:  EE509-1

```
        1                   5                      10                       15
Cys Thr Arg Pro Asn Asn Asn Thr Arg Lys Gly Ile His Ile Gly
TGT ACA AGA CCC AAC AAC AAT ACA AGG AAA GGT ATA CAT ATA GGA


                            20                      25                       30
Pro Gly Arg Ala Phe Tyr Ala Thr Gly Glu Ile Ile Gly Asp Ile
CCG GGG AGA GCA TTT TAT GCA ACA GGA GAA ATA ATA GGA GAT ATA


                            35
Arg Gln Ala His Cys
AGA CAA GCA CAT TGT
```

```
(2)        INFORMATION FOR SEQ ID NO: EE509-2
           (i)        SEQUENCE CHARACTERISTICS:
                      (A)       LENGTH:  105
                      (B)       TYPE:  Nucleic Acid
                      (C)       STRANDEDNESS:  Single
                      (D)       TOPOLOGY:  Linear
           (ii)       KIND: cDNA to genomic RNA
           (ii)       KIND (if peptide or protein):
                      (A)       SEQUENCE ASSEMBLY METHOD:  Overlap
                      (B)       FRAGMENT TYPE:  Internal Fragment
                      (C)       HYPOTHETICAL:  _____
           (iii)      ORIGINAL SOURCE: HIV
                      (E)       INDIVIDUAL ISOLATE:  _____
           (iv)       IMMEDIATE SOURCE:
                      (C)       CLONE:  _____
           (v)        POSITION IN GENOME: Within Env Gene
           (vi)       PROPERTIES OF SEQUENCE:  Expresses conserved antigenic
                      determinant
           (viii)     SEQUENCE DESCRIPTION:
```

SEQ ID NO:  EE509-2

```
        1                   5                      10                       15
Cys Thr Arg Pro Asn Asn Asn Thr Arg Lys Gly Ile His Ile Gly
TGT ACA AGA CCC AAC AAC AAT ACA AGG AAA GGT ATA CAT ATA GGA
```

129

```
                        20                      25                      30
Pro Gly Arg Ala Phe Tyr Ala Thr Gly Glu Ile Ile Gly Asp Ile
CCG GGG AGA GCA TTT TAT GCA ACA GGA GAA ATA ATA GGA GAT ATA


                        35
Arg Gln Ala His Cys
AGA CAA GCA CAT TGT
```

(2)        INFORMATION FOR SEQ ID NO: EE510-1
           (i)      SEQUENCE CHARACTERISTICS:
                    (A)      LENGTH:  102
                    (B)      TYPE:  Nucleic Acid
                    (C)      STRANDEDNESS:  Single
                    (D)      TOPOLOGY:  Linear
           (ii)     KIND: cDNA to genomic RNA
           (ii)     KIND (if peptide or protein):
                    (A)      SEQUENCE ASSEMBLY METHOD:  Overlap
                    (B)      FRAGMENT TYPE:  Internal Fragment
                    (C)      HYPOTHETICAL:  _____
           (iii)    ORIGINAL SOURCE: HIV
                    (E)      INDIVIDUAL ISOLATE:  _____
           (iv)     IMMEDIATE SOURCE:
                    (C)      CLONE:  _____
           (v)      POSITION IN GENOME: Within Env Gene
           (vi)     PROPERTIES OF SEQUENCE:  Expresses conserved antigenic
                    determinant
           (viii)   SEQUENCE DESCRIPTION:


SEQ ID NO:   EE510-1


```
1                       5                       10                      15
Cys Thr Arg Pro Ser Asn Asn Thr Arg Arg Gly Ile His Ile Gly
TGT ACA AGA CCC AGT AAC AAT ACA AGA AGA GGT ATA CAT ATA GGT


                        20                      25                      30
Pro Gly Ala Phe Tyr Thr Thr Gly Asp Ile Ile Gly Asp Ile Arg
CCA GGA GCA TTT TAT ACA ACA GGA GAC ATA ATA GGA GAT ATA AGA


                        35
Gln Ala His Cys
CAA GCA CAT TGT
```

(2)      INFORMATION FOR SEQ ID NO: EE510-2
      (i)      SEQUENCE CHARACTERISTICS:
            (A)      LENGTH:  102
            (B)      TYPE:  Nucleic Acid
            (C)      STRANDEDNESS:  Single
            (D)      TOPOLOGY:  Linear
      (ii)    KIND: cDNA to genomic RNA
      (ii)    KIND (if peptide or protein):
            (A)      SEQUENCE ASSEMBLY METHOD:  Overlap
            (B)      FRAGMENT TYPE:  Internal Fragment
            (C)      HYPOTHETICAL:  _____
      (iii)   ORIGINAL SOURCE: HIV
            (E)      INDIVIDUAL ISOLATE:  _____
      (iv)    IMMEDIATE SOURCE:
            (C)      CLONE:  _____
      (v)     POSITION IN GENOME: Within Env Gene
      (vi)    PROPERTIES OF SEQUENCE:  Expresses conserved antigenic
            determinant
      (viii)  SEQUENCE DESCRIPTION:


SEQ ID NO:  EE510-2


```
1                   5                   10                  15
Cys Thr Arg Pro Ser Asn Asn Thr Arg Arg Gly Ile His Ile Gly
TGT ACA AGA CCC AGT AAC AAT ACA AGA AGA GGT ATA CAT ATA GGT


                    20                  25                  30
Pro Gly Ala Phe Tyr Thr Thr Gly Asp Ile Ile Gly Asp Ile Arg
CCA GGA GCA TTT TAT ACA ACA GGA GAC ATA ATA GGA GAT ATA AGA


                    35
Gln Ala His Cys
CAA GCA CAT TGT
```


(2)      INFORMATION FOR SEQ ID NO: EE510-3
      (i)      SEQUENCE CHARACTERISTICS:
            (A)      LENGTH:  102
            (B)      TYPE:  Nucleic Acid
            (C)      STRANDEDNESS:  Single
            (D)      TOPOLOGY:  Linear
      (ii)    KIND: cDNA to genomic RNA

```
(ii)    KIND (if peptide or protein):
        (A)     SEQUENCE ASSEMBLY METHOD:  Overlap
        (B)     FRAGMENT TYPE:  Internal Fragment
        (C)     HYPOTHETICAL:   _____
(iii)   ORIGINAL SOURCE: HIV
        (E)     INDIVIDUAL ISOLATE:  _____
(iv)    IMMEDIATE SOURCE:
        (C)     CLONE:  _____
(v)     POSITION IN GENOME: Within Env Gene
(vi)    PROPERTIES OF SEQUENCE:  Expresses conserved antigenic
        determinant
(viii)  SEQUENCE DESCRIPTION:
```

SEQ ID NO:   EE510-3

```
1                 5                      10                     15
Cys Thr Arg Leu Ser Asn Asn Thr Arg Arg Gly Ile His Ile Gly
TGT ACA AGA CTC AGC AAC AAT ACA AGA AGA GGT ATA CAT ATA GGT


                  20                     25                     30
Pro Gly Ala Phe Tyr Thr Thr Gly Asp Ile Ile Gly Asp Ile Arg
CCA GGA GCA TTT TAT ACA ACA GGA GAT ATA ATA GGA GAT ATA AGA


                  35
Gln Ala His Cys
CAG GCA CAT TGT
```

```
(2)       INFORMATION FOR SEQ ID NO: EE520-1
          (i)      SEQUENCE CHARACTERISTICS:
                   (A)      LENGTH:  105
                   (B)      TYPE:  Nucleic Acid
                   (C)      STRANDEDNESS:  Single
                   (D)      TOPOLOGY:  Linear
          (ii)     KIND: cDNA to genomic RNA
          (ii)     KIND (if peptide or protein):
                   (A)      SEQUENCE ASSEMBLY METHOD:  Overlap
                   (B)      FRAGMENT TYPE:  Internal Fragment
                   (C)      HYPOTHETICAL:  _____
          (iii)    ORIGINAL SOURCE: HIV
                   (E)      INDIVIDUAL ISOLATE:  _____
          (iv)     IMMEDIATE SOURCE:
                   (C)      CLONE:  _____
          (v)      POSITION IN GENOME: Within Env Gene
          (vi)     PROPERTIES OF SEQUENCE:  Expresses conserved
                                            antigenic determinant
          (viii)   SEQUENCE DESCRIPTION:


SEQ ID NO:  EE520-1


    1                 5                     10                    15
Cys Thr Arg Pro Asn Asn Asn Thr Arg Lys Ser Ile His Ile Gly
TGT ACA AGA CCC AAC AAC AAT ACA AGA AAA AGT ATA CAT ATA GGA


                      20                    25                    30
Pro Gly Arg Ala Phe Tyr Ala Thr Gly Glu Ile Ile Gly Asp Ile
CCA GGG AGA GCA TTT TAT GCA ACA GGA GAA ATA ATA GGA GAT ATA


                      35
Arg Gln Ala His Cys
AGA CAA GCA CAT TGT


(2)       INFORMATION FOR SEQ ID NO: EE520-2
          (i)      SEQUENCE CHARACTERISTICS:
                   (A)      LENGTH:  105
                   (B)      TYPE:  Nucleic Acid
                   (C)      STRANDEDNESS:  Single
                   (D)      TOPOLOGY:  Linear
          (ii)     KIND: cDNA to genomic RNA
          (ii)     KIND (if peptide or protein):
                   (A)      SEQUENCE ASSEMBLY METHOD:  Overlap
                   (B)      FRAGMENT TYPE:  Internal Fragment
                   (C)      HYPOTHETICAL:  _____
```

```
(iii)   ORIGINAL SOURCE: HIV
        (E)      INDIVIDUAL ISOLATE:   _____
(iv)    IMMEDIATE SOURCE:
        (C)      CLONE:   _____
(v)     POSITION IN GENOME: Within Env Gene          .
(vi)    PROPERTIES OF SEQUENCE:  Expresses conserved
                                 antigenic determinant
(viii)  SEQUENCE DESCRIPTION:
```

SEQ ID NO:   EE520-2

```
1                   5                    10                   15
Cys Thr Arg Pro Asn Asn Asn Thr Arg Lys Ser Ile His Ile Gly
TGT ACA AGA CCC AAC AAC AAT ACA AGA AAA AGT ATA CAT ATA GGA


                    20                   25                   30
Pro Gly Arg Ala Phe Tyr Ala Thr Gly Glu Ile Ile Gly Asp Ile
CCA GGG AGA GCA TTT TAT GCA ACA GGA GAA ATA ATA GGA GAT ATA


                    35
Arg Gln Ala His Cys
AGA CAA GCA CAT TGT
```

```
(2)     INFORMATION FOR SEQ ID NO: EE520-3
        (i)      SEQUENCE CHARACTERISTICS:
                 (A)      LENGTH:  105
                 (B)      TYPE:  Nucleic Acid
                 (C)      STRANDEDNESS:  Single
                 (D)      TOPOLOGY:  Linear
        (ii)     KIND: cDNA to genomic RNA
        (ii)     KIND (if peptide or protein):
                 (A)      SEQUENCE ASSEMBLY METHOD:  Overlap
                 (B)      FRAGMENT TYPE:  Internal Fragment
                 (C)      HYPOTHETICAL:   _____
        (iii)    ORIGINAL SOURCE: HIV
                 (E)      INDIVIDUAL ISOLATE:   _____
        (iv)     IMMEDIATE SOURCE:
                 (C)      CLONE:   _____
        (v)      POSITION IN GENOME: Within Env Gene
        (vi)     PROPERTIES OF SEQUENCE:  Expresses conserved
                                          antigenic determinant
        (viii)   SEQUENCE DESCRIPTION:
```

SEQ ID NO:  EE520-3

```
1                5                    10                   15
Cys Thr Arg Pro Asn Asn Asn Thr Arg Lys Ser Ile His Ile Gly
TGT ACA AGA CCC AAC AAC AAT ACA AGA AAA AGT ATA CAT ATA GGA


                 20                   25                   30
Pro Gly Arg Ala Phe Tyr Ala Thr Gly Glu Ile Ile Gly Asp Ile
CCA GGG AGA GCA TTT TAT GCA ACA GGA GAA ATA ATA GGA GAT ATA


                 35
Arg Gln Ala His Cys
AGA CAA GCA CAT TGC
```

(2)      INFORMATION FOR SEQ ID NO: EE528-1
         (i)     SEQUENCE CHARACTERISTICS:
                 (A)     LENGTH:  105
                 (B)     TYPE:  Nucleic Acid
                 (C)     STRANDEDNESS:  Single
                 (D)     TOPOLOGY:  Linear
         (ii)    KIND: cDNA to genomic RNA
         (ii)    KIND (if peptide or protein):
                 (A)     SEQUENCE ASSEMBLY METHOD:  Overlap
                 (B)     FRAGMENT TYPE:  Internal Fragment
                 (C)     HYPOTHETICAL:  _____
         (iii)   ORIGINAL SOURCE: HIV
                 (E)     INDIVIDUAL ISOLATE:  _____
         (iv)    IMMEDIATE SOURCE:
                 (C)     CLONE:  _____
         (v)     POSITION IN GENOME: Within Env Gene
         (vi)    PROPERTIES OF SEQUENCE:  Expresses conserved
                                          antigenic determinant
         (viii)  SEQUENCE DESCRIPTION:

SEQ ID NO:  EE528-1

```
1                5                    10                   15
Cys Thr Arg Pro Asn Asn Asn Thr Arg Arg Gly Ile His Ile Gly
TGT ACA AGA CCC AAC AAC AAT ACG AGG AGA GGT ATA CAT ATA GGA
```

```
                20                25                30
Pro Gly Arg Ala Val Tyr Ala Thr Asp Lys Ile Ile Gly Asn Ile
CCA GGG AGA GCA GTT TAT GCA ACA GAT AAA ATA ATA GGA AAT ATA


                35
Arg Gln Ala His Cys
AGA CAA GCA CAT TGT
```

```
(2)       INFORMATION FOR SEQ ID NO: EE528-2
          (i)      SEQUENCE CHARACTERISTICS:
                   (A)      LENGTH:  105
                   (B)      TYPE:  Nucleic Acid
                   (C)      STRANDEDNESS:  Single
                   (D)      TOPOLOGY:  Linear
          (ii)     KIND: cDNA to genomic RNA
          (ii)     KIND (if peptide or protein):
                   (A)      SEQUENCE ASSEMBLY METHOD:  Overlap
                   (B)      FRAGMENT TYPE:  Internal Fragment
                   (C)      HYPOTHETICAL:  _____
          (iii)    ORIGINAL SOURCE: HIV
                   (E)      INDIVIDUAL ISOLATE:  _____
          (iv)     IMMEDIATE SOURCE:
                   (C)      CLONE:  _____
          (v)      POSITION IN GENOME: Within Env Gene
          (vi)     PROPERTIES OF SEQUENCE:  Expresses conserved
                                            antigenic determinant
          (viii)   SEQUENCE DESCRIPTION:
```

SEQ ID NO:  EE528-2

```
1                 5                 10                15
Cys Thr Arg Pro Asn Asn Asn Thr Arg Arg Gly Ile His Ile Gly
TGT ACA AGA CCC AAC AAC AAT ACG AGG AGA GGT ATA CAT ATA GGA


                20                25                30
Pro Gly Arg Ala Val Tyr Ala Thr Asp Lys Ile Ile Gly Asn Ile
CCA GGG AGA GCA GTT TAT GCA ACA GAT AAA ATA ATA GGA AAT ATA


                35
Arg Gln Ala His Cys
AGA CAA GCA CAT TGT
```

136

(2)       INFORMATION FOR SEQ ID NO: EE528-3
     (i)      SEQUENCE CHARACTERISTICS:
          (A)      LENGTH:  105
          (B)      TYPE:  Nucleic Acid
          (C)      STRANDEDNESS:  Single
          (D)      TOPOLOGY:  Linear
     (ii)    KIND: cDNA to genomic RNA
     (ii)    KIND (if peptide or protein):
          (A)      SEQUENCE ASSEMBLY METHOD:  Overlap
          (B)      FRAGMENT TYPE:  Internal Fragment
          (C)      HYPOTHETICAL:  _____
     (iii)   ORIGINAL SOURCE: HIV
          (E)      INDIVIDUAL ISOLATE:  _____
     (iv)    IMMEDIATE SOURCE:
          (C)      CLONE:  _____
     (v)     POSITION IN GENOME: Within Env Gene
     (vi)    PROPERTIES OF SEQUENCE:  Expresses conserved
                                antigenic determinant
     (viii)  SEQUENCE DESCRIPTION:

SEQ ID NO:  EE528-3


```
1                 5                    10                   15
Cys Thr Arg Pro Asn Asn Asn Thr Arg Arg Gly Ile His Ile Gly
TGT ACA AGA CCC AAC AAC AAT ACG AGG AGA GGT ATA CAT ATA GGA


                  20                   25                   30
Pro Gly Arg Ala Val Tyr Ala Thr Asp Lys Ile Ile Gly Asn Ile
CCA GGG AGA GCA GTT TAT GCA ACA GAT AAA ATA ATA GGA AAT ATA


                  35
Arg Gln Ala His Cys
AGA CAA GCA CAT TGT
```


(2)       INFORMATION FOR SEQ ID NO: EE529-1
     (i)      SEQUENCE CHARACTERISTICS:
          (A)      LENGTH:  105
          (B)      TYPE:  Nucleic Acid
          (C)      STRANDEDNESS:  Single
          (D)      TOPOLOGY:  Linear
     (ii)    KIND: cDNA to genomic RNA
     (ii)    KIND (if peptide or protein):
          (A)      SEQUENCE ASSEMBLY METHOD:  Overlap
          (B)      FRAGMENT TYPE:  Internal Fragment
          (C)      HYPOTHETICAL:  _____

```
        (iii)   ORIGINAL SOURCE: HIV
                (E)      INDIVIDUAL ISOLATE:  _____
        (iv)    IMMEDIATE SOURCE:
                (C)      CLONE:  _____
        (v)     POSITION IN GENOME: Within Env Gene
        (vi)    PROPERTIES OF SEQUENCE:  Expresses conserved
                                          antigenic determinant
        (viii)  SEQUENCE DESCRIPTION:


SEQ ID NO:  EE529-1


    1              5               10              15
Cys Thr Arg Pro Ser Asn Asn Thr Arg Arg Ser Ile Pro Ile Gly
TGT ACA AGA CCC AGC AAC AAT ACA AGA AGA AGT ATA CCT ATA GGA


               20              25              30
Pro Gly Arg Ala Phe Tyr Thr Thr Gly Asp Ile Ile Gly Asp Ile
CCA GGG AGA GCA TTT TAT ACA ACA GGA GAT ATA ATA GGA GAT ATA


               35
Arg Gln Ala His Cys
AGA CAA GCA CAT TGT


    (2)      INFORMATION FOR SEQ ID NO: EE529-2
             (i)     SEQUENCE CHARACTERISTICS:
                     (A)      LENGTH:  105
                     (B)      TYPE:  Nucleic Acid
                     (C)      STRANDEDNESS:  Single
                     (D)      TOPOLOGY:  Linear
             (ii)    KIND: cDNA to genomic RNA
             (ii)    KIND (if peptide or protein):
                     (A)      SEQUENCE ASSEMBLY METHOD:  Overlap
                     (B)      FRAGMENT TYPE:  Internal Fragment
                     (C)      HYPOTHETICAL:  _____
             (iii)   ORIGINAL SOURCE: HIV
                     (E)      INDIVIDUAL ISOLATE:  _____
             (iv)    IMMEDIATE SOURCE:
                     (C)      CLONE:  _____
             (v)     POSITION IN GENOME: Within Env Gene
             (vi)    PROPERTIES OF SEQUENCE:  Expresses conserved
                                              antigenic determinant
             (viii)  SEQUENCE DESCRIPTION:


SEQ ID NO:  EE529-2
```

```
 1              5               10              15
Cys Thr Arg Pro Asn Asn Asn Thr Arg Lys Ser Ile Thr Ile Gly
TGT ACA AGA CCT AAC AAT AAT ACA AGA AAA AGT ATA ACT ATA GGA


               20              25              30
Pro Gly Arg Ala Phe Tyr Ala Thr Gly Asp Ile Ile Gly Asp Ile
CCG GGG AGA GCA TTT TAT GCA ACA GGA GAC ATA ATA GGA GAT ATA


               35
Arg Gln Ala His Cys
AGA CAA GCA CAT TGT
```

(2)       INFORMATION FOR SEQ ID NO: EE533-1
          (i)      SEQUENCE CHARACTERISTICS:
                   (A)      LENGTH:  105
                   (B)      TYPE:  Nucleic Acid
                   (C)      STRANDEDNESS:  Single
                   (D)      TOPOLOGY:  Linear
          (ii)     KIND: cDNA to genomic RNA
          (ii)     KIND (if peptide or protein):
                   (A)      SEQUENCE ASSEMBLY METHOD:  Overlap
                   (B)      FRAGMENT TYPE:  Internal Fragment
                   (C)      HYPOTHETICAL:  _____
          (iii)    ORIGINAL SOURCE: HIV
                   (E)      INDIVIDUAL ISOLATE:  _____
          (iv)     IMMEDIATE SOURCE:
                   (C)      CLONE:  _____
          (v)      POSITION IN GENOME: Within Env Gene
          (vi)     PROPERTIES OF SEQUENCE:  Expresses conserved
                                            antigenic determinant
          (viii)   SEQUENCE DESCRIPTION:


SEQ ID NO:  EE533-1


```
 1              5               10              15
Cys Thr Arg Pro Asn Asn Asn Thr Arg Lys Ser Ile Pro Ile Gly
TGT ACA AGA CCC AAC AAC AAT ACA AGA AAA AGT ATA CCT ATA GGA


               20              25              30
Pro Gly Arg Ala Phe Tyr Thr Thr Gly Asp Ile Ile Gly Asp Ile
CCA GGG AGA GCA TTT TAT ACA ACA GGA GAT ATA ATA GGA GAT ATA
```

```
                      35
Arg Gln Ala His Cys
AGA CAA GCA CAT TGT


(2)       INFORMATION FOR SEQ ID NO: EE533-2
          (i)     SEQUENCE CHARACTERISTICS:
                  (A)     LENGTH:  105
                  (B)     TYPE:  Nucleic Acid
                  (C)     STRANDEDNESS:  Single
                  (D)     TOPOLOGY:  Linear
          (ii)    KIND: cDNA to genomic RNA
          (ii)    KIND (if peptide or protein):
                  (A)     SEQUENCE ASSEMBLY METHOD:  Overlap
                  (B)     FRAGMENT TYPE:   Internal Fragment
                  (C)     HYPOTHETICAL:  _____
          (iii)   ORIGINAL SOURCE: HIV
                  (E)     INDIVIDUAL ISOLATE:  _____
          (iv)    IMMEDIATE SOURCE:
                  (C)     CLONE:  _____
          (v)     POSITION IN GENOME: Within Env Gene
          (vi)    PROPERTIES OF SEQUENCE:   Expresses conserved
                                            antigenic determinant
          (viii)  SEQUENCE DESCRIPTION:


SEQ ID NO:  EE533-2


1                 5                  10                 15
Cys Thr Arg Pro Asn Asn Asn Thr Arg Lys Ser Ile Pro Ile Gly
TGT ACA AGA CCC AAC AAC AAT ACA AGA AAA AGT ATA CCT ATA GGA


                  20                 25                 30
Pro Gly Arg Ala Phe Tyr Thr Thr Gly Asp Ile Ile Gly Asp Ile
CCA GGG AGA GCA TTT TAT ACA ACA GGA GAT ATA ATA GGA GAT ATA


                  35
Arg Gln Ala His Cys
AGA CAA GCA CAT TGT


(2)       INFORMATION FOR SEQ ID NO: EE533-3
          (i)     SEQUENCE CHARACTERISTICS:
                  (A)     LENGTH:  105
                  (B)     TYPE:  Nucleic Acid
                  (C)     STRANDEDNESS:  Single
```

```
              (D)     TOPOLOGY:  Linear
     (ii)    KIND: cDNA to genomic RNA
     (ii)    KIND (if peptide or protein):
              (A)     SEQUENCE ASSEMBLY METHOD:  Overlap
              (B)     FRAGMENT TYPE:  Internal Fragment
              (C)     HYPOTHETICAL:  _____
     (iii)   ORIGINAL SOURCE: HIV
              (E)     INDIVIDUAL ISOLATE:  _____
     (iv)    IMMEDIATE SOURCE:
              (C)     CLONE:  _____
     (v)     POSITION IN GENOME: Within Env Gene
     (vi)    PROPERTIES OF SEQUENCE:   Expresses conserved
                                       antigenic determinant
     (viii)  SEQUENCE DESCRIPTION:
```

SEQ ID NO:  EE533-3

```
1                5                          10                     15
Cys Thr Arg Pro Asn Asn Asn Thr Arg Lys Ser Ile Pro Ile Gly
TGT ACA AGA CCC AAC AAC AAT ACA AGA AAA AGT ATA CCT ATA GGA


                 20                         25                     30
Pro Gly Arg Ala Phe Tyr Thr Thr Gly Asp Ile Ile Gly Asp Ile
CCA GGG AGA GCA TTT TAT ACA ACA GGA GAT ATA ATA GGA GAT ATA


                 35
Arg Gln Ala His Cys
AGA CAA GCA CAT TGT
```

```
     (2)     INFORMATION FOR SEQ ID NO: EE535-1
             (i)     SEQUENCE CHARACTERISTICS:
                     (A)     LENGTH:  105
                     (B)     TYPE:  Nucleic Acid
                     (C)     STRANDEDNESS:  Single
                     (D)     TOPOLOGY:  Linear
             (ii)    KIND: cDNA to genomic RNA
             (ii)    KIND (if peptide or protein):
                     (A)     SEQUENCE ASSEMBLY METHOD:  Overlap
                     (B)     FRAGMENT TYPE:  Internal Fragment
                     (C)     HYPOTHETICAL:  _____
             (iii)   ORIGINAL SOURCE: HIV
                     (E)     INDIVIDUAL ISOLATE:  _____
             (iv)    IMMEDIATE SOURCE:
                     (C)     CLONE:  _____
             (v)     POSITION IN GENOME: Within Env Gene
```

141

(vi)     PROPERTIES OF SEQUENCE:  Expresses conserved
                                  antigenic determinant
(viii) SEQUENCE DESCRIPTION:


SEQ ID NO:  EE535-1


```
1                 5                    10                    15
Cys Thr Arg Pro Asn Asn Asn Thr Arg Lys Ser Ile His Ile Gly
TGT ACA AGA CCC AAC AAC AAT ACA AGA AAA AGT ATA CAT ATA GGA


                  20                   25                    30
Pro Gly Arg Ala Phe Tyr Ala Thr Gly Glu Ile Ile Gly Asp Ile
CCA GGG AGA GCA TTT TAT GCA ACA GGA GAA ATA ATA GGA GAT ATA


                  35
Arg Gln Ala His Cys
AGA CAA GCA CAT TGT
```


(2)     INFORMATION FOR SEQ ID NO: EE535-2
        (i)     SEQUENCE CHARACTERISTICS:
                (A)     LENGTH:  105
                (B)     TYPE:  Nucleic Acid
                (C)     STRANDEDNESS:  Single
                (D)     TOPOLOGY:  Linear
        (ii)    KIND: cDNA to genomic RNA
        (ii)    KIND (if peptide or protein):
                (A)     SEQUENCE ASSEMBLY METHOD:  Overlap
                (B)     FRAGMENT TYPE:  Internal Fragment
                (C)     HYPOTHETICAL:  _____
        (iii)   ORIGINAL SOURCE: HIV
                (E)     INDIVIDUAL ISOLATE:  _____
        (iv)    IMMEDIATE SOURCE:
                (C)     CLONE:  _____
        (v)     POSITION IN GENOME: Within Env Gene
        (vi)    PROPERTIES OF SEQUENCE:  Expresses conserved
                                         antigenic determinant
        (viii)  SEQUENCE DESCRIPTION:


SEQ ID NO:  EE535-2


```
1                 5                    10                    15
Cys Thr Arg Pro Asn Asn Asn Thr Arg Lys Ser Ile His Ile Gly
TGT ACA AGA CCC AAC AAC AAT ACA AGA AAA AGT ATA CAT ATA GGA
```

142

```
                    20                      25                      30
Pro Gly Arg Ala Phe Tyr Ala Thr Gly Glu Ile Ile Gly Asp Ile
CCA GGG AGA GCA TTT TAT GCA ACA GGA GAA ATA ATA GGA GAT ATA


                    35
Arg Gln Ala His Cys
AGA CAA GCA CAT TGT
```

(2)        INFORMATION FOR SEQ ID NO: EE543-1
       (i)      SEQUENCE CHARACTERISTICS:
                (A)      LENGTH:  105
                (B)      TYPE:  Nucleic Acid
                (C)      STRANDEDNESS:  Single
                (D)      TOPOLOGY:  Linear
       (ii)     KIND: cDNA to genomic RNA
       (ii)     KIND (if peptide or protein):
                (A)      SEQUENCE ASSEMBLY METHOD:  Overlap
                (B)      FRAGMENT TYPE:  Internal Fragment
                (C)      HYPOTHETICAL:  _____
       (iii)    ORIGINAL SOURCE: HIV
                (E)      INDIVIDUAL ISOLATE:  _____
       (iv)     IMMEDIATE SOURCE:
                (C)      CLONE:  _____
       (v)      POSITION IN GENOME: Within Env Gene
       (vi)     PROPERTIES OF SEQUENCE:  Expresses conserved
                                         antigenic determinant
       (viii)   SEQUENCE DESCRIPTION:


SEQ ID NO:   EE543-1


```
1                   5                       10                      15
Cys Thr Arg Pro Asn Asn Asn Thr Arg Arg Gly Ile Ser Ile Gly
TGT ACA AGA CCC AAC AAC AAT ACA AGA AGG GGT ATA AGT ATA GGA


                    20                      25                      30
Pro Gly Arg Ala Phe Val Tyr Ala Thr Lys Ile Ile Gly Asp Ile
CCA GGG AGA GCA TTT GTT TAT GCA ACA AAA ATA ATA GGA GAT ATA


                    35
Arg Gln Ala His Cys
AGA CAA GCA CAT TGT
```

(2)      INFORMATION FOR SEQ ID NO: EE543-2
         (i)      SEQUENCE CHARACTERISTICS:
                  (A)      LENGTH:  105
                  (B)      TYPE:  Nucleic Acid
                  (C)      STRANDEDNESS:  Single
                  (D)      TOPOLOGY:  Linear
         (ii)   KIND: cDNA to genomic RNA
         (ii)   KIND (if peptide or protein):
                  (A)      SEQUENCE ASSEMBLY METHOD:  Overlap
                  (B)      FRAGMENT TYPE:  Internal Fragment
                  (C)      HYPOTHETICAL:  _____
         (iii)  ORIGINAL SOURCE: HIV
                  (E)      INDIVIDUAL ISOLATE:  _____
         (iv)   IMMEDIATE SOURCE:
                  (C)      CLONE:  _____
         (v)    POSITION IN GENOME: Within Env Gene
         (vi)   PROPERTIES OF SEQUENCE:  Expresses conserved
                                         antigenic determinant
         (viii) SEQUENCE DESCRIPTION:


SEQ ID NO:   EE543-2


1                    5                        10                        15
Cys Thr Arg Pro Asn Asn Asn Thr Arg Lys Ser Ile Thr Ile Gly
TGT ACA AGA CCC AAT AAC AAT ACA AGA AAA AGT ATA ACT ATA GGA


                     20                       25                        30
Pro Gly Arg Ala Phe Tyr Ala Thr Gly Glu Ile Ile Gly Asp Ile
CCA GGG AGA GCA TTT TAT GCA ACA GGA GAA ATA ATA GGA GAT ATA


                     35
Arg Gln Ala His Cys
AGA CAA GCA CAT TGT


(2)      INFORMATION FOR SEQ ID NO: EE543-3
         (i)      SEQUENCE CHARACTERISTICS:
                  (A)      LENGTH:  105
                  (B)      TYPE:  Nucleic Acid
                  (C)      STRANDEDNESS:  Single
                  (D)      TOPOLOGY:  Linear
         (ii)   KIND: cDNA to genomic RNA
         (ii)   KIND (if peptide or protein):
                  (A)      SEQUENCE ASSEMBLY METHOD:  Overlap
                  (B)      FRAGMENT TYPE:  Internal Fragment

```
                    (C)      HYPOTHETICAL:  _____
         (iii)   ORIGINAL SOURCE: HIV
                    (E)      INDIVIDUAL ISOLATE:  _____
         (iv)    IMMEDIATE SOURCE:
                    (C)      CLONE:  _____
         (v)     POSITION IN GENOME: Within Env Gene
         (vi)    PROPERTIES OF SEQUENCE:   Expresses conserved
                                           antigenic determinant
         (viii)  SEQUENCE DESCRIPTION:


SEQ ID NO:   EE543-3


    1                5                    10                   15
Cys Thr Arg Pro Asn Asn Asn Thr Arg Lys Ser Ile Thr Ile Gly
TGT ACA AGA CCC AAT AAC AAT ACA AGA AAA AGT ATA ACT ATA GGA


                    20                   25                   30
Pro Gly Arg Ala Phe Tyr Ala Thr Gly Glu Ile Ile Gly Asp Ile
CCA GGG AGA GCA TTT TAT GCA ACA GGA GAA ATA ATA GGA GAT ATA


                    35
Arg Gln Ala His Cys
AGA CAA GCA CAT TGT


(2)      INFORMATION FOR SEQ ID NO: EE558-1
         (i)     SEQUENCE CHARACTERISTICS:
                    (A)      LENGTH:  105
                    (B)      TYPE:  Nucleic Acid
                    (C)      STRANDEDNESS:   Single
                    (D)      TOPOLOGY:  Linear
         (ii)    KIND: cDNA to genomic RNA
         (ii)    KIND (if peptide or protein):
                    (A)      SEQUENCE ASSEMBLY METHOD:  Overlap
                    (B)      FRAGMENT TYPE:  Internal Fragment
                    (C)      HYPOTHETICAL:  _____
         (iii)   ORIGINAL SOURCE: HIV
                    (E)      INDIVIDUAL ISOLATE:  _____
         (iv)    IMMEDIATE SOURCE:
                    (C)      CLONE:  _____
         (v)     POSITION IN GENOME: Within Env Gene
         (vi)    PROPERTIES OF SEQUENCE:   Expresses conserved
                                           antigenic determinant
         (viii)  SEQUENCE DESCRIPTION:
```

SEQ ID NO:  EE558-1


```
     1                   5                        10                       15
Cys Thr Arg Pro Asn Asn Asn Thr Arg Lys Ser Ile His Ile Gly
TGT ACA AGA CCC AAC AAC AAT ACA AGA AAA AGT ATA CAT ATA GGA


                        20                       25                       30
Pro Gly Arg Ala Phe Tyr Ala Thr Gly Glu Ile Ile Gly Asp Ile
CCA GGG AGA GCA TTT TAT GCA ACA GGA GAA ATA ATA GGA GAT ATA


                        35
Arg Gln Ala His Cys
AGA CAA GCA CAT TGC
```


(2)      INFORMATION FOR SEQ ID NO: EE558-2
         (i)      SEQUENCE CHARACTERISTICS:
                  (A)      LENGTH:  105
                  (B)      TYPE:  Nucleic Acid
                  (C)      STRANDEDNESS:  Single
                  (D)      TOPOLOGY:  Linear
         (ii)     KIND: cDNA to genomic RNA
         (ii)     KIND (if peptide or protein):
                  (A)      SEQUENCE ASSEMBLY METHOD:  Overlap
                  (B)      FRAGMENT TYPE:  Internal Fragment
                  (C)      HYPOTHETICAL:  _____
         (iii)    ORIGINAL SOURCE: HIV
                  (E)      INDIVIDUAL ISOLATE:  _____
         (iv)     IMMEDIATE SOURCE:
                  (C)      CLONE:  _____
         (v)      POSITION IN GENOME: Within Env Gene
         (vi)     PROPERTIES OF SEQUENCE:  Expresses conserved
                                           antigenic determinant
         (viii)   SEQUENCE DESCRIPTION:


SEQ ID NO:  EE558-2


```
     1                   5                        10                       15
Cys Thr Arg Pro Asn Asn Asn Thr Arg Lys Ser Ile His Leu Gly
TGT ACA AGA CCC AAC AAC AAT ACA AGA AAA AGT ATA CAT CTA GGG


                        20                       25                       30
Pro Gly Arg Ala Phe Tyr Ala Thr Gly Glu Ile Ile Gly Asp Ile
CCA GGG AGA GCA TTT TAT GCA ACA GGA GAA ATA ATA GGA GAT ATA
```

```
                    35
Arg Gln Ala His Cys
AGA CAA GCA CAT TGC


(2)      INFORMATION FOR SEQ ID NO: EE558-3
         (i)     SEQUENCE CHARACTERISTICS:
                 (A)      LENGTH:  105
                 (B)      TYPE:  Nucleic Acid
                 (C)      STRANDEDNESS:  Single
                 (D)      TOPOLOGY:  Linear
         (ii)    KIND: cDNA to genomic RNA
         (ii)    KIND (if peptide or protein):
                 (A)      SEQUENCE ASSEMBLY METHOD:  Overlap
                 (B)      FRAGMENT TYPE:  Internal Fragment
                 (C)      HYPOTHETICAL:  _____
         (iii)   ORIGINAL SOURCE: HIV
                 (E)      INDIVIDUAL ISOLATE:  _____
         (iv)    IMMEDIATE SOURCE:
                 (C)      CLONE:  _____
         (v)     POSITION IN GENOME: Within Env Gene
         (vi)    PROPERTIES OF SEQUENCE:  Expresses conserved
                                          antigenic determinant
         (viii)  SEQUENCE DESCRIPTION:


SEQ ID NO:  EE558-3


1                5                10               15
Cys Thr Arg Pro Asn Asn Asn Thr Arg Lys Ser Ile His Leu Gly
TGT ACA AGA CCC AAC AAC AAT ACA AGA AAA AGT ATA CAT CTA GGG


                 20               25               30
Pro Gly Arg Ala Phe Tyr Thr Thr Gly Asp Ile Ile Gly Asp Ile
CCA GGG AGA GCA TTT TAT ACA ACA GGA GAC ATA ATA GGA GAT ATA


                    35
Arg Gln Ala His Cys
AGA CAA GCA CAT TGT


(2)      INFORMATION FOR SEQ ID NO: EE594-1
         (i)     SEQUENCE CHARACTERISTICS:
                 (A)      LENGTH:  105
                 (B)      TYPE:  Nucleic Acid
```

```
            (C)      STRANDEDNESS:  Single
            (D)      TOPOLOGY:  Linear
      (ii)  KIND: cDNA to genomic RNA
      (ii)  KIND (if peptide or protein):
            (A)      SEQUENCE ASSEMBLY METHOD:  Overlap
            (B)      FRAGMENT TYPE:  Internal Fragment
            (C)      HYPOTHETICAL:  _____ .
      (iii) ORIGINAL SOURCE: HIV
            (E)      INDIVIDUAL ISOLATE:  _____
      (iv)  IMMEDIATE SOURCE:
            (C)      CLONE:  _____
      (v)   POSITION IN GENOME: Within Env Gene
      (vi)  PROPERTIES OF SEQUENCE:  Expresses conserved
                                      antigenic determinant
      (viii) SEQUENCE DESCRIPTION:


SEQ ID NO:  EE594-1


1                5                     10                      15
Cys Thr Arg Pro Asn Asn Asn Thr MET Lys Ser Ile His Ile Gly
TGT ACA AGA CCC AAC AAC AAT ACA ATG AAA AGT ATA CAT ATA GGA


                 20                    25                      30
Pro Gly Arg Ala Phe Tyr Thr Thr Gly Gln Ile Ile Gly Asp Ile
CCA GGG AGA GCA TTT TAT ACA ACA GGA CAA ATA ATA GGA GAT ATA


                 35
Arg Gln Ala His Cys
AGA CAA GCA CAT TGT


(2)   INFORMATION FOR SEQ ID NO: EE594-2
      (i)   SEQUENCE CHARACTERISTICS:
            (A)      LENGTH:  105
            (B)      TYPE:  Nucleic Acid
            (C)      STRANDEDNESS:  Single
            (D)      TOPOLOGY:  Linear
      (ii)  KIND: cDNA to genomic RNA
      (ii)  KIND (if peptide or protein):
            (A)      SEQUENCE ASSEMBLY METHOD:  Overlap
            (B)      FRAGMENT TYPE:  Internal Fragment
            (C)      HYPOTHETICAL:  _____
      (iii) ORIGINAL SOURCE: HIV
            (E)      INDIVIDUAL ISOLATE:  _____
      (iv)  IMMEDIATE SOURCE:
```

```
              (C)      CLONE:  _____
      (v)      POSITION IN GENOME: Within Env Gene
      (vi)     PROPERTIES OF SEQUENCE:  Expresses conserved
                                        antigenic determinant
      (viii)   SEQUENCE DESCRIPTION:


SEQ ID NO:  EE594-2


  1                5                      10                      15
Cys Thr Arg Pro Asn Asn Asn Thr MET Lys Ser Ile His Ile Gly
TGT ACA AGA CCC AAC AAC AAT ACA ATG AAA AGT ATA CAT ATA GGA


                    20                      25                      30
Pro Gly Arg Ala Phe Tyr Thr Thr Gly Gln Ile Ile Gly Asp Ile
CCA GGG AGA GCA TTT TAT ACA ACA GGA CAA ATA ATA GGA GAT ATA


                    35
Arg Gln Ala His Cys
AGA CAA GCA CAT TGT


(2)      INFORMATION FOR SEQ ID NO: EE594-3
      (i)       SEQUENCE CHARACTERISTICS:
                (A)      LENGTH:  105
                (B)      TYPE:  Nucleic Acid
                (C)      STRANDEDNESS:  Single
                (D)      TOPOLOGY:  Linear
      (ii)     KIND: cDNA to genomic RNA
      (ii)     KIND (if peptide or protein):
                (A)      SEQUENCE ASSEMBLY METHOD:  Overlap
                (B)      FRAGMENT TYPE:  Internal Fragment
                (C)      HYPOTHETICAL:  _____
      (iii)    ORIGINAL SOURCE: HIV
                (E)      INDIVIDUAL ISOLATE:  _____
      (iv)     IMMEDIATE SOURCE:
                (C)      CLONE:  _____
      (v)      POSITION IN GENOME: Within Env Gene
      (vi)     PROPERTIES OF SEQUENCE:  Expresses conserved
                                        antigenic determinant
      (viii)   SEQUENCE DESCRIPTION:


SEQ ID NO:  EE594-3
```

```
     1               5                    10                   15
Cys Thr Arg Pro Asn Asn Asn Thr MET Lys Ser Ile His Ile Gly
TGT ACA AGA CCC AAC AAC AAT ACA ATG AAA AGT ATA CAT ATA GGA


                    20                   25                   30
Pro Gly Arg Ala Phe Tyr Thr Thr Gly Gln Ile Ile Gly Asp Ile
CCA GGG AGA GCA TTT TAT ACA ACA GGA CAA ATA ATA GGA GAT ATA


                    35
Arg Gln Ala His Cys
AGA CAA GCA CAT TGT
```

(2)      INFORMATION FOR SEQ ID NO: EE628-1
    (i)       SEQUENCE CHARACTERISTICS:
        (A)       LENGTH:  105
        (B)       TYPE:  Nucleic Acid
        (C)       STRANDEDNESS:  Single
        (D)       TOPOLOGY:  Linear
    (ii)      KIND: cDNA to genomic RNA
    (ii)      KIND (if peptide or protein):
        (A)       SEQUENCE ASSEMBLY METHOD:  Overlap
        (B)       FRAGMENT TYPE:  Internal Fragment
        (C)       HYPOTHETICAL:  _____
    (iii)     ORIGINAL SOURCE: HIV
        (E)       INDIVIDUAL ISOLATE:  _____
    (iv)      IMMEDIATE SOURCE:
        (C)       CLONE:  _____
    (v)       POSITION IN GENOME: Within Env Gene
    (vi)      PROPERTIES OF SEQUENCE:  Expresses conserved
                             antigenic determinant
    (viii)    SEQUENCE DESCRIPTION:


SEQ ID NO:  EE628-1


```
     1               5                    10                   15
Cys Thr Arg Pro Asn Asn Asn Thr Arg Lys Gly Ile His MET Gly
TGT ACA AGA CCC AAC AAT AAT ACA AGA AAA GGT ATA CAT ATG GGA


                    20                   25                   30
Pro Gly Lys Ala Phe Tyr Ala Thr Gly Asp Ile Ile Gly Asn Ile
CCA GGG AAA GCA TTT TAT GCA ACA GGG GAC ATA ATA GGA AAT ATA
```

EP 0 471 407 A2

```
                          35
Arg Gln Ala His Cys
AGA CAA GCA CAT TGT


(2)        INFORMATION FOR SEQ ID NO: EE628-2
           (i)     SEQUENCE CHARACTERISTICS:
                   (A)     LENGTH:  105
                   (B)     TYPE:  Nucleic Acid
                   (C)     STRANDEDNESS:  Single
                   (D)     TOPOLOGY:  Linear
           (ii)    KIND: cDNA to genomic RNA
           (ii)    KIND (if peptide or protein):
                   (A)     SEQUENCE ASSEMBLY METHOD:  Overlap
                   (B)     FRAGMENT TYPE:  Internal Fragment
                   (C)     HYPOTHETICAL:  _____
           (iii)   ORIGINAL SOURCE: HIV
                   (E)     INDIVIDUAL ISOLATE:  _____
           (iv)    IMMEDIATE SOURCE:
                   (C)     CLONE:  _____
           (v)     POSITION IN GENOME: Within Env Gene
           (vi)    PROPERTIES OF SEQUENCE:  Expresses conserved
                                            antigenic determinant
           (viii)  SEQUENCE DESCRIPTION:


SEQ ID NO:  EE628-2


1                  5                   10                  15
Cys Thr Arg Pro Asn Asn Asn Thr Arg Lys Gly Ile His MET Gly
TGT ACA AGA CCC AAC AAC AAT ACA AGA AAA GGT ATA CAT ATG GGA


                   20                  25                  30
Pro Gly Lys Ala Phe Tyr Ala Thr Gly Asp Ile Ile Gly Asn Ile
CCA GGG AAA GCA TTT TAT GCA ACA GGG GAC ATA ATA GGA AAT ATA


                   35
Arg Gln Ala His Cys
AGA CAA GCA CAT TGT


(2)        INFORMATION FOR SEQ ID NO: EE628-3
           (i)     SEQUENCE CHARACTERISTICS:
                   (A)     LENGTH:  105
                   (B)     TYPE:  Nucleic Acid
                   (C)     STRANDEDNESS:  Single
```

151

```
                   (D)      TOPOLOGY:  Linear
          (ii)     KIND: cDNA to genomic RNA
          (ii)     KIND (if peptide or protein):
                   (A)      SEQUENCE ASSEMBLY METHOD:  Overlap
                   (B)      FRAGMENT TYPE:  Internal Fragment
                   (C)      HYPOTHETICAL:  _____
          (iii)    ORIGINAL SOURCE: HIV
                   (E)      INDIVIDUAL ISOLATE:  _____
          (iv)     IMMEDIATE SOURCE:
                   (C)      CLONE:  _____
          (v)      POSITION IN GENOME: Within Env Gene
          (vi)     PROPERTIES OF SEQUENCE:  Expresses conserved
                                            antigenic determinant
          (viii)   SEQUENCE DESCRIPTION:
```

SEQ ID NO:   EE628-3

```
     1               5                    10                   15
Cys Thr Arg Pro Asn Asn Asn Thr Arg Lys Gly Ile His MET Gly
TGT ACA AGA CCC AAC AAC AAT ACA AGA AAA GGT ATA CAT ATG GGA


                    20                   25                   30
Pro Gly Lys Ala Phe Tyr Ala Thr Gly Asp Ile Ile Gly Asn Ile
CCA GGG AAA GCA TTT TAT GCA ACA GGG GAC ATA ATA GGA AAT ATA


                    35
Arg Gln Ala His Cys
AGA CAA GCA CAT TGT
```

```
     (2)     INFORMATION FOR SEQ ID NO: EE639-1
             (i)      SEQUENCE CHARACTERISTICS:
                      (A)      LENGTH:  105
                      (B)      TYPE:  Nucleic Acid
                      (C)      STRANDEDNESS:  Single
                      (D)      TOPOLOGY:  Linear
             (ii)     KIND: cDNA to genomic RNA
             (ii)     KIND (if peptide or protein):
                      (A)      SEQUENCE ASSEMBLY METHOD:  Overlap
                      (B)      FRAGMENT TYPE:  Internal Fragment
                      (C)      HYPOTHETICAL:  _____
             (iii)    ORIGINAL SOURCE: HIV
                      (E)      INDIVIDUAL ISOLATE:  _____
             (iv)     IMMEDIATE SOURCE:
                      (C)      CLONE:  _____
```

(v)      POSITION IN GENOME: Within Env Gene
(vi)     PROPERTIES OF SEQUENCE:  Expresses conserved
                                  antigenic determinant
(viii)   SEQUENCE DESCRIPTION:


SEQ ID NO:   EE639-1


```
 1                    5                     10                    15
Cys Thr Arg Pro Asn Asn His Thr Glu Lys Arg Ile Thr Leu Gly
TGT ACA AGA CCC AAC AAC CAT ACA GAA AAA CGT ATA ACT CTA GGA


                      20                    25                    30
Pro Gly Arg Val Leu Tyr Thr Thr Gly Arg Ile Ile Gly Asp Ile
CCG GGG AGA GTA CTT TAT ACA ACA GGA AGA ATA ATA GGA GAT ATA


                      35
Arg Arg Ala His Cys
AGA CGA GCA CAT TGT
```


(2)      INFORMATION FOR SEQ ID NO: EE639-2
         (i)      SEQUENCE CHARACTERISTICS:
                  (A)      LENGTH:   105
                  (B)      TYPE:   Nucleic Acid
                  (C)      STRANDEDNESS:   Single
                  (D)      TOPOLOGY:   Linear
         (ii)     KIND: cDNA to genomic RNA
         (ii)     KIND (if peptide or protein):
                  (A)      SEQUENCE ASSEMBLY METHOD:   Overlap
                  (B)      FRAGMENT TYPE:   Internal Fragment
                  (C)      HYPOTHETICAL:  _____
         (iii)    ORIGINAL SOURCE: HIV
                  (E)      INDIVIDUAL ISOLATE:  _____
         (iv)     IMMEDIATE SOURCE:
                  (C)      CLONE:  _____
         (v)      POSITION IN GENOME: Within Env Gene
         (vi)     PROPERTIES OF SEQUENCE:   Expresses conserved
                                            antigenic determinant
         (viii)   SEQUENCE DESCRIPTION:


SEQ ID NO:   EE639-2


153

```
1                   5                        10                       15
Cys Thr Arg Pro Asn Asn His Thr Glu Lys Arg Ile Thr Leu Gly
TGT ACA AGA CCC AAC AAC CAT ACA GAA AAA CGT ATA ACT CTA GGA


                    20                       25                       30
Pro Gly Arg Val Leu Tyr Thr Thr Gly Arg Ile Ile Gly Asp Ile
CCG GGG AGA GTA CTT TAT ACA ACA GGA AGA ATA ATA GGA GAT ATA


                    35
Arg Arg Ala His Cys
AGA CGA GCA CAT TGT
```

```
(2)       INFORMATION FOR SEQ ID NO: EE639-3
          (i)     SEQUENCE CHARACTERISTICS:
                  (A)     LENGTH:  105
                  (B)     TYPE:  Nucleic Acid
                  (C)     STRANDEDNESS:  Single
                  (D)     TOPOLOGY:  Linear
          (ii)    KIND: cDNA to genomic RNA
          (ii)    KIND (if peptide or protein):
                  (A)     SEQUENCE ASSEMBLY METHOD:  Overlap
                  (B)     FRAGMENT TYPE:  Internal Fragment
                  (C)     HYPOTHETICAL:  _____
          (iii)   ORIGINAL SOURCE: HIV
                  (E)     INDIVIDUAL ISOLATE:  _____
          (iv)    IMMEDIATE SOURCE:
                  (C)     CLONE:  _____
          (v)     POSITION IN GENOME: Within Env Gene
          (vi)    PROPERTIES OF SEQUENCE:  Expresses conserved
                                           antigenic determinant
          (viii)  SEQUENCE DESCRIPTION:


SEQ ID NO:  EE639-3


1                   5                        10                       15
Cys Thr Arg Pro Asn Asn Asn Thr Arg Lys Ser Ile Pro Ile Gly
TGT ACA AGA CCC AAC AAC AAT ACA AGG AAA AGT ATA CCA ATA GGA


                    20                       25                       30
Pro Gly Arg Ala Phe Tyr Ala Thr Gly Asp Ile Ile Gly Asp Ile
CCA GGG AGA GCA TTT TAT GCA ACA GGA GAC ATA ATA GGA GAT ATA
```

```
                    35
Arg Gln Ala His Cys
AGA CAA GCA CAT TGT
```

(2)        INFORMATION FOR SEQ ID NO: EE660-1
           (i)     SEQUENCE CHARACTERISTICS:
                   (A)     LENGTH:  105
                   (B)     TYPE:  Nucleic Acid
                   (C)     STRANDEDNESS:  Single
                   (D)     TOPOLOGY:  Linear
           (ii)    KIND: cDNA to genomic RNA
           (ii)    KIND (if peptide or protein):
                   (A)     SEQUENCE ASSEMBLY METHOD:  Overlap
                   (B)     FRAGMENT TYPE:  Internal Fragment
                   (C)     HYPOTHETICAL:  _____
           (iii)   ORIGINAL SOURCE: HIV
                   (E)     INDIVIDUAL ISOLATE:  _____
           (iv)    IMMEDIATE SOURCE:
                   (C)     CLONE:  _____
           (v)     POSITION IN GENOME: Within Env Gene
           (vi)    PROPERTIES OF SEQUENCE:  Expresses conserved
                                            antigenic determinant
           (viii)  SEQUENCE DESCRIPTION:


SEQ ID NO:  EE660-1


```
  1             5                 10                  15
Cys Thr Arg Pro Asn Asn Asn Thr Arg Lys Ser Ile Pro Ile Gly
TGT ACA AGA CCC AAC AAC AAT ACA AGA AAA AGT ATA CCT ATA GGA


                20                25                  30
Pro Gly Arg Ala Phe Tyr Thr Thr Gly Asp Val Ile Gly Asp Ile
CCA GGA AGA GCA TTT TAT ACA ACA GGA GAT GTA ATA GGA GAT ATA


                35
Arg Gln Ala Arg Cys
AGA CAA GCA CGT TGT
```

(2)        INFORMATION FOR SEQ ID NO: EE660-2
           (i)     SEQUENCE CHARACTERISTICS:
                   (A)     LENGTH:  105
                   (B)     TYPE:  Nucleic Acid
                   (C)     STRANDEDNESS:  Single

```
                (D)      TOPOLOGY:  Linear
       (ii)   KIND: cDNA to genomic RNA
       (ii)   KIND (if peptide or protein):
                (A)      SEQUENCE ASSEMBLY METHOD:  Overlap
                (B)      FRAGMENT TYPE:  Internal Fragment
                (C)      HYPOTHETICAL: _____
       (iii)  ORIGINAL SOURCE: HIV
                (E)      INDIVIDUAL ISOLATE: _____
       (iv)   IMMEDIATE SOURCE:
                (C)      CLONE: _____
       (v)    POSITION IN GENOME: Within Env Gene
       (vi)   PROPERTIES OF SEQUENCE:  Expresses conserved
                                        antigenic determinant
       (viii) SEQUENCE DESCRIPTION:
```

SEQ ID NO:  EE660-2

```
    1                   5                   10                  15
Cys Thr Arg Pro Asn Asn Asn Thr Arg Arg Ser Ile Asn Ile Gly
TGT ACA AGA CCC AAC AAC AAT ACA AGA AGA AGT ATA AAT ATA GGA


                    20                  25                  30
Pro Gly Arg Ala Phe Tyr Ala Thr Gly Ala Ile Ile Gly Asp Ile
CCA GGG AGA GCA TTC TAT GCA ACA GGA GCC ATA ATA GGA GAT ATA


                    35
Arg Gln Ala His Cys
AGA CAA GCA CAT TGT
```

```
    (2)     INFORMATION FOR SEQ ID NO: EE661-1
            (i)    SEQUENCE CHARACTERISTICS:
                    (A)      LENGTH:  105
                    (B)      TYPE:  Nucleic Acid
                    (C)      STRANDEDNESS:  Single
                    (D)      TOPOLOGY:  Linear
            (ii)   KIND: cDNA to genomic RNA
            (ii)   KIND (if peptide or protein):
                    (A)      SEQUENCE ASSEMBLY METHOD:  Overlap
                    (B)      FRAGMENT TYPE:  Internal Fragment
                    (C)      HYPOTHETICAL: _____
            (iii)  ORIGINAL SOURCE: HIV
                    (E)      INDIVIDUAL ISOLATE: _____
            (iv)   IMMEDIATE SOURCE:
                    (C)      CLONE: _____
```

(v)      POSITION IN GENOME: Within Env Gene
(vi)     PROPERTIES OF SEQUENCE:   Expresses conserved
                                   antigenic determinant
(viii)  SEQUENCE DESCRIPTION:


SEQ ID NO:   EE661-1


```
1                   5                        10                      15
Cys Thr Arg Pro Asn Asn Asn Thr Arg Lys Ser Ile His Ile Gly
TGT ACA AGA CCC AAC AAC AAT ACA AGA AAA AGT ATA CAT ATA GGA


                    20                       25                      30
Pro Gly Arg Ala Phe Tyr Ala Thr Gly Glu Ile Ile Gly Asp Ile
CCA GGG AGA GCA TTT TAT GCA ACA GGA GAA ATA ATA GGA GAT ATA


                    35
Arg Gln Ala His Cys
AGA CAA GCA CAT TGT
```


(2)      INFORMATION FOR SEQ ID NO: EE661-2
         (i)     SEQUENCE CHARACTERISTICS:
                 (A)     LENGTH:   105
                 (B)     TYPE:   Nucleic Acid
                 (C)     STRANDEDNESS:   Single
                 (D)     TOPOLOGY:   Linear
         (ii)    KIND: cDNA to genomic RNA
         (ii)    KIND (if peptide or protein):
                 (A)     SEQUENCE ASSEMBLY METHOD:   Overlap
                 (B)     FRAGMENT TYPE:   Internal Fragment
                 (C)     HYPOTHETICAL:   _____
         (iii)   ORIGINAL SOURCE: HIV
                 (E)     INDIVIDUAL ISOLATE:   _____
         (iv)    IMMEDIATE SOURCE:
                 (C)     CLONE:   _____
         (v)     POSITION IN GENOME: Within Env Gene
         (vi)    PROPERTIES OF SEQUENCE:   Expresses conserved
                                           antigenic determinant
         (viii)  SEQUENCE DESCRIPTION:


SEQ ID NO:   EE661-2

157

```
 1               5                  10                 15
Cys Thr Arg Pro Asn Asn Asn Thr Arg Lys Ser Ile His Ile Gly
TGT ACA AGA CCC AAC AAC AAT ACA AGA AAA AGT ATA CAT ATA GGA


                20                 25                 30
Pro Gly Arg Ala Phe Tyr Ala Thr Gly Glu Ile Ile Gly Asp Ile
CCA GGG AGA GCA TTT TAT GCA ACA GGA GAA ATA ATA GGA GAT ATA


                35
Arg Gln Ala His Cys
AGA CAA GCA CAT TGT
```

(2)      INFORMATION FOR SEQ ID NO: EE661-3
         (i)      SEQUENCE CHARACTERISTICS:
                  (A)      LENGTH:  105
                  (B)      TYPE:  Nucleic Acid
                  (C)      STRANDEDNESS:  Single
                  (D)      TOPOLOGY:  Linear
         (ii)     KIND: cDNA to genomic RNA
         (ii)     KIND (if peptide or protein):
                  (A)      SEQUENCE ASSEMBLY METHOD:  Overlap
                  (B)      FRAGMENT TYPE:  Internal Fragment
                  (C)      HYPOTHETICAL:  _____
         (iii)    ORIGINAL SOURCE: HIV
                  (E)      INDIVIDUAL ISOLATE:  _____
         (iv)     IMMEDIATE SOURCE:
                  (C)      CLONE:  _____
         (v)      POSITION IN GENOME: Within Env Gene
         (vi)     PROPERTIES OF SEQUENCE:  Expresses conserved
                                           antigenic determinant
         (viii)   SEQUENCE DESCRIPTION:


SEQ ID NO:   EE661-3


```
 1               5                  10                 15
Cys Thr Arg Pro Asn Asn Asn Thr Arg Lys Ser Ile Ser Ile Gly
TGT ACA AGA CCC AAC AAC AAT ACA AGA AAA AGT ATA TCT ATA GGA


                20                 25                 30
Pro Gly Arg Ala Phe Phe Thr Thr Gly Gln Ile Ile Gly Asp Ile
CCA GGG AGA GCA TTT TTT ACA ACA GGA CAA ATA ATA GGA GAT ATA
```

158

```
                    35
Arg Gln Ala His Cys
AGA CAA GCA CAT TGT
```

(2)      INFORMATION FOR SEQ ID NO: EE663-1
         (i)      SEQUENCE CHARACTERISTICS:
                  (A)      LENGTH:  105
                  (B)      TYPE:  Nucleic Acid
                  (C)      STRANDEDNESS:  Single
                  (D)      TOPOLOGY:  Linear
         (ii)     KIND: cDNA to genomic RNA
         (ii)     KIND (if peptide or protein):
                  (A)      SEQUENCE ASSEMBLY METHOD:  Overlap
                  (B)      FRAGMENT TYPE:  Internal Fragment
                  (C)      HYPOTHETICAL:  _____
         (iii)    ORIGINAL SOURCE: HIV
                  (E)      INDIVIDUAL ISOLATE:  _____
         (iv)     IMMEDIATE SOURCE:
                  (C)      CLONE:  _____
         (v)      POSITION IN GENOME: Within Env Gene
         (vi)     PROPERTIES OF SEQUENCE:  Expresses conserved
                                           antigenic determinant
         (viii)   SEQUENCE DESCRIPTION:


SEQ ID NO:  EE663-1


```
1                5                10                15
Cys Thr Arg Pro Asn Asn Asn Thr Arg Lys Ser Ile Thr Ile Gly
TGT ACA AGA CCC AAT AAC AAT ACA AGA AAA AGT ATA ACT ATA GGA


                20                25                30
Pro Gly Arg Ala Phe Tyr Ala Thr Gly Glu Ile Ile Gly Asp Ile
CCA GGG AGA GCA TTT TAT GCA ACA GGA GAA ATA ATA GGA GAT ATA


                35
Arg Gln Ala His Cys
AGA CAA GCA CAT TGT
```

(2)      INFORMATION FOR SEQ ID NO: EE663-2
         (i)      SEQUENCE CHARACTERISTICS:
                  (A)      LENGTH:  105
                  (B)      TYPE:  Nucleic Acid
                  (C)      STRANDEDNESS:  Single

```
                    (D)     TOPOLOGY:  Linear
            (ii)    KIND: cDNA to genomic RNA
            (ii)    KIND (if peptide or protein):
                    (A)     SEQUENCE ASSEMBLY METHOD:  Overlap
                    (B)     FRAGMENT TYPE:  Internal Fragment
                    (C)     HYPOTHETICAL:  _____
            (iii)   ORIGINAL SOURCE: HIV
                    (E)     INDIVIDUAL ISOLATE:  _____
            (iv)    IMMEDIATE SOURCE:
                    (C)     CLONE:  _____
            (v)     POSITION IN GENOME: Within Env Gene
            (vi)    PROPERTIES OF SEQUENCE:  Expresses conserved
                                             antigenic determinant
            (viii)  SEQUENCE DESCRIPTION:


SEQ ID NO:  EE663-2


    1                   5                   10                  15
Cys Thr Arg Pro Asn Asn Asn Thr Arg Lys Gly Ile His Ile Gly
TGT ACA AGA CCC AAC AAC AAT ACA AGA AAA GGT ATA CAT ATA GGA


                        20                  25                  30
Pro Gly Arg Ala Phe Tyr Thr Thr Gly Glu Ile Ile Gly Asn Ile
CCA GGG AGA GCA TTT TAT ACA ACA GGA GAA ATA ATA GGA AAT ATA


                        35
Arg Gln Ala His Cys
AGA CAA GCA CAT TGT


    (2)     INFORMATION FOR SEQ ID NO: EE663-3
            (i)     SEQUENCE CHARACTERISTICS:
                    (A)     LENGTH:  105
                    (B)     TYPE:  Nucleic Acid
                    (C)     STRANDEDNESS:  Single
                    (D)     TOPOLOGY:  Linear
            (ii)    KIND: cDNA to genomic RNA
            (ii)    KIND (if peptide or protein):
                    (A)     SEQUENCE ASSEMBLY METHOD:  Overlap
                    (B)     FRAGMENT TYPE:  Internal Fragment
                    (C)     HYPOTHETICAL:  _____
            (iii)   ORIGINAL SOURCE: HIV
                    (E)     INDIVIDUAL ISOLATE:  _____
            (iv)    IMMEDIATE SOURCE:
                    (C)     CLONE:  _____
```

```
(v)      POSITION IN GENOME: Within Env Gene
(vi)     PROPERTIES OF SEQUENCE:  Expresses conserved
                                   antigenic determinant
(viii)  SEQUENCE DESCRIPTION:


SEQ ID NO:  EE663-3


  1                  5                  10                 15
Cys Thr Arg Pro Asn Asn Asn Thr Ile Lys Ser Ile Thr Ile Gly
TGT ACA AGA CCC AAC AAC AAT ACA ATA AAA AGT ATA ACT ATA GGA


                   20                 25                 30
Pro Gly Arg Ala Phe Tyr Ala Thr Gly Glu Ile Ile Gly Asp Ile
CCA GGG AGA GCA TTT TAT GCA ACA GGA GAA ATA ATA GGA GAT ATA


                   35
Arg Gln Ala His Cys
AGA CAA GCA CAT TGT


(2)      INFORMATION FOR SEQ ID NO: EE665-1
         (i)      SEQUENCE CHARACTERISTICS:
                  (A)      LENGTH:  105
                  (B)      TYPE:  Nucleic Acid
                  (C)      STRANDEDNESS:  Single
                  (D)      TOPOLOGY:  Linear
         (ii)     KIND: cDNA to genomic RNA
         (ii)     KIND (if peptide or protein):
                  (A)      SEQUENCE ASSEMBLY METHOD:  Overlap
                  (B)      FRAGMENT TYPE:  Internal Fragment
                  (C)      HYPOTHETICAL:  _____
         (iii)    ORIGINAL SOURCE: HIV
                  (E)      INDIVIDUAL ISOLATE:  _____
         (iv)     IMMEDIATE SOURCE:
                  (C)      CLONE:  _____
         (v)      POSITION IN GENOME: Within Env Gene
         (vi)     PROPERTIES OF SEQUENCE:  Expresses conserved
                                           antigenic determinant
         (viii)  SEQUENCE DESCRIPTION:


SEQ ID NO:  EE665-1
```

```
        1                 5                      10                    15
Cys Thr Arg Pro Asn Asn Asn Thr Arg Arg Ser Ile Pro Ile Gly
TGT ACA AGA CCC AAC AAC AAT ACA AGA AGA AGT ATA CCT ATA GGA


                         20                    25                    30
Pro Gly Arg Ala Phe Tyr Ala Thr Gly Gln Ile Ile Gly Asp Ile
CCA GGG AGA GCA TTT TAT GCA ACA GGA CAA ATA ATA GGA GAT ATA


                         35
Arg Gln Ala His Cys
AGA CAA GCA CAT TGT
```

(2)       INFORMATION FOR SEQ ID NO: EE665-2
          (i)       SEQUENCE CHARACTERISTICS:
                    (A)      LENGTH:  105
                    (B)      TYPE:  Nucleic Acid
                    (C)      STRANDEDNESS:  Single
                    (D)      TOPOLOGY:  Linear
          (ii)      KIND: cDNA to genomic RNA
          (ii)      KIND (if peptide or protein):
                    (A)      SEQUENCE ASSEMBLY METHOD:  Overlap
                    (B)      FRAGMENT TYPE:  Internal Fragment
                    (C)      HYPOTHETICAL:  _____
          (iii)     ORIGINAL SOURCE: HIV
                    (E)      INDIVIDUAL ISOLATE:  _____
          (iv)      IMMEDIATE SOURCE:
                    (C)      CLONE:  _____
          (v)       POSITION IN GENOME: Within Env Gene
          (vi)      PROPERTIES OF SEQUENCE:   Expresses conserved
                                              antigenic determinant
          (viii)    SEQUENCE DESCRIPTION:


SEQ ID NO:  EE665-2


```
        1                 5                      10                    15
Cys Thr Arg Pro Asn Asn Asn Thr Arg Arg Ser Ile Pro Ile Gly
TGT ACA AGA CCC AAC AAC AAT ACA AGA AGA AGT ATA CCT ATA GGA


                         20                    25                    30
Pro Gly Arg Ala Phe Tyr Ala Thr Gly Gln Ile Ile Gly Asp Ile
CCA GGG AGA GCA TTT TAT GCA ACA GGA CAA ATA ATA GGA GAT ATA
```

```
                35
Arg Gln Ala His Cys
AGA CAA GCA CAT TGT


(2)      INFORMATION FOR SEQ ID NO: EE665-3
         (i)      SEQUENCE CHARACTERISTICS:
                  (A)      LENGTH:  105
                  (B)      TYPE:  Nucleic Acid
                  (C)      STRANDEDNESS:  Single
                  (D)      TOPOLOGY:  Linear
         (ii)     KIND: cDNA to genomic RNA
         (ii)     KIND (if peptide or protein):
                  (A)      SEQUENCE ASSEMBLY METHOD:  Overlap
                  (B)      FRAGMENT TYPE:  Internal Fragment
                  (C)      HYPOTHETICAL:  _____
         (iii)    ORIGINAL SOURCE: HIV
                  (E)      INDIVIDUAL ISOLATE:  _____
         (iv)     IMMEDIATE SOURCE:
                  (C)      CLONE:  _____
         (v)      POSITION IN GENOME: Within Env Gene
         (vi)     PROPERTIES OF SEQUENCE:  Expresses conserved
                                           antigenic determinant
         (viii)   SEQUENCE DESCRIPTION:


SEQ ID NO:   EE665-3


1                 5                   10                  15
Cys Thr Arg Pro Asn Asn Asn Thr Arg Arg Ser Ile Pro Ile Gly
TGT ACA AGA CCC AAC AAC AAT ACA AGA AGA AGT ATA CCT ATA GGA


                  20                  25                  30
Pro Gly Arg Ala Phe Tyr Ala Thr Gly Gln Ile Ile Gly Asp Ile
CCA GGG AGA GCA TTT TAT GCA ACA GGA CAA ATA ATA GGA GAT ATA


                35
Arg Gln Ala His Cys
AGA CAA GCA CAT TGT


(2)      INFORMATION FOR SEQ ID NO: EE667-1
         (i)      SEQUENCE CHARACTERISTICS:
                  (A)      LENGTH:  105
                  (B)      TYPE:  Nucleic Acid
                  (C)      STRANDEDNESS:  Single
```

163

```
              (D)       TOPOLOGY:  Linear
     (ii)     KIND: cDNA to genomic RNA
     (ii)     KIND (if peptide or protein):
              (A)       SEQUENCE ASSEMBLY METHOD:  Overlap
              (B)       FRAGMENT TYPE:  Internal Fragment
              (C)       HYPOTHETICAL:  _____
     (iii)    ORIGINAL SOURCE: HIV
              (E)       INDIVIDUAL ISOLATE:  _____
     (iv)     IMMEDIATE SOURCE:
              (C)       CLONE:  _____
     (v)      POSITION IN GENOME: Within Env Gene
     (vi)     PROPERTIES OF SEQUENCE:  Expresses conserved
                                       antigenic determinant
     (viii)   SEQUENCE DESCRIPTION:
```

SEQ ID NO:  EE667-1

```
1                5                    10                   15
Cys Thr Arg Pro Asn Asn Asn Thr Arg Lys Arg Ile Thr Thr Gly
TGT ACA AGA CCC AAC AAC AAT ACA AGA AAA AGA ATA ACT ACG GGA


                 20                   25                   30
Pro Gly Arg Val Tyr Tyr Thr Thr Gly Asp Ile Ile Gly Asp Ile
CCG GGG AGA GTA TAT TAT ACA ACA GGA GAT ATA ATA GGA GAT ATA


                 35
Arg Gln Ala His Cys
AGA CAA GCA CAT TGT
```

```
(2)      INFORMATION FOR SEQ ID NO: EE667-2
         (i)       SEQUENCE CHARACTERISTICS:
                   (A)       LENGTH:  105
                   (B)       TYPE:  Nucleic Acid
                   (C)       STRANDEDNESS:  Single
                   (D)       TOPOLOGY:  Linear
         (ii)      KIND: cDNA to genomic RNA
         (ii)      KIND (if peptide or protein):
                   (A)       SEQUENCE ASSEMBLY METHOD:  Overlap
                   (B)       FRAGMENT TYPE:  Internal Fragment
                   (C)       HYPOTHETICAL:  _____
         (iii)     ORIGINAL SOURCE: HIV
                   (E)       INDIVIDUAL ISOLATE:  _____
         (iv)      IMMEDIATE SOURCE:
                   (C)       CLONE:  _____
```

```
(v)       POSITION IN GENOME: Within Env Gene
(vi)      PROPERTIES OF SEQUENCE:  Expresses conserved
                                   antigenic determinant
(viii)  SEQUENCE DESCRIPTION:


SEQ ID NO:  EE667-2


  1               5                   10                  15
Cys Thr Arg Pro Ser Asn Asn Thr Arg Lys Ser Ile His Ile Gly
TGT ACA AGA CCC AGC AAC AAT ACA AGA AAA AGT ATA CAT ATA GGA


                 20                  25                  30
Pro Gly Arg Ala Phe Tyr Thr Thr Gly Glu Ile Ile Glu Asn Ile
CCA GGG AGA GCA TTT TAT ACA ACA GGA GAA ATA ATA GAA AAT ATA



                 35
Arg Gln Ala His Cys
AGA CAA GCA CAC TGT


(2)       INFORMATION FOR SEQ ID NO: EE667-3
          (i)     SEQUENCE CHARACTERISTICS:
                  (A)      LENGTH:  105
                  (B)      TYPE:  Nucleic Acid
                  (C)      STRANDEDNESS:  Single
                  (D)      TOPOLOGY:  Linear
          (ii)    KIND: cDNA to genomic RNA
          (ii)    KIND (if peptide or protein):
                  (A)      SEQUENCE ASSEMBLY METHOD:  Overlap
                  (B)      FRAGMENT TYPE:  Internal Fragment
                  (C)      HYPOTHETICAL:  _____
          (iii)   ORIGINAL SOURCE: HIV
                  (E)      INDIVIDUAL ISOLATE:  _____
          (iv)    IMMEDIATE SOURCE:
                  (C)      CLONE:  _____
          (v)     POSITION IN GENOME: Within Env Gene
          (vi)    PROPERTIES OF SEQUENCE:  Expresses conserved
                                           antigenic determinant
          (viii)  SEQUENCE DESCRIPTION:


SEQ ID NO:  EE667-3


  1               5                   10                  15
Cys Thr Arg Pro Ser Asn Asn Thr Arg Lys Ser Ile His Ile Ala
TGC ACA AGG CCC AGC AAC AAT ACA AGA AAA AGT ATA CAT ATA GCA
```

```
                  20                   25                  30
Pro Gly Arg Ala Phe Tyr Thr Thr Gly Glu Ile Ile Glu Asn Ile
CCA GGG AGA GCA TTT TAT ACA ACA GGA GAA ATA ATA GAA AAT ATA



                  35
Arg Gln Ala His Cys
AGA CAA GCA CAC TGT
```

(2)      INFORMATION FOR SEQ ID NO: EE669-1
         (i)      SEQUENCE CHARACTERISTICS:
                  (A)      LENGTH:  105
                  (B)      TYPE:  Nucleic Acid
                  (C)      STRANDEDNESS:  Single
                  (D)      TOPOLOGY:  Linear
         (ii)     KIND: cDNA to genomic RNA
         (ii)     KIND (if peptide or protein):
                  (A)      SEQUENCE ASSEMBLY METHOD:  Overlap
                  (B)      FRAGMENT TYPE:  Internal Fragment
                  (C)      HYPOTHETICAL:  _____
         (iii)    ORIGINAL SOURCE: HIV
                  (E)      INDIVIDUAL ISOLATE:  _____
         (iv)     IMMEDIATE SOURCE:
                  (C)      CLONE:  _____
         (v)      POSITION IN GENOME: Within Env Gene
         (vi)     PROPERTIES OF SEQUENCE:  Expresses conserved
                                           antigenic determinant
         (viii)   SEQUENCE DESCRIPTION:


SEQ ID NO:  EE669-1


```
1                 5                   10                  15
Cys Thr Arg Pro Asn Asn Asn Thr Arg Lys Ser Ile Pro Ile Gly
TGT ACA AGA CCT AAC AAC AAT ACA AGA AAA AGT ATA CCT ATA GGA



                  20                   25                  30
Pro Gly Arg Ala Ile Tyr Ala Thr Gly Glu Ile Ile Gly Asp Ile
CCA GGG AGA GCA ATT TAT GCA ACA GGA GAA ATA ATA GGA GAT ATA



                  35
Arg Gln Ala His Cys
AGA CAA GCA CAT TGT
```

(2)      INFORMATION FOR SEQ ID NO: EE669-2
      (i)     SEQUENCE CHARACTERISTICS:
          (A)    LENGTH:  105
          (B)    *TYPE: Nucleic Acid*
          (C)    STRANDEDNESS:  Single
          (D)    TOPOLOGY:  Linear
      (ii)    KIND: cDNA to genomic RNA
      (ii)    KIND (if peptide or protein):
          (A)    SEQUENCE ASSEMBLY METHOD:  Overlap
          (B)    FRAGMENT TYPE:  Internal Fragment
          (C)    HYPOTHETICAL:  _____
      (iii)   ORIGINAL SOURCE: HIV
          (E)    INDIVIDUAL ISOLATE:  _____
      (iv)   IMMEDIATE SOURCE:
          (C)    CLONE:  _____
      (v)    POSITION IN GENOME: Within Env Gene
      (vi)   PROPERTIES OF SEQUENCE:  Expresses conserved
                          antigenic determinant
      (viii) SEQUENCE DESCRIPTION:

SEQ ID NO:  EE669-2

```
1                 5                 10                15
Cys Thr Arg Pro Asn Asn Asn Thr Arg Lys Ser Ile Pro Ile Gly
TGT ACA AGA CCT AAC AAC AAT ACA AGA AAA AGT ATA CCT ATA GGA


                  20                25                30
Pro Gly Arg Ala Ile Tyr Ala Thr Gly Glu Ile Ile Gly Asp Ile
CCA GGG AGA GCA ATT TAT GCA ACA GGA GAA ATA ATA GGA GAT ATA


                  35
Arg Gln Ala His Cys
AGA CAA GCA CAT TGT
```

(2)      INFORMATION FOR SEQ ID NO: EE669-3
      (i)     SEQUENCE CHARACTERISTICS:
          (A)    LENGTH:  105
          (B)    TYPE:  Nucleic Acid
          (C)    STRANDEDNESS:  Single
          (D)    TOPOLOGY:  Linear
      (ii)    KIND: cDNA to genomic RNA
      (ii)    KIND (if peptide or protein):
          (A)    SEQUENCE ASSEMBLY METHOD:  Overlap
          (B)    FRAGMENT TYPE:  Internal Fragment

```
                    (C)      HYPOTHETICAL:  _____
          (iii)    ORIGINAL SOURCE: HIV
                    (E)      INDIVIDUAL ISOLATE:  _____
          (iv)     IMMEDIATE SOURCE:
                    (C)      CLONE:  _____
          (v)      POSITION IN GENOME: Within Env Gene
          (vi)     PROPERTIES OF SEQUENCE:  Expresses conserved
                                             antigenic determinant
          (viii)   SEQUENCE DESCRIPTION:


     SEQ ID NO:   EE669-3


      1               5                     10                    15
     Cys Thr Arg Pro Asn Asn Asn Thr Arg Lys Ser Ile Pro Ile Gly
     TGT ACA AGA CCT AAC AAC AAT ACA AGA AAA AGT ATA CCT ATA GGA


                     20                    25                    30
     Pro Gly Arg Ala Phe Tyr Thr Thr Gly Glu Ile Ile Gly Asp Ile
     CCA GGG AGA GCA TTT TAT ACA ACA GGA GAA ATA ATA GGA GAT ATA


                     35
     Arg Gln Ala His Cys
     AGA CAA GCA CAT TGT


     (2)      INFORMATION FOR SEQ ID NO: EE1476-1
              (i)      SEQUENCE CHARACTERISTICS:
                       (A)      LENGTH:  102
                       (B)      TYPE:  Nucleic Acid
                       (C)      STRANDEDNESS:  Single
                       (D)      TOPOLOGY:  Linear
              (ii)     KIND: cDNA to genomic RNA
              (ii)     KIND (if peptide or protein):
                       (A)      SEQUENCE ASSEMBLY METHOD:  Overlap
                       (B)      FRAGMENT TYPE:  Internal Fragment
                       (C)      HYPOTHETICAL:  _____
              (iii)    ORIGINAL SOURCE: HIV
                       (E)      INDIVIDUAL ISOLATE:  _____
              (iv)     IMMEDIATE SOURCE:
                       (C)      CLONE:  _____
              (v)      POSITION IN GENOME: Within Env Gene
              (vi)     PROPERTIES OF SEQUENCE:  Expresses conserved
                                                 antigenic determinant
              (viii)   SEQUENCE DESCRIPTION:
```

SEQ ID NO: EE1476-1

| 1 | | | | 5 | | | | | 10 | | | | | 15 |
|---|---|---|---|---|---|---|---|---|---|---|---|---|---|---|
| Cys | Thr | Arg | Pro | Tyr | Asn | Asn | Ile | Lys | Ile | Arg | Ser | Ile | His | Ile |
| TGT | ACA | AGG | CCC | TAC | AAC | AAT | ATA | AAA | ATA | AGA | AGT | ATA | CAT | ATA |

| | | | | 20 | | | | | 25 | | | | | 30 |
|---|---|---|---|---|---|---|---|---|---|---|---|---|---|---|
| Gly | Pro | Gly | Arg | Pro | Phe | Tyr | Thr | Thr | Lys | Ile | Gly | Asp | Ile | Arg |
| GGA | CCA | GGG | AGA | CCA | TTT | TAT | ACA | ACA | AAA | ATA | GGA | GAT | ATA | AGA |

| | | | 35 |
|---|---|---|---|
| Gln | Ala | Tyr | Cys |
| CAA | GCA | TAT | TGT |

(2)      INFORMATION FOR SEQ ID NO: EE3032-1
      (i)      SEQUENCE CHARACTERISTICS:
            (A)      LENGTH: 105
            (B)      TYPE: Nucleic Acid
            (C)      STRANDEDNESS: Single
            (D)      TOPOLOGY: Linear
      (ii)    KIND: cDNA to genomic RNA
      (ii)    KIND (if peptide or protein):
            (A)      SEQUENCE ASSEMBLY METHOD: Overlap
            (B)      FRAGMENT TYPE: Internal Fragment
            (C)      HYPOTHETICAL: _____
      (iii)   ORIGINAL SOURCE: HIV
            (E)      INDIVIDUAL ISOLATE: _____
      (iv)    IMMEDIATE SOURCE:
            (C)      CLONE: _____
      (v)     POSITION IN GENOME: Within Env Gene
      (vi)    PROPERTIES OF SEQUENCE: Expresses conserved
                            antigenic determinant
      (viii) SEQUENCE DESCRIPTION:

SEQ ID NO: EE3032-1

| 1 | | | | 5 | | | | | 10 | | | | | 15 |
|---|---|---|---|---|---|---|---|---|---|---|---|---|---|---|
| Cys | Thr | Arg | Pro | Asn | Asn | Asn | Thr | Arg | Lys | Ser | Ile | His | Ile | Gly |
| TGT | ACA | AGG | CCC | AAT | AAC | AAT | ACA | AGA | AAA | AGT | ATA | CAT | ATA | GGA |

| | | | | 20 | | | | | 25 | | | | | 30 |
|---|---|---|---|---|---|---|---|---|---|---|---|---|---|---|
| Pro | Gly | Arg | Ala | Phe | Tyr | Thr | Thr | Gly | Asp | Ile | Ile | Gly | Asp | Ile |
| CCA | GGG | AGG | GCA | TTT | TAT | ACA | ACA | GGA | GAC | ATA | ATA | GGA | GAT | ATA |

```
                35
Arg Gln Ala His Cys
AGA CAA GCA CAT TGT
```

```
(2)      INFORMATION FOR SEQ ID NO: EE3032-2
         (i)     SEQUENCE CHARACTERISTICS:
                 (A)     LENGTH:  105
                 (B)     TYPE:  Nucleic Acid
                 (C)     STRANDEDNESS:  Single
                 (D)     TOPOLOGY:  Linear
         (ii)    KIND: cDNA to genomic RNA
         (ii)    KIND (if peptide or protein):
                 (A)     SEQUENCE ASSEMBLY METHOD:  Overlap
                 (B)     FRAGMENT TYPE:  Internal Fragment
                 (C)     HYPOTHETICAL:  _____
         (iii)   ORIGINAL SOURCE: HIV
                 (E)     INDIVIDUAL ISOLATE:  _____
         (iv)    IMMEDIATE SOURCE:
                 (C)     CLONE:  _____
         (v)     POSITION IN GENOME: Within Env Gene
         (vi)    PROPERTIES OF SEQUENCE:   Expresses conserved
                                           antigenic determinant
         (viii)  SEQUENCE DESCRIPTION:
```

```
SEQ ID NO:  EE3032-2
```

```
1                5                      10                     15
Cys Thr Arg Pro Asn Asn Asn Thr Arg Lys Gly Ile His MET Gly
TGT ACA AGG CCC AAT AAC AAT ACA AGA AAA GGT ATA CAT ATG GGA
```

```
                20                     25                     30
Pro Gly Arg Ala Phe Tyr Thr Thr Gly Asp Ile Ile Gly Asp Ile
CCA GGG AGG GCA TTT TAT ACA ACA GGA GAC ATA ATA GGA GAT ATA
```

```
                35
Arg Gln Ala His Cys
AGA CAA GCA CAT TGT
```

```
(2)      INFORMATION FOR SEQ ID NO: EE3032-3
         (i)     SEQUENCE CHARACTERISTICS:
                 (A)     LENGTH:  105
                 (B)     TYPE:  Nucleic Acid
                 (C)     STRANDEDNESS:  Single
```

```
              (D)      TOPOLOGY:  Linear
      (ii)    KIND: cDNA to genomic RNA
      (ii)    KIND (if peptide or protein):
              (A)      SEQUENCE ASSEMBLY METHOD:  Overlap
              (B)      FRAGMENT TYPE:  Internal Fragment
              (C)      HYPOTHETICAL:  _____
      (iii)   ORIGINAL SOURCE: HIV
              (E)      INDIVIDUAL ISOLATE:  _____
      (iv)    IMMEDIATE SOURCE:
              (C)      CLONE:  _____
      (v)     POSITION IN GENOME: Within Env Gene
      (vi)    PROPERTIES OF SEQUENCE:  Expresses conserved
                                       antigenic determinant
      (viii)  SEQUENCE DESCRIPTION:
```

SEQ ID NO:  EE3032-3

```
  1                5                        10                      15
Cys Thr Arg Pro Asn Asn Asn Thr Arg Lys Ser Ile His Ile Gly
TGT ACA AGG CCC AAT AAC AAT ACA AGA AAA AGT ATA CAT ATA GGA


                   20                       25                      30
Pro Gly Arg Ala Phe Tyr Thr Thr Gly Asp Ile Ile Gly Asp Ile
CCA GGG AGG GCA TTT TAT ACA ACA GGA GAC ATA ATA GGA GAT ATA


                   35
Arg Gln Ala His Cys
AGA CAA GCA CAT TGT
```

```
(2)      INFORMATION FOR SEQ ID NO: EEE6405-1
         (i)      SEQUENCE CHARACTERISTICS:
                  (A)      LENGTH:  105
                  (B)      TYPE:  Nucleic Acid
                  (C)      STRANDEDNESS:  Single
                  (D)      TOPOLOGY:  Linear
         (ii)     KIND: cDNA to genomic RNA
         (ii)     KIND (if peptide or protein):
                  (A)      SEQUENCE ASSEMBLY METHOD:  Overlap
                  (B)      FRAGMENT TYPE:  Internal Fragment
                  (C)      HYPOTHETICAL:  _____
         (iii)    ORIGINAL SOURCE: HIV
                  (E)      INDIVIDUAL ISOLATE:  _____
         (iv)     IMMEDIATE SOURCE:
                  (C)      CLONE:  _____
         (v)      POSITION IN GENOME: Within Env Gene
```

```
                        20                        25                        30
Pro Gly Arg Ala Phe Tyr Ala Thr Gly Glu Ile MET Gly Asp Ile
CCA GGG AGA GCA TTT TAT GCA ACA GGA GAA ATA ATG GGA GAT ATA


                        35
Arg Gln Ala His Cys
AGA CAA GCA CAT TGT
```

(2)      INFORMATION FOR SEQ ID NO: EE6405-3
         (i)      SEQUENCE CHARACTERISTICS:
                  (A)      LENGTH:  105
                  (B)      TYPE:  Nucleic Acid
                  (C)      STRANDEDNESS:  Single
                  (D)      TOPOLOGY:  Linear
         (ii)     KIND: cDNA to genomic RNA
         (ii)     KIND (if peptide or protein):
                  (A)      SEQUENCE ASSEMBLY METHOD:  Overlap
                  (B)      FRAGMENT TYPE:  Internal Fragment
                  (C)      HYPOTHETICAL:  _____
         (iii)    ORIGINAL SOURCE: HIV
                  (E)      INDIVIDUAL ISOLATE:  _____
         (iv)     IMMEDIATE SOURCE:
                  (C)      CLONE:  _____
         (v)      POSITION IN GENOME: Within Env Gene
         (vi)     PROPERTIES OF SEQUENCE:  Expresses conserved
                                           antigenic determinant
         (viii)   SEQUENCE DESCRIPTION:


SEQ ID NO:  EE6405-3


```
    1                    5                        10                        15
Cys Thr Arg Pro Asn Asn Asn Thr Arg Lys Ser Ile Pro Ile Gly
TGT ACA AGA CCC AAC AAC AAT ACA AGG AAA AGT ATA CCT ATA GGA


                        20                        25                        30
Pro Arg Arg Ala Phe Tyr Ala Thr Gly Asp Ile Ile Gly Asp Ile
CCA AGG AGA GCA TTT TAT GCA ACA GGA GAC ATA ATA GGA GAT ATA


                        35
Arg Gln Ala His Cys
AGA CAA GCA CAT TGT
```

(2)     INFORMATION FOR SEQ ID NO: EE6636-1
        (i)     SEQUENCE CHARACTERISTICS:
                (A)     LENGTH:  105
                (B)     TYPE:  Nucleic Acid
                (C)     STRANDEDNESS:  Single
                (D)     TOPOLOGY:  Linear
        (ii)    KIND: cDNA to genomic RNA
        (ii)    KIND (if peptide or protein):
                (A)     SEQUENCE ASSEMBLY METHOD:  Overlap
                (B)     FRAGMENT TYPE:  Internal Fragment
                (C)     HYPOTHETICAL:  _____
        (iii)   ORIGINAL SOURCE: HIV
                (E)     INDIVIDUAL ISOLATE:  _____
        (iv)    IMMEDIATE SOURCE:
                (C)     CLONE:  _____
        (v)     POSITION IN GENOME: Within Env Gene
        (vi)    PROPERTIES OF SEQUENCE:   Expresses conserved
                                          antigenic determinant
        (viii)  SEQUENCE DESCRIPTION:


SEQ ID NO:   EE6636-1


```
1                     5                     10                    15
Cys Thr Arg Pro Asn Asn Asn Thr Arg Lys Ser Ile His Ile Gly
TGT ACA AGA CCC AAC AAC AAT ACA AGA AAA AGT ATA CAT ATA GGA


                      20                    25                    30
Pro Gly Arg Ala Phe Tyr Thr Thr Gly Glu Ile Ile Gly Asn Ile
CCA GGG AGA GCA TTT TAT ACA ACA GGA GAA ATA ATA GGA AAT ATA


                      35
Arg Gln Ala His Cys
AGA CAA GCA CAT TGT
```


(2)     INFORMATION FOR SEQ ID NO: EE6636-2
        (i)     SEQUENCE CHARACTERISTICS:
                (A)     LENGTH:  105
                (B)     TYPE:  Nucleic Acid
                (C)     STRANDEDNESS:  Single
                (D)     TOPOLOGY:  Linear
        (ii)    KIND: cDNA to genomic RNA
        (ii)    KIND (if peptide or protein):
                (A)     SEQUENCE ASSEMBLY METHOD:  Overlap
                (B)     FRAGMENT TYPE:  Internal Fragment

```
                    (C)      HYPOTHETICAL:  _____
          (iii)  ORIGINAL SOURCE: HIV
                    (E)      INDIVIDUAL ISOLATE:  _____
          (iv)   IMMEDIATE SOURCE:
                    (C)      CLONE:  _____
          (v)    POSITION IN GENOME: Within Env Gene
          (vi)   PROPERTIES OF SEQUENCE:  Expresses conserved
                                          antigenic determinant
          (viii) SEQUENCE DESCRIPTION:
```

SEQ ID NO:  EE6636-2

```
    1                5                        10                      15
Cys Thr Arg Pro Asn Asn Asn Thr Arg Lys Ser Ile His Ile Gly
TGT ACA AGA CCC AAC AAC AAT ACA AGA AAA AGT ATA CAT ATA GGA


                    20                       25                      30
Pro Gly Arg Ala Phe Tyr Thr Thr Gly Glu Ile Ile Gly Asn Ile
CCA GGG AGA GCA TTT TAT ACA ACA GGA GAA ATA ATA GGA AAT ATA


                    35
Arg Gln Ala His Cys
AGA CAA GCA CAT TGT
```

```
(2)      INFORMATION FOR SEQ ID NO: EE6636-3
          (i)    SEQUENCE CHARACTERISTICS:
                    (A)      LENGTH:  105
                    (B)      TYPE:  Nucleic Acid
                    (C)      STRANDEDNESS:  Single
                    (D)      TOPOLOGY:  Linear
          (ii)   KIND: cDNA to genomic RNA
          (ii)   KIND (if peptide or protein):
                    (A)      SEQUENCE ASSEMBLY METHOD:  Overlap
                    (B)      FRAGMENT TYPE:  Internal Fragment
                    (C)      HYPOTHETICAL:  _____
          (iii)  ORIGINAL SOURCE: HIV
                    (E)      INDIVIDUAL ISOLATE:  _____
          (iv)   IMMEDIATE SOURCE:
                    (C)      CLONE:  _____
          (v)    POSITION IN GENOME: Within Env Gene
          (vi)   PROPERTIES OF SEQUENCE:  Expresses conserved
                                          antigenic determinant
          (viii) SEQUENCE DESCRIPTION:
```

SEQ ID NO: EE6636-3

```
            1                   5                  10                  15
        Cys Thr Arg Pro Asn Asn Asn Thr Arg Lys Ser Ile His Ile Gly
        TGT ACA AGA CCC AAC AAC AAT ACA AGA AAA AGT ATA CAT ATA GGA


                               20                  25                  30
        Pro Gly Arg Ala Phe Tyr Thr Thr Gly Glu Ile Ile Gly Asn Ile
        CCA GGG AGA GCA TTT TAT ACA ACA GGA GAA ATA ATA GGA AAT ATA


                               35
        Arg Gln Ala His Cys
        AGA CAA GCA CAT TGT
```

## Claims

1. An antigenic conjugate of HIV major neutralization determinant covalently linked to purified outer membrane proteosome of Neisseria, comprising an antigenic conjugate of the formula

   (PND)$_n$~(Omp),

   or pharmaceutically acceptable salt thereof, wherein:
   PND      is the major neutralization determinant of HIV, which is a polypeptide of one or more amino acid sequences;
   n        indicates the number of polypeptides of PND covalently linked to Omp and is 1-50;
   ~        indicates covalent linkage;
   Omp      is purified outer membrane proteosome of Neisseria,
      said polypeptide having a sequence of 35 amino acids or less, but at least 5 amino acids in length;
      said polypeptide containing in its sequence Gly-X-Gly, wherein X is proline, leucine, alanine, glutamine or serine;
      said polypeptide having any of the sequences given in the sequence listing with the exception of sequence nos. EE90-1, EE90-2, EE90-3, EE312-1, EE360-1, EE360-2, EE360-3, EE667-3 and EE6405-3.

2. The antigenic conjugate of claim 1 wherein X is proline.

3. The antigenic conjugate of claim 1 wherein the covalent linkage between PND and Omp consists essentially of a bigeneric spacer.

4. The antigenic conjugate of claims 1-3, in combination with any of the antivirals, immunomodulators, anti-infectives or vaccines of Table I.

5. The antigenic conjugate of claims 1-3, wherein said Omp is derived from Neisseria meningitidis.

6. A cocktail of antigenic conjugates consisting essentially of a mixture of more than one molecular species of the antigenic conjugates of claims 1-3.

7. An AIDS vaccine comprising an antigenic conjugate of HIV major neutralization determinant covalently linked to purified outer membrane proteosome of Neisseria, said conjugate of the formula

   (PND)$_n$~(Omp),

175

or pharmaceutically acceptable salt thereof, wherein:

PND     is the major neutralization determinant of HIV, which is a polypeptide of one or more amino acid sequences;

n     indicates the number of polypeptides of PND covalently linked to Omp and is 1-50;

~     indicates covalent linkage;

Omp     is purified outer membrane proteosome of Neisseria;

said polypeptide having a sequence of 35 amino acids or less, but at least 5 amino acids in length;

said polypeptide containing in its sequence Gly-X-Gly, wherein X is proline, leucine, alanine, glutamine or serine;

said polypeptide having any of the sequences given in the sequence listing;

said conjugate mixed with a suitable immunological adjuvant, carrier or vector, said vaccine to be used pre- and post-exposure to prevent or treat HIV infection or disease, said vaccine capable of eliciting specific HIV neutralizing antibodies.

8. The AIDS vaccine of claim 7 wherein X is proline.

9. The AIDS vaccine of claim 7 wherein the covalent linkage betwen PND and Omp consists essentially of a bigeneric spacer.

10. The AIDS vaccine of claims 7-9 in combination with any of the antivirals, immunomodulators, anti-infectives or vaccines of Table I.

11. The AIDS vaccine of claims 7-9, wherein said Omp is derived from Neisseria meningitidis.

12. The AIDS vaccine of claism 7-9 comprising a cocktail of said antigenic conjugates, said cocktail consisting essentially of a mixture of more than one molecular species of said antigenic conjugates.

13. A pharmaceutical composition comprising an antigenic conjugate of HIV major neutralization determinant covalently linked to purified outer membrane proteosome of Neisseria, said antigenic conjugate of the formula

(PND)n~(Omp),

or pharmaceutically acceptable salt thereof, wherein:

PND     is the major neutralization determinant of HIV, which is a polypeptide of one or more amino acid sequences;

n     indicates the number of polupeptides of PND covalently linked to Omp and is 1-50;

~     indicates covalent linkage;

Omp     is purified outer membrane proteosome of Neisseria,

said polypeptide having a sequence of 35 amino acids or less, but at least 5 amino acids in length;

said polypeptide containing in its sequence Gly-X-Gly, wherein X is proline, leucine, alanine, glutamine or serine;

said polypeptide having any of the sequences given in the sequence listing;

said conjugate mixed with a suitable immunological adjuvant, said composition useful as a vaccine capable of producing specific HIV neutralizing antibody in mammals.

14. The composition of claim 13 wherein X is proline.

15. The composition of claim 13 wherein the covalent linkage between PND and Omp consists essentially of a bigeneric spacer.

16. The composition of claims 13-15, in combination with any of the antivirals, immunomodulators, anti-infectives or vaccines of Table I.

17. The composition of claims 13-15, wherein said Omp is derived from Neisseria meningitidis.

18. A pharmaceutical composition containing a cocktail of antigenic conjugates consisting essentially of a mixture of more than one molecular species of the antigenic conjugates of claims 13-15.

19. The use of a conjugate as claimed in claim 1 for the preparation of a medicament for vaccinating against AIDS or ARC.

20. The use of a conjugate as claimed in claim 2 for the preparation of a medicament for vaccinating against AIDS or ARC.

21. The use of a conjugate as claimed in claim 3 for the preparation of a medicament for vaccinating against AIDS or ARC.

22. The use of a conjugate as claimed in claim 1 together with any of the antivirals, immunolodulators or anti-infectives of Table I for the preparation of a medicament for vaccinating against AIDS or ARC.

23. The use as claimed in claim 19 or 20 wherein the Omp is derived from Neisseria meningitidis.

24. The use of a conjugate as claimed in claim 1 for the preparation of a medicament for the prevention or treatment of infection by HIV, or for the treatment of AIDS.

25. The use of a conjugate as claimed in claim 2 for the preparation of a medicament for the prevention or treatment of infection by HIV, or for the treatment of AIDS.

26. The use of a conjugate as claimed in claim 3 for the preparation of a medicament for the prevention or treatment of infection by HIV, or for the treatment of AIDS.

27. The use of a conjugate as claimed in claim 1 together with any of the antivirals, immunolodulators or anti-infectives of Table I for the preparation of a medicament for the prevention or treatment of infection by HIV, or for the treatment of AIDS.

28. The use as claimed in claim 24 or 25 wherein the Omp is derived from Neisseria meningitidis.